# EUROPÄISCHE PATENTSCHRIFT

(11) **EP 1 963 288 B1**
(45) Veröffentlichungstag und Bekanntmachung des Hinweises auf die Patenterteilung: **07.09.2011**
(21) Anmeldenummer: 06841029.9
(22) Anmeldetag: 19.12.2006
(51) Int. Cl.: C07D 263/48, C07D 413/04, C07D 413/06, C07D 413/14, A61K 31/41, A61K 31/435, A61K 31/495, A61P 29/00

(54) **SUBSTITUIERTE OXAZOL-DERIVATE MIT ANALGETISCHER WIRKUNG**
SUBSTITUTED OXAZOLE DERIVATIVES WITH AN ANALGESIC ACTION
DERIVES OXAZOL SUBSTITUES A EFFET ANALGESIQUE

(30) Priorität: 22.12.2005 DE 102005061429
(43) Veröffentlichungstag der Anmeldung: 03.09.2008
(73) Patentinhaber: Grünenthal GmbH, 52078 Aachen (DE)
(72) Erfinder: MERLA, Beatrix, 52078 Aachen (DE); OBERBÖRSCH, Stefan, 52078 Aachen (DE); SUNDERMANN, Bernd, 61381 Friedrichsdorf (DE); ENGLBERGER, Werner, 52223 Stolberg (DE); GRAUBAUM, Heinz, 12557 Berlin (DE)
(86) Internationale Anmeldenummer: PCT/EP2006/012222
(87) Internationale Veröffentlichungsnummer: WO 2007/079928

(56) Entgegenhaltungen:
- WO-A-99/09829
- WO-A-99/09979

## Beschreibung

Die vortiegende Erfindung betrifft substituierte Oxazol-Derivate, Verfahren zu deren Herstellung, Arzneimittel enthaltend diese Verbindungen und die Verwendung von substituierten Oxazol-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

In Org. Lett 2001, 3, 877-880 werden Oxazol-Derivate offenbart, die ebenfalls in 5-Position mit einem sekundären Amin substituiert sind. Diese Oxazol-Derivate sind jedoch nicht in 2-Position über eine substituierte Alkylkette mit einem aminomethylsubstituierten Cyclohexylrest substituiert.

Weiterhin offenbaren WO 99/09829 A1 und WO 99/09979 A1 unter anderem Phenyloxazole für die Behandlung von Schmerz bzw.neuropathischem Schmerz insbesondere bei Tieren, wobei diese Oxazol-Derivate in 5-Position zum einen nicht mit einem sekundären Amin substituiert sind und zum anderen in der 2-Position mit einem substituierten Phenyl substituiert sind.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen.

Gegenstand der Erfindung sind daher substituierte Oxazol-Derivate der allgemeinen Formell, 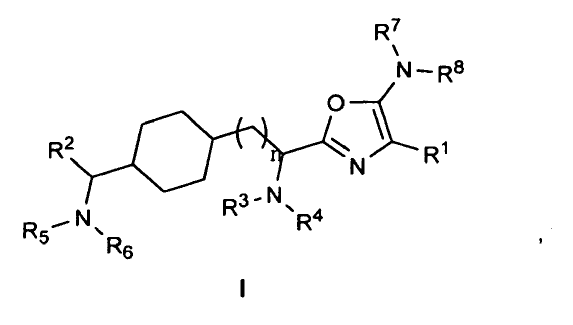 worin
n 0 oder 1 bedeutet
R¹ Benzyl bedeutet;
R² Phenyl, unsubstituiert oder einfach substituiert mit Cl oder F oder Thienyl bedeutet;
R³ und R⁴ unabhängig voneinander Phenyl, Ethyl oder Methyl bedeuten oder die Reste R³ und R⁴ zusammen CH₂CH₂OCH₂CH₂, CH₂CH₂NR⁹CH₂CH₂, (CH₂)₄₋₅ oder 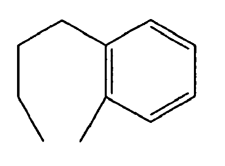 bedeuten, wobei R⁹ für Benzyl, 4-F-Phenyl oder 4-Methoxyphenyl steht,
R⁵ und R⁶ CH3 bedeuten,
R⁷ und R⁸ unabhängig voneinander Methyl, Ethyl, Benzyl oder Phenethyl bedeuten;
oder die Reste R⁷ und R⁸ CH₂CH₂SCH₂CH₂, CH₂CH₂OCH₂CH₂, (CH₂)₄ oder (CH₂)₅, CH₂CH₂NR¹⁰CH₂CH₂, wobei einzeine H-Atome durch Methyl oder Benzyl ersetzt sein können, und R¹⁰ Phenyl, 4-Methoxyphenyl oder Benzyl bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Verbindungen weisen eine Affinität zum µ-Opioid-Rezeptor auf.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt ist die Salzsäure.

Unter dem Begriff (CH₂)₃₋₆ bzw. (CH₂)₄₋₅ ist -CH₂-CH₂-CH₂-, -CH₂-CH₂-CH₂-CH₂-, - CH₂-CH₂-CH₂-CH₂-CH₂- und CH₂-CH₂-CH₂-CH₂-CH₂-CH₂- bzw -CH₂-CH₂-CH₂-CH₂-, -CH₂-CH₂-CH₂-CH₂-CH₂ zu verstehen.

Ganz besonders bevorzugt sind substituierte Oxazol-Derivate aus der Gruppe
17. [{4-[(4-Benzyl-5-pyrrolidin-1-yl-oxazol-2-yl)-piperidin-1-yl-methyl]-cyclohexyl}-(4-fluor-phenyl)-methyl]-dimethyl-amin
18. ((4-Benzyl-5-morpholin-4-yl-oxazol-2-yl)-{4-[dimethylamino-(4-fluor-phenyl)-methyl]-cyclohexyl}-methyl)-methyl-phenyl-amin
19. ([4-Benzyl-5-(4-methyl-piperidin-1-yl)-oxazol-2-yl]-{4-[dimethylamino-(4-fluorphenyl)-methyl]-cyclohexyl}-methyl)-methyl-phenyl-amin
20. ([4-Benzyl-5-(4-methyl-piperidin-1-yl)-oxazol-2-yl]-{4-[(4-chlor-phenyl)-dimethylamino-methyl]-cyclohexyl}-methyl)-diethyl-amin
21. Benzyl-[4-benzyl-2-({4-[(4-chlor-phenyl)-dimethylamino-methyl]-cyclohexyl}-diethylamino-methyl)-oxazol-5-yl]-methyl-amin
22. Benzyl-{4-benzyl-2-[{4-[(4-chlor-phenyl)-dimethylamino-methyl]-cyclohexyl}-(methyl-phenyl-amino)-methyl]-oxazol-5-yl}-methyl-amin
23. [(4-{[4-Benzyl-5-(4-benzyl-piperidin-1-yl)-oxazol-2-yl]-piperidin-1-yl-methyl}-cyclohexyl)-thiophen-2-yl-methyl]-dimethyl-amin
24. [(4-{[4-Benzyl-5-(4-methyl-piperidin-1-yl)-oxazol-2-yl]-piperidin-1-yl-methyl}-cyclohexyl)-thiophen-2-yl-methyl]-dimethyl-amin
25. Benzyl-(4-benzyl-2-{[4-(dimethylamino-thiophen-2-yl-methyl)-cyclohexyl]-piperidin-1-yl-methyl}-oxazol-5-yl)-methyl-amin
26. [(4-{[4-Benzyl-5-(4-phenyl-piperazin-1-yl)-oxazol-2-yl]-piperidin-1-yl-methyl}-cyclohexyl)-thiophen-2-yl-methyl]-dimethyl-amin
27. {[4-Benzyl-5-(4-benzyl-piperidin-1-yl)-oxazol-2-yl]-[4-(dimethylamino-thiophen-2-yl-methyl)-cyclohexyl]-methyl}-diethyl-amin
28. {[4-Benzyl-5-(4-benzyl-piperazin-1-yl)-oxazol-2-yl]-[4-(dimethylamino-thiophen-2-yl-methyl)-cyclohexyl]-methyl}-diethyl-amin
29. {[4-Benzyl-5-(4-methyl-piperidin-1-yl)-oxazol-2-yl]-[4-(dimethylamino-thiophen-2-yl-methyl)-cyclohexyl]-methyl}-diethyl-amin
30. Benzyl-(4-benzyl-2-{diethylamino-[4-(dimethylamino-thiophen-2-yl-methyl)-cyclohexyl]-methyl}-oxazol-5-yl)-methyl-amin
31. {[4-Benzyl-5-(4-phenyl-piperazin-1-yl)-oxazol-2-yl]-[4-(dimethylamino-thiophen-2-yl-methyl)-cyctohexyl]-methyl}-diethyl-amin
32. ({4-[[4-Benzyl-5-(4-benryl-piperidin-1-yl)-oxazol-2-yl]-(4-benzyl-piperazin-1-yl)-methyl]-cyclohexyl}-thiophen-2-yl-methyl)-dimethyl-amin
33. [(4-{[4-Benzyl-5-(3-methyl-piperidin-1-yl)-oxazo-2-yl]-piperidin-1-yl-methyl}-cyclohexyl)-(4-fluor-phenyl)-methyl]-dimethyl-amin
34. [4-Benzyl-2-(diethylamino-{4-[dimethylamino-(4-fluor-phenyl)-methyl]-cyclohexyl}-methyl)-oxazol-5-yl]-methyl-phenethyl-amin
35. [4-Benzyl-2-(diethylamino-{4-[dimethylamino-(4-fluor-phenyl)-methyl]-cyclohexyl}-methyl)-oxazol-5-yl]-diethyl-amin
36. ([4-Benzyl-5-(3-methyl-piperidin-1-yl)-oxazol-2-yl]-{4-[dimethylamino-(4-fluorphenyl)-methyl]-cyclohexyl}-methyl)-diethyl-amin
37. ([4-Benzyl-5-(3,5-dimethyl-piperidin-1-yl)-oxazol-2-yl]-{4-[dimethylamino-(4-fluorphenyl)-methyl]-cyclohexyl}-methyl)-methyl-phenyl-amin
38. {4-Benzyl-2-[{4-[dimethylamino-(4-fluor-phenyl)-methyl]-cyclohexyl}-(methylphenyl-amino)-methyl]-oxazol-5-yl}-methyl-phenethyl-amin
39. [4-Benzyl-2-({4-[(4-chlor-phenyl)-dimethylamino-methyl]-cyclohexyl}-piperidin-1-yl-methyl)-oxazol-5-yl]-diethyl-amin
40. [(4-{[4-Benzyl-5-(3-methyl-piperidin-1-yl)-oxazol-2-yl]-piperidin-1-yl-methyl}-cyclohexyl)-(4-chlor-phenyl)-methyl]-dimethyl-amin
41. ([4-Benzyl-5-(3,5-dimethyl-piperidin-1-yl)-oxazol-2-yl]-{4-[(4-chlor-phenyl)-dimethylamino-methyl]-cyclohexyl}-methyl)-diethyl-amin
42. ([4-Benzyl-5-(3,5-dimethyl-piperidin-1-yl)-oxazol-2-yl]-{4-[(4-chlor-phenyl)-dimethylamino-methyl]-cyclohexyl}-methyl)-methyl-phenyl-amin
43. {4-Benzyl-2-[{4-[(4-chlor-phenyl)-dimethylamino-methyl]-cyclohexyl}-(methylphenyl-amino)-methyl]-oxazol-5-yl}-methyl-phenethyl-amin
44. [4-Benzyl-2-((4-benzyl-piperazin-1-yl)-{4-[(4-chlor-phenyl)-dimethylaminomethyl]-cyclohexyl}-methyl)-oxazol-5-yl]-diethyl-amin
45. [(4-{[4-Benzyl-5-(3,5-dimethyl-piperidin-1-yl)-oxazol-2-y1]-piperidin-1-yl-methyl}-cyclohexyl)-thiophen-2-yl-methyl]-dimethyl-amin
46. (4-Benzyl-2-{[4-(dimethylamino-thiophen-2-yl-methyl)-cyclohexyl]-piperidin-1-yl-methyl}-oxazol-5-yl)-methyl-phenethyl-amin
47. (4-Benzyl-2-{[4-(dimethylamino-thiophen-2-yl-methyl)-cyclohexyl]-piperidin-1-yl-methyl}-oxazol-5-yl)-diethyl-amin
48. ({4-[(4-Benzyl-5-thiomorpholin-4-yl-oxazol-2-yl)-piperidin-1-yl-methyl]-cyclohexyl}-thiophen-2-yl-methyl)-dimethyl-amin
49. (4-Benzyl-2-{diethylamino-[4-(dimethylamino-thiophen-2-yl-methyl)-cyclohexyl]-methyl}-oxazol-5-yl)-methyl-phenethyl-amin
50. (4-Benzyl-2-{diethylamino-[4-(dimethylamino-thiophen-2-yl-methyl)-cyclohexyl]-methyl}-oxazol-5-yl)-diethyl-amin
51. {[4-Benzyl-5-(3-methyl-piperidin-1-yl)-oxazol-2-yl]-[4-(dimethylamino-thiophen-2-yl-methyl)-cyclohexyl]-methyl}-diethyl-amin
52. {(4-Benzyl-5-thiomorpholin-4-yl-oxazol-2-yl)-[4-(dimethylamino-thiophen-2-yl-methyl)-cyclohexyl]-methyl}-methyl-phenyl-amin
53. ({4-[[4-Benzyl-5-(3,5-dimethyl-piperidin-1-yl)-oxazol-2-yl]-(4-benzyl-piperazin-1-yl)-methyl]-cyclohexyl}-thiophen-2-yl-methyl)-dimethyl-amin
54. (4-Benzyl-2-{(4-benzyl-piperazin-1-yl)-[4-(dimethylamino-thiophen-2-yl-methyl)-cyclohexyl]-methyl}-oxazol-5-yl)-methyl-phenethyl-amin
55. ({4-[(4-Benzyl-piperazin-1-yl)-(4-benzyl-5-thiomorpholin-4-yl-oxazol-2-yl)-methyl]-cyclohexyl}-thiophen-2-yl-methyl)-dimethyl-amin
56. [(4-{2-[4-Benzyl-5-(4-benzyl-piperazin-1-yl)-oxazol-2-yl]-2-piperidin-1-yl-ethyl}-cyclohexyl)-(4-chlor-phenyl)-methyl]-dimethyl-amin
57. [(4-{2-[4-Benzyl-5-(4-methyl-piperidin-1-yl)-oxazol-2-yl]-2-piperidin-1-yl-ethyl}-cyclohexyl)-(4-chlor-phenyl)-methyl]-dimethyl-amin
58. Benzyl-[4-benzyl-2-(2-{4-[(4-chlor-phenyl)-dimethylamino-methyl]-cyclohexyl}-1-piperidin-1-yl-ethyl)-oxazol-5-yl]-methyl-amin
59. (1-[4-Benzyl-5-(4-benzyl-piperidin-1-yl)-oxazol-2-yl]-2-{4-[(4-chlor-phenyl)-dimethylamino-methyl]-cyclohexyl}-ethyl)-diethyl-amin
60. (1-[4-Benzyl-5-(4-methyl-piperidin-1-yl)-oxazol-2-yl]-2-{4-[(4-chlor-phenyl)-dimethylamino-methyl]-cyclohexyl}-ethyl)-diethyl-amin
61. Benzyl-[4-benzyl-2-(2-{4-[(4-chlor-phenyl)-dimethylamino-methyl]-cyclohexyl}-1-diethylamino-ethyl)-oxazol-5-yl]-methyl-amin
62. [{4-[2-[4-Benzyl-5-(4-benzyl-piperidin-1-yl)-oxazol-2-yl]-2-(4-benzyl-piperazin-1-yl)-ethyl]-cyclohexyl}-(4-chlor-phenyl)-methyl]-dimethyl-amin
63. [{4-[2-[4-Benzyl-5-(4-benzyl-piperazin-1-yl)-oxazol-2-yl]-2-(4-benzyl-piperazin-1-yl)-ethyl]-cyclohexyl}-(4-chlor-phenyl)-methyl]-dimethyl-amin
64. [{4-[2-[4-Benzyl-5-(4-methyl-piperidin-1-yl)-oxazol-2-yl]-2-(4-benzyl-piperazin-1-yl)-ethyl]-cyclohexyl}-(4-chlor-phenyl)-methyl]-dimethyl-amin
65. [(4-{2-[4-Benzyl-5-(4-methyl-piperidin-1-yl)-oxazol-2-yl]-2-piperidin-1-yl-ethyl}-cyclohexyl)-(4-fluor-phenyl)-methyl]-dimethyl-amin
66. Benzyl-[4-benzyl-2-(2-{4-[dimethylamino-(4-fluor-phenyl)-methyl]-cyclohexyl}-1-piperidin-1-yl-ethyl)-oxazol-5-yl]-methyl-amin
67. (1-[4-Benzyl-5-(4-benzyl-piperidin-1-yl)-oxazol-2-yl]-2-{4-[dimethylamino-(4-fluor-phenyl)-methyl]-cyclohexyl}-ethyl)-diethyl-amin
68. (1-[4-Benzyl-5-(4-benzyl-piperazin-1-yl)-oxazol-2-yl]-2-{4-[dimethylamino-(4-fluor-phenyl)-methyl]-cyclohexyl}-ethyl)-diethyl-amin
69. (1-[4-Benzyl-5-(4-methyl-piperidin-1-yl)-oxazol-2-yl]-2-{4-[dimethylamino-(4-fluor-phenyl)-methyl]-cyclohexyl}-ethyl)-diethyl-amin
70. Benzyl-[4-benzyl-2-(1-diethylamino-2-{4-[dimethylamino-(4-fluor-phenyl)-methyl]-cyclohexyl}-ethyl)-oxazol-5-yl]-methyl-amin
71. [{4-[2-[4-Benzyl-5-(4-methyl-piperidin-1-yl)-oxazol-2-yl]-2-(4-benzyl-piperazin-1-yl)-ethyl]-cyclohexyl}-(4-fluor-phenyl)-methyl]-dimethyl-amin
72. [(4-{2-[4-Benzyl-5-(4-benzyl-piperidin-1-yl)-oxazol-2-yl]-2-piperidin-1-yl-ethyl}-cyclohexyl)-thiophen-2-yl-methyl]-dimethyl-amin
73. [(4-{2-[4-Benzyl-5-(4-benzyl-piperazin-1-yl)-oxazol-2-yl]-2-piperidin-1-yl-ethyl}-cyclohexyl)-thiophen-2-yl-methyl]-dimethyl-amin
74. [(4-{2-[4-Benzyl-5-(4-methyl-piperidin-1-yl)-oxazol-2-yl]-2-piperidin-1-yl-ethyl}-cyclohexyl)-thiophen-2-yl-methyl]-dimethyl-amin
75. Benzyl-(4-benzyl-2-{2-[4-(dimethylamino-thiophen-2-yl-methyl)-cyclohexyl]-1-piperidin-1-yl-ethyl}-oxazol-5-yl)-methyl-amin
76. [(4-{2-[4-Benzyl-5-(4-phenyl-piperazin-1-yl)-oxazol-2-yl]-2-piperidin-1-yl-ethyl}-cyclohexyl)-thiophen-2-yl-methyl]-dimethyl-amin
77. {1-[4-Benzyl-5-(4-benzyl-piperidin-1-yl)-oxazol-2-yl]-2-[4-(dimethylamino-thiophen-2-yl-methyl)-cyclohexyl]-ethyl}-diethyl-amin
78. {1-[4-Benzyl-5-(4-benzyl-piperazin-1-yl)-oxazol-2-yl]-2-[4-(dimethylamino-thiophen-2-yl-methyl)-cyclohexyl]-ethyl}-diethyl-amin
79. {1-[4-Benzyl-5-(4-methyl-piperidin-1-yl)-oxazol-2-yl]-2-[4-(dimethylamino-thiophen-2-yl-methyl)-cyclohexyl]-ethyl}-diethyl-amin
80. Benzyl-(4-benzyl-2-{1-diethylamino-2-[4-(dimethylamino-thiophen-2-yl-methyl)-cyclohexyl]-ethyl}-oxazol-5-yl)-methyl-amin
81. ({4-[2-[4-Benzyl-5-(4-benzyl-piperidin-1-yl)-oxazol-2-yl]-2-(4-benzyl-piperazin-1-yl)-ethyl]-cyclohexyl}-thiophen-2-yl-methyl)-dimethyl-amin
82. ({4-[2-[4-Benzyl-5-(4-benzyl-piperazin-1-yl)-oxazol-2-yl]-2-(4-benzyl-piperazin-1-yl)-ethyl]-cyclohexyl}-thiophen-2-yl-methyl)-dimethyl-amin
83. ({4-[2-[4-Benzyl-5-(4-methyl-piperidin-1-yl)-oxazol-2-yl]-2-(4-benzyl-piperazin-1-yl)-ethyl]-cyclohexyl}-thiophen-2-yl-methyl)-dimethyl-amin
84. Benzyl-(4-benzyl-2-{1-(4-benzyl-piperazin-1-yl)-2-[4-(dimethylamino-thiophen-2-yl-methyl)-cyclohexyl]-ethyl}-oxazol-5-yl)-methyl-amin
85. ({4-[2-[4-Benzyl-5-(4-phenyl-piperazin-1-yl)-oxazol-2-yl]-2-(4-benzyl-piperazin-1-yl)-ethyl]-cyclohexyl}-thiophen-2-yl-methyl)-dimethyl-amin
86. [4-Benzyl-2-(2-{4-[(4-chlor-phenyl)-dimethylamino-methyl]-cyclohexyl}-1-piperidin-1-yl-ethyl)-oxazol-5-yl]-methyl-phenethyl-amin
87. [4-Benzyl-2-(2-{4-[(4-chlor-phenyl)-dimethylamino-methyl]-cyclohexyl}-1-piperidin-1-yl-ethyl)-oxazol-5-yl]-diethyl-amin
88. (1-[4-Benzyl-5-(3,5-dimethyl-piperidin-1-yl)-oxazol-2-yl]-2-{4-[(4-chlor-phenyl)-dimethylamino-methyl]-cyclohexyl}-ethyl)-diethyl-amin
89. [4-Benzyl-2-(2-{4-[(4-chlor-phenyl)-dimethylamino-methyl]-cyclohexyl}-1-diethylamino-ethyl)-oxazol-5-yl]-methyl-phenethyl-amin
90. [4-Benzyl-2-(2-{4-[(4-chlor-phenyl)-dimethylamino-methyl]-cyclohexyl}-1-diethylamino-ethyl)-oxazol-5-yl]-diethyl-amin
91. (1-(4-Benzyl-5-thiomorpholin-4-yl-oxazol-2-yl)-2-{4-[(4-chlor-phenyl)-dimethylamino-methyl]-cyclohexyl}-ethyl)-diethyl-amin
92. (1-(4-Benzyl-5-thiomorpholin-4-yl-oxazol-2-yl)-2-{4-[(4-chlor-phenyl)-dimethylamino-methyl]-cyclohexyl}-ethyl)-methyl-phenyl-amin
93. [4-Benzyl-2-(1-(4-benzyl-piperazin-1-yl)-2-{4-[(4-chlor-phenyl)-dimethylaminomethyl]-cyclohexyl}-ethyl)-oxazol-5-yl]-methyl-phenethyl-amin
94. [4-Benzyl-2-(1-(4-benzyl-piperazin-1-yl)-2-{4-[(4-chlor-phenyl)-dimethylaminomethyl]-cyclohexyl}-ethyl)-oxazol-5-yl]-diethyl-amin
95. [4-Benzyl-2-(2-{4-[dimethylamino-(4-fluor-phenyl)-methyl]-cyclohexyl}-1-piperidin-1-yl-ethyl)-oxazol-5-yl]-methyl-phenethyl-amin
96. (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-diethyl-amin
97. [4-Benzyl-2-(1-diethylamino-2-{4-[dimethylamino-(4-fluor-phenyl)-methyl]-cyclohexyl}-ethyl)-oxazol-5-yl]-methyl-phenethyl-amin
98. [4-Benzyl-2-(1-diethylamino-2-{4-[dimethylamino-(4-fluor-phenyl)-methyl]-cyclohexyl}-ethyl)-oxazol-5-yl]-diethyl-amin
99. (1-(4-Benzyl-5-thiomorpholin-4-yl-oxazol-2-yl)-2-{4-[dimethylamino-(4-fluorphenyl)-methyl]-cyclohexyl}-ethyl)-diethyl-amin
100. (1-[4-Benzyl-5-(3-methyl-piperidin-1-yl)-oxazol-2-yl]-2-{4-[dimethylamino-(4-fluor-phenyl)-methyl]-cyclohexyl}-ethyl)-diethyl-amin
101. (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-methyl-phenyl-amin
102. {4-Benzyl-2-[2-{4-[dimethylamino-(4-fluor-phenyl)-methyl]-cyclohexyl}-1-(methylphenyl-amino)-ethyl]-oxazol-5-yl}-methyl-phenethyl-amin
103. {4-Benzyl-2-[2-{4-[dimethylamino-(4-fluor-phenyl)-methyl]-cyclohexyl}-1-(methylphenyl-amino)-ethyl]-oxazol-5-yl}-diethyl-amin
104. (1-(4-Benzyl-5-thiomorpholin-4-yl-oxazol-2-yl)-2-{4-[dimethylamino-(4-fluorphenyl)-methyl]-cyclohexyl}-ethyl)-methyl-phenyl-amin
105. (1-[4-Benzyl-5-(3-methyl-piperidin-1-yl)-oxazol-2-yl]-2-{4-[dimethylamino-(4-fluor-phenyl)-methyl]-cyclohexyl}-ethyl)-methyl-phenyl-amin
106. [{4-[2-[4-Benzyl-5-(3-methyl-piperidin-1-yl)-oxazol-2-yl]-2-(4-benzyl-piperazin-1-yl)-ethyl]-cyclohexyl}-(4-fluor-phenyl)-methyl]-dimethyl-amin
107. [(4-{2-[4-Benzyl-5-(3,5-dimethyl-piperidin-1-yl)-oxazol-2-yl]-2-piperidin-1-yl-ethyl}-cyclohexyl)-thiophen-2-yl-methyl]-dimethyl-amin
108. (4-Benzyl-2-{2-[4-(dimethylamino-thiophen-2-yl-methyl)-cyclohexyl]-1-piperidin-1-yl-ethyl}-oxazol-5-yl)-methyl-phenethyl-amin
109. (4-Benzyl-2-{2-[4-(dimethylamino-thiophen-2-yl-methyl)-cyclohexyl]-1-piperidin-1-yl-ethyl}-oxazol-5-yl)-diethyl-amin
110. ({4-[2-(4-Benzyl-5-thiomorpholin-4-yl-oxazol-2-yl)-2-piperidin-1-yl-ethyl]-cyclohexyl}-thiophen-2-yl-methyl)-dimethyl-amin
111. [(4-{2-[4-Benzyl-5-(3-methyl-piperidin-1-yl)-oxazol-2-yl]-2-piperidin-1-yl-ethyl}-cyclohexyl)-thiophen-2-yl-methyl]-dimethyl-amin
112. {1-[4-Benzyl-5-(3,5-dimethyl-piperidin-1-yl)-oxazol-2-yl]-2-[4-(dimethylamino-thiophen-2-yl-methyl)-cyclohexyl]-ethyl}-diethyl-amin
113. (4-Benzyl-2-{1-diethylamino-2-[4-(dimethylamino-thiophen-2-yl-methyl)-cyclohexyl]-ethyl}-oxazol-5-yl)-methyl-phenethyl-amin
114. (4-Benzyl-2-{1-diethylamino-2-[4-(dimethylamino-thiophen-2-yl-methyl)-cyclohexyl]-ethyl}-oxazol-5-yl)-diethyl-amin
115. {1-(4-Benzyl-5-thiomorpholin-4-yl-oxazol-2-yl)-2-[4-(dimethylamino-thiophen-2-yl-methyl)-cyclohexyl]-ethyl}-diethyl-amin
116. {1-[4-Benzyl-5-(3-methyl-piperidin-1-yl)-oxazol-2-yl]-2-[4-(dimethylamino-thiophen-2-yl-methyl)-cyclohexyl]-ethyl}-diethyl-amin
117. {1-[4-Benzyl-5-(3-methyl-piperidin-1-yl)-oxazol-2-yl]-2-[4-(dimethylamino-thiophen-2-yl-methyl)-cyclohexyl]-ethyl}-methyl-phenyl-amin
118. ({4-[2-(4-Benzyl-piperazin-1-yl)-2-(4-benzyl-5-thiomorpholin-4-yl-oxazol-2-yl)-ethyl]-cyclohexyl}-thiophen-2-yl-methyl)-dimethyl-amin
119. ({4-[2-[4-Benzyl-5-(3-methyl-piperidin-1-yl)-oxazol-2-yl]-2-(4-benzyl-piperazin-1-yl)-ethyl]-cyclohexyl}-thiophen-2-yl-methyl)-dimethyl-amin
120. [{4-[(4-Benzyl-5-pyrrolidin-1-yl-oxazol-2-yl)-morpholin-4-yl-methyl]-cyclohexyl}-(3-fluor-phenyl)-methyl]-dimethyl-amin
121. [{4-[(4-Benzyl-5-morpholin-4-yl-oxazol-2-yl)-morpholin-4-yl-methyl]-cyclohexyl}-(3-fluor-phenyl)-methyl]-dimethyl-amin
122. [(4-{(4-Benzyl-5-pyrrolidin-1-yl-oxazol-2-yl)-[4-(4-fluor-phenyl)-piperazin-1-yl]-methyl}-cyclohexyl)-(3-fluor-phenyl)-methyl]-dimethyl-amin
123. [(4-{(4-Benzyl-5-pyrrolidin-1-yl-oxazol-2-yl)-[4-(4-methoxy-phenyl)-piperazin-1-yl]-methyl}-cyclohexyl)-(3-fluor-phenyl)-methyl]-dimethyl-amin
124. [(4-{(4-Benzyl-5-morpholin-4-yl-oxazol-2-yl)-[4-(4-methoxy-phenyl)-piperazin-1-yl]-methyl}-cyclohexyl)-(3-fluor-phenyl)-methyl]-dimethyl-amin
125. [{4-[(4-Benzyl-5-pyrrolidin-1-yl-oxazol-2-yl)-(3,4-dihydro-1 H-isochinolin-2-yl)-methyl]-cyclohexyl}-(3-fluor-phenyl)-methyl]-dimethyl-amin
126. [{4-[(4-Benzyl-5-morpholin-4-yl-oxazol-2-yl)-(3,4-dihydro-1 H-isochinolin-2-yl)-methyl]-cyclohexyl}-(3-fluor-phenyl)-methyl]-dimethyl-amin
127. [{4-[{4-Benzyl-5-[4-(4-methoxy-phenyl)-piperazin-1-yl]-oxazol-2-yl}-(3,4-dihydro-1 H-isochinolin-2-yl)-methyl]-cyclohexyl}-(3-fluor-phenyl)-methyl]-dimethyl-amin
128. [{4-[[4-Benzyl-5-(2,6-dimethyl-morpholin-4-yl)-oxazol-2-yl]-(3,4-dihydro-1 H-isochinolin-2-yl)-methyl]-cyclohexyl}-(3-fluor-phenyl)-methyl]-dimethyl-amin
129. [{4-[(4-Benzyl-5-pyrrolidin-1-yl-oxazol-2-yl)-morpholin-4-yl-methyl]-cyclohexyl}-(4-chlor-phenyl)-methyl]-dimethyl-amin
130. [{4-[(4-Benzyl-5-morpholin-4-yl-oxazol-2-yl)-morpholin-4-yl-methyl]-cyclohexyl}-(4-chlor-phenyl)-methyl]-dimethyl-amin
131. [[4-({4-Benzyl-5-[4-(4-methoxy-phenyl)-piperazin-1-yl]-oxazol-2-yl}-morpholin-4-yl-methyl)-cyclohexyl]-(4-chlor-phenyl)-methyl]-dimethyl-amin
132. [(4-{[4-Benzyl-5-(2,6-dimethyl-morpholin-4-yl)-oxazol-2-yl]-morpholin-4-yl-methyl}-cyclohexyl)-(4-chlor-phenyl)-methyl]-dimethyl-amin
133. [(4-{(4-Benzyl-5-pyrrolidin-1-yl-oxazol-2-yl)-[4-(4-fluor-phenyl)-piperazin-1-yl]-methyl}-cyclohexyl)-(4-chlor-phenyl)-methyl]-dimethyl-amin
134. [(4-{(4-Benzyl-5-pyrrolidin-1-yl-oxazol-2-yl)-[4-(4-methoxy-phenyl)-piperazin-1-yl]-methyl}-cyclohexyt)-(4-chlor-phenyl)-methyl]-dimethyl-amin
135. [(4-{(4-Benzyl-5-morpholin-4-yl-oxazol-2-yl)-[4-(4-methoxy-phenyl)-piperazin-1-yl]-methyl}-cyclohexyl)-(4-chlor-phenyl)-methyl]-dimethyl-amin
136. [(4-{[4-Benzyl-5-(2,6-dimethyl-morpholin-4-yl)-oxazol-2-yl]-[4-(4-methoxyphenyl)-piperazin-1-yl]-methyl}-cyclohexyl)-(4-chlor-phenyl)-methyl]-dimethyl-amin
137. [{4-[(4-Benzyl-5-pyrrolidin-1-yl-oxazol-2-yl)-(3,4-dihydro-1 H-isochinolin-2-yl)-methyl]-cyclohexyl}-(4-chlor-phenyl)-methyl]-dimethyl-amin
138. ({4-[(4-Benzyl-5-pyrrolidin-1-yl-oxazol-2-yl)-morpholin-4-yl-methyl]-cyclohexyl}-thiophen-2-yl-methyl)-dimethyl-amin
139. ({4-[(4-Benzyl-5-morpholin-4-yl-oxazol-2-yl)-morpholin-4-yl-methyl]-cyclohexyl}-t.hiophen-2-yl-methyl)-dimethyl-amin
140. {[4-({4-Benzyl-5-[4-(4-methoxy-phenyl)-piperazin-1-yl]-oxazol-2-yl}-morpholin-4-yl-methyl)-cyclohexyl]-thiophen-2-yl-methyl}-dimethyl-amin
141. [(4-{[4-Benzyl-5-(2,6-dimethyl-morpholin-4-yl)-oxazol-2-yl]-morpholin-4-yl-methyl}-cyclohexyl)-thiophen-2-yl-methyl]-dimethyl-amin
142. [(4.-{(4-Benzyl-5-pyrrolidin-1-yl-oxazol-2-yl)-[4-(4-fluor-phenyl)-piperazin-1-yl]-me.thyl}-cyclohexyl)-thiophen-2-yl-methyl]-dimethyl-amin
143. [(4-{(4-Benzyl-5-pyrrolidin-1-yl-oxazol-2-yl)-[4-(4-methoxy-phenyl)-piperazin-1-yl]-methyl}-cyclohexyl)-thiophen-2-yl-methyl]-dimethyl-amin
144. [(4-{(4-Benzyl-5-morpholin-4-yl-oxazol-2-yl)-[4-(4-methoxy-phenyl)-piperazin-1-yl]-methyl}-cyclohexyl)-thiophen-2-yl-methyl]-dimethyl-amin
145. [(4-{[4-Benzyl-5-(2,6-dimethyl-morpholin-4-yl)-oxazol-2-yl]-[4-(4-methoxyphenyl)-piperazin-1-yl]-methyl}-cyclohexyl)-thiophen-2-yl-methyl]-dimethyl-amin
146. ({4-[(4-Benzyl-5-pyrrolidin-1-yl-oxazol-2-yl)-(3,4-dihydro-1H-isochinolin-2-yl)-methyl]-cyclohexyl}-thiophen-2-yl-methyl)-dimethyl-amin
147. ({4-[{4-Benzyl-5-[4-(4-methoxy-phenyl)-piperazin-1-yl]-oxazol-2-yl}-(3,4-dihydro-1H-isochinolin-2-yl)-methyl]-cyclohexyl}-thiophen-2-yl-methyl)-dimethyl-amin
148. ({4-[[4-Benzyl-5-(2,6-dimethyl-morpholin-4-yl)-oxazol-2-yl]-(3,4-dihydro-1 H-isochinolin-2-yl)-methyl]-cyclohexyl}-thiophen-2-yl-methyl)-dimethyl-amin
149. [{4-[2-(4-Benzyl-5-pyrrolidin-1-yl-oxazol-2-yl)-2-morpholin-4-yl-ethyl]-cyclohexyl}-(3-fluor-phenyl)-methyl]-dimethyl-amin
150. [{4-[2-(4-Benzyl-5-morpholin-4-yl-oxazol-2-yl)-2-morpholin-4-yl-ethyl]-cyclohexyl}-(3-fluor-phenyl)-methyl]-dimethyl-amin
151. [[4-(2-{4-Benzyl-5-[4-(4-methoxy-phenyl)-piperazin-1-yl]-oxazol-2-yl}-2-morpholin-4-yl-ethyl)-cyclohexyl]-(3-fluor-phenyl)-methyl]-dimethyl-amin
152. [(4-{2-[4-Benzyl-5-(2,6-dimethyl-morpholin-4-yt)-oxazol-2-yl]-2-morpholin-4-yl-ethyl}-cyclohexyl)-(3-fluor-phenyl)-methyl]-dimethyl-amin
153. [(4-{2-[4-Benzyl-5-(2,6-dimethyl-morpholin-4-yl)-oxazol-2-yl]-2-[4-(4-fluorphenyl)-piperazin-1-yl]-ethyl}-cyclohexyl)-(3-fluor-phenyl)-methyl]-dimethyl-amin
154. [(4-{2-(4-Benzyl-5-morpholin-4-yl-oxazol-2-yl)-2-[4-(4-methoxy-phenyl)-piperazin-1-yl]-ethyl}-cyclohexyl)-(3-fluor-phenyl)-methyl]-dimethyl-amin
155. [(4-{2-{4-Benzyl-5-[4-(4-methoxy-phenyl)-piperazin-1-yl]-oxazol-2-yl}-2-[4-(4-methoxy-phenyl)-piperazin-1-yl]-ethyl}-cyclohexyl)-(3-fluor-phenyl)-methyl]-dimethyl-amin
156. [(4-{2-[4-Benzyl-5-(2,6-dimethyl-morpholin-4-yl)-oxazol-2-yl]-2-[4-(4-methoxyphenyl)-piperazin-1-yl]-ethyl}-cyclohexyl)-(3-fluor-phenyl)-methyl]-dimethyl-amin
157. [{4-[2-{4-Benzyl-5-[4-(4-methoxy-phenyl)-piperazin-1-yl]-oxazol-2-yl}-2-(3,4-dihydro-1H-isochinolin-2-yl)-ethyl]-cyclohexyl}-(3-fluor-phenyl)-methyl]-dimethyl-amin
158. ({4-[2-(4-Benzyl-5-morpholin-4-yl-oxazol-2-yl)-2-morpholin-4-yl-ethyl]-cyclohexyl}-thiophen-2-yl-methyl)-dimethyl-amin
159. {[4-(2-{4-Benzyl-5-[4-(4-methoxy-phenyl)-piperazin-1-yl]-oxazol-2-yl}-2-morpholin-4-yl-ethyl)-cyclohexyl]-thiophen-2-yl-methyl}-dimethyl-amin
160. [(4-{2-[4-Benzyl-5-(2,6-dimethyl-morpholin-4-yl)-oxazol-2-yl]-2-morpholin-4-yl-ethyl}-cyclohexyl)-thiophen-2-yl-methyl]-dimethyl-amin
161. [(4-{2-(4-Benzyl-5-morpholin-4-yl-oxazol-2-yl)-2-[4-(4-fluor-phenyl)-piperazin-1-yl]-ethyl}-cyclohexyl)-thiophen-2-yl-methyl]-dimethyl-amin
162. [(4-{2-{4-Benzyl-5-[4-(4-methoxy-phenyl)-piperazin-1-yl]-oxazol-2-yl}-2-[4-(4-fluor-phenyl)-piperazin-1-yl]-ethyl}-cyclohexyl)-thiophen-2-yl-methyl]-dimethyl-amin
163. [(4-{2-(4-Benzyl-5-pyrrolidin-1-yl-oxazol-2-yl)-2-[4-(4-methoxy-phenyl)-piperazin-1-yl]-ethyl}-cyclohexyl)-thiophen-2-yl-methyl]-dimethyl-amin
164. [(4-{2-{4-Benzyl-5-[4-(4-methoxy-phenyl)-piperazin-1-yl]-oxazol-2-yl}-2-[4-(4-methoxy-phenyl)-piperazin-1-yl]-ethyl}-cyclohexyl)-thiophen-2-y1-methyl]-dimethyl-amin
165. [(4-{2-[4-Benzyl-5-(2,6-dimethyl-morpholin-4-yl)-oxazol-2-yl]-2-[4-(4-methoxyphenyl)-piperazin-1-yl]-ethyl}-cyclohexyl)-thiophen-2-yl-methyl]-dimethyl-amin
166. ({4-[2-{4-Benzyl-5-[4-(4-methoxy-phenyl)-piperazin-1-yl]-oxazol-2-yl}-2-(3,4-dihydro-1H-isochinolin-2-yl)-ethyl]-cyclohexyl}-thiophen-2-yl-methyl)-dimethyl-amin

Ein weiterer Gegenstand der Erfindung ist ein Verfahren zur Herstellung eines erfindungsgemäßen Oxazol-Derivates. Die erfindungsgemäßen Substanzen können hergestellt werden, indem man Aldehyde der allgemeinen Formel **A** mit Aminen der allgemeinen Formel **B** und Isonitrilamiden der allgemeinen Formel **C** in einem organischen Lösungsmittel, beispielesweise Methanol oder Ethanol, 1-10 h bei einer Temperatur zwischen 30 und 100°C, vorzugsweise 40-80°C, erwärmt.

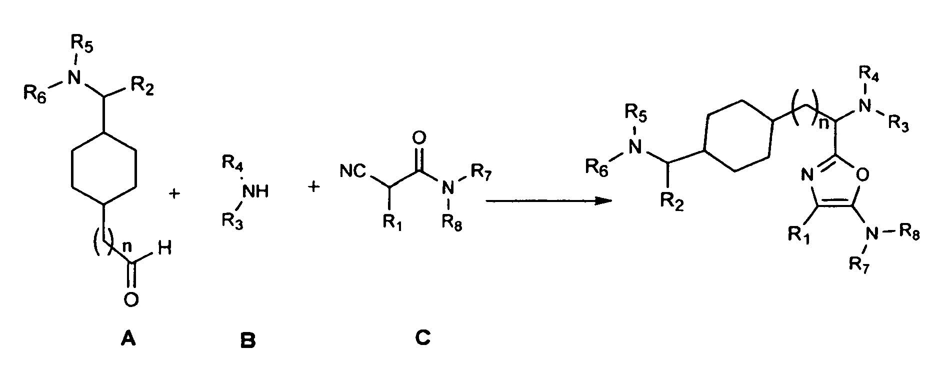

Die Komponente C kann analog der Beschreibung in K. Numani et al, Agric. Biol. Chem. 1985, 49, 10, 3023 - 3028 nach dem folgendem Reaktionsschema hergestellt werden. Nach Formulierung des entsprechenden Aminosäureesters, wie bei V. Wehner et al., Tetrahedron 2004, 60, 19, 4295 - 4302 beschrieben, wird der Isonitrilester unter wasserentziehenden Bedingungen generiert. Nach anschließender Umsetzung des Esters mit einem Amin erhält man das gewünschte Isonitrilamid.

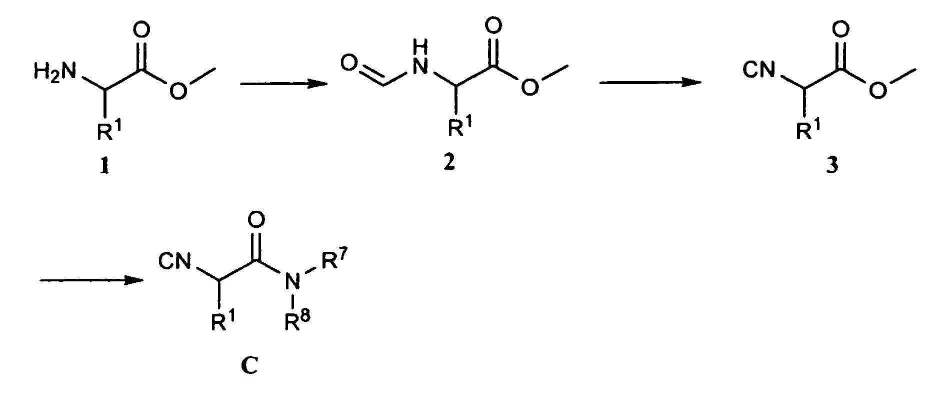

Die Amine der allgemeinen Formel **B** sind käuflich erhältlich oder werden nach dem Fachmann bekannten Methoden hergestellt.

Für die Herstellung der Aldehyde der allgemeinen Formel **A** schützt man die Ketofunktion von 4-Oxo-cyclohexancarbonsäureester, 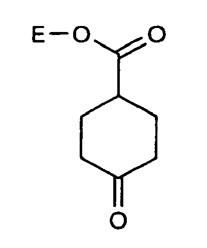 wobei E für einen C₁₋₆-Alkylrest, vorzugsweise Ethyl, steht nach dem Fachmann bekannten Methoden, 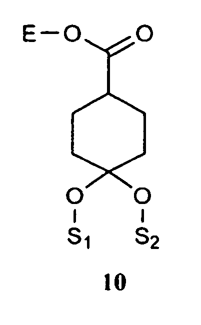 Wobei S¹ und S² jeweils für eine Schutzgruppe stehen, vorzugsweise einen Ring bilden und zusammen für -CH₂-CH₂- stehen. Der Ester **10** wird mit einem Reduktionsmittel, beispielsweise Diisobutylaluminiumhydrid zum Aldehyd **11** 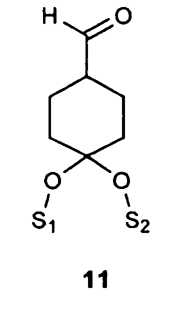 reduziert. Durch Zugabe eines Amins der allgemeinen Formel R⁵R⁶NH und eines Cyanids, beispielsweise KCN oder NaCN, wird der Aldehyd **11** unter Zugabe einer Säure, beispielsweise Salzsäure, in einem organischen Lösungsmittel, beispielsweise Methanol oder Ethanol, zum Nitril **12** umgesetzt.

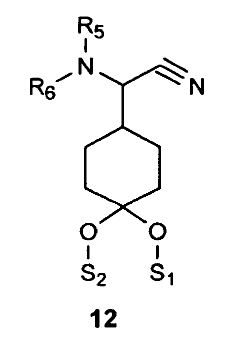

Das Nitril **12** wird mit einem Grignard-Reagenz der allgemeinen Formel R²MgHal, wobei Hal für Br, Cl oder I steht, oder einer metallorganischen Verbindung der allgemeinen Formel R²Li in einem organischen Lösungsmittel, beispielsweise Diethylether, Dioxan oder Tetrahydrofuran, zu einer Verbindung der allgemeinen Formel **13** umgesetzt.

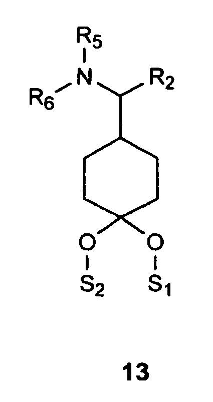

Die Schutzgruppen werden nach den üblichen Methoden abgespalten, wobei man das Keton **14** erhält.

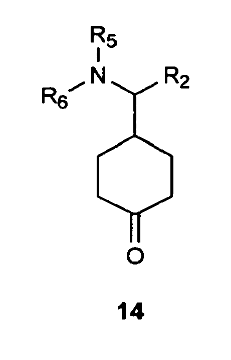

Der Aldehyd **15** 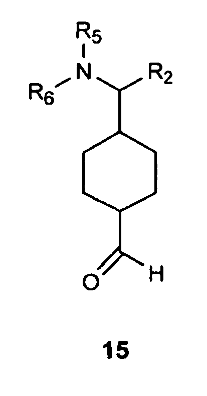 wird durch Umsetzung des Ketons **14** mit (Methoxymethyl)triphenylphosphoniumchlorid und einer starken Base, beispielsweise Kalium-tert-butylat, bei einer Temperatur von -20°C und +30°C, erhalten. Durch Umsetzung von Aldehyd **15** mit (Methoxymethyl)triphenylphosphoniumchlorid und einer starken Base, beispielsweise Kalium-tert-butylat, bei einer Temperatur von - 20°C und +30°C, wird ein Aldehyd der allgemeinen Formel **16** erhalten.

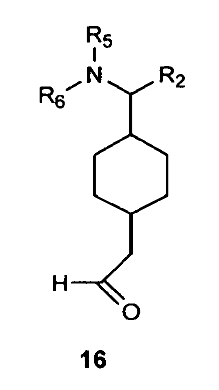

Über eine Wiederholung des letzten Reaktionsschrittes können Aldehyde erhalten werden, bei denen n für 2 steht.

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes Oxazol-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Oxazol-Derivat gegebenenfalls geeignete Zusatzund/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Oxazol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Oxazol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen Oxazol-Derivats appliziert.

Das Arzneimittel kann ein erfindungsgemäßes Oxazol-Derivat als reines Diastereomer und/oder Enantiomer, als Razemat oder als nicht-äquimolare oder äquimolare Mischung der Diastereomere und/oder Enantiomere enthalten.

Ein weiterer Gegenstand der Erfindung ist die Verwendung eines erfindungsgemäßen Oxazol-Derivats zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen Oxazol-Derivats zur Herstellung eines Arzneimittels zur Behandlung von Depressionen und/oder zur Anxiolyse.

Weiterhin lassen sich die substituierten Oxazol-Derivate der allgemeinen Formel I zur Behandlung von Harninkontinenz, Diarrhöe, Pruritus, Alkohol- und Drogenmißbrauch, Medikamentenabhängigkeit und Antriebslosigkeit einsetzen.

Gegenstand der Erfindung ist daher auch die Verwendung eines substituierten Oxazol-Derivates der allgemeinen Formel I zur Herstellung eines Arzneimittels zur Behandlung von von Harninkontinenz, Diarrhöe, Pruritus, Alkohol- und Drogenmißbrauch, Medikamentenabhängigkeit und Antriebslosigkeit.

### Beispiele

### Herstellung der Isonitrilamide

### Methyl 2-(formylamino)-3-phenylpropanoat 2i

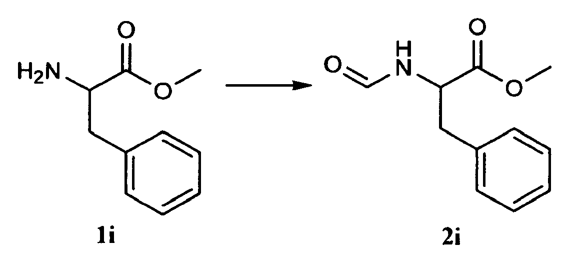

Ameisensäurecyanmethylester (Aldrich, Bestell-Nr. 453579, 5,05g, 59,3 mmol) wurden zusammen mit dem Phenylalaninmethylester Hydrochlorid (Aldrich, Bestell-Nr 525472, 12,80g, 59,3 mmol) in 80 ml Dichlormethan suspendiert und im Eisbad auf 0 °C gekühlt. Anschließend wurde Triethylamin (6,01g, 59,3 mmol) gelöst in 15 ml Dichlormethan zugegeben und 16 h bei Raumtemperatur gerührt. Es entstand eine klare Lösung.
Zur Aufarbeitung wurde mit 100 ml Dichlormethan verdünnt und zunächst mit 200 ml gesättigter NaHCO₃-Lösun und anschließend zweimal mit gesättiger NaCl-Lösung gewaschen. Die organische Phase wurde über Magnesiumsulfat getrocknet und eingeengt. Das entstandene Produkt wurde ohne weitere Aufreinigung für die nächste Stufe verwendet.
Ausbeute: 12,5 g farbloses Öl
¹H-NMR (300 MHz, CDCl₃): δ = 3,07 - 3,22 (m, 2 H); 3,74 (s, 3 H); 4,92 - 5,01 (m, 1 H); 6,20 (bs, 1 H); 7,07 - 7,15 (m, 2 H); 7,23 - 7,34 (m, 3 H); 8,15 (s, 1 H).

### 2-Isocyano-3-phenyl-propionsäuremethylester 3i

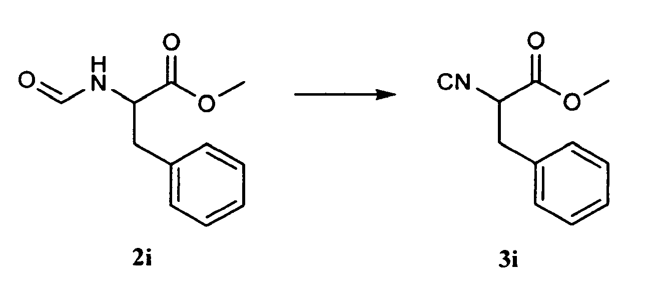

Methyl 2-(formylamino)-3-phenylpropanoat 2i (2,00g, 9,7 mmol) wurden in 20 ml Dichlormethan gelöst und auf 0 °C gekühlt. Nach der Zugabe des Triethylamins (4,88g, 48,3 mmol) zugegeben, anschließend das in 10 ml Dichlormethan gelöste Phosphoroxychlorid (2,22g, 14,5 mmol) langsam zugetropft. Die zunächst farblose Lsg. färbte sich gelblich, schließlich fiel ein oranger Feststoff aus. Nach 1 h rühren im Eisbad wurde Kaliumcarbonat-Lösung (0,8 M in Wasser, 39 ml) langsam zugetropft. Zur Aufarbeitung wurden die Phasen getrennt. Die organische Phase wurde zunächst zweimal mit je 20 ml Wasser und anschließend einmal mit 20 ml gesättigter NaCl-Lösung gewaschen. Die Lösung wurde über Natriumsulfat getrocknet und eingeengt. Das Produkt wurde ohne weitere Aufreinigung für die nächste Stufe verwendet.
Ausbeute: m = 1,55 g orangebraune Flüssigkeit
¹H-NMR (300 MHz, CDCl₃): δ = 3,14 (dd, 1H, *J*_{AA'} = 8,66 Hz, *J*_{AB} = 8,29 Hz); 3,23 (dd, 1H, *J*_{AA'} = 4,52 Hz, *J*_{AB} = 4,90 Hz); 3,80 (s, 3 H), 4,46 (dd, 1H, *J* = 8,29; *J* = 4,90 Hz); 7,22 - 7,39 (m, 5 H).

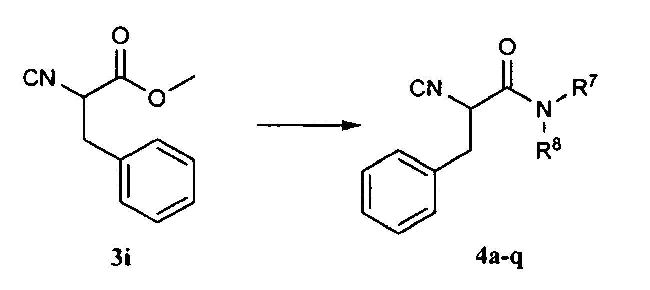

### Allgemeine Vorschrift:

2-lsocyano-3-phenyl-propionsäuremethylester 3i (13g, 68,71 mmol) und das entsprechende Amin (137,42 mmol) wurden zusammengegeben und bei Raumtemperatur gerührt. Nach abgeschlossener Reaktion (DC-Kontrolle wurde zunächst am Rotationsverdampfer eingeengt und anschließend säulenchromatographisch aufgereinigt (Laufmittel: Gradient: Hexan bis Ether:Hexan =1:1)

### 1-[(2R)-2-isocyano-3-phenylpropanoyl]piperidin 4a (NR⁷R⁸ = Piperidinyl)

¹H-NMR (300 MHz, CDCl₃): δ = 1,26 - 1,44 (m, 1H); 1,46 - 1,73 (m, 5 H); 3,10 - 3,34 (m, 2 H); 3,35 - 3,56 (m, 2 H); 3,57 - 3,69 (m, 1H); 4,66 (dd, 1H, *J* = 6,03 Hz, *J* = 9,0 Hz); 7,23 - 7,39 (m, 5 H).

### 1-[(2R)-2-isocyano-3-phenylpropanoyl]pyrrolidin 4b (NR⁷R⁸ = Pyrrolidinyl)

¹H-NMR (300 MHz, CDCl₃): δ =1,70 - 1,99 (m, 4H); 2,98 - 3,11 (m, 1H); 3,12 - 3,35 (m, 2 H); 3,36 - 3,59 (m, 3 H); 4,40 (dd, 1H, JA,B = 7,53 Hz, JA,B' = 7,16 Hz); 7,23 - 7,38 (m, 5 H).

### 4-[(2R)-2-isocyano-3-phenylpropanoyl]morpholin 4c (NR⁷R⁸ = Morpholinyl)

¹H-NMR (300 MHz, CDCl₃): δ = 3,13 - 3,52 (m, 5 H); 3,52 - 3,80 (m, 5 H); 4,47-4,61 (m, 1H); 7,19 - 7,43 (m, 5 H).

### (2R)-1-(4-Benzyl-piperidin-1-yl)-2-isocyano-3-phenyl-propan-1-on 4d (NR⁷R⁸ = 4-Benzylpiperidinyl)

¹³C-NMR (75 MHz, CDCl₃) beide Diastereomere: δ = 31,45, 31,80 (t); 37,77, 38,18 (d); 38,82, 39,25 (t); 42,68, 42,73 (t); 43,21 (t); 46,11, 46,34 (t); 55,10, 55,60 (d); 126,15 (d); 127,51, 127,60 (d); 128,36 (d); 128,74, 128,87 (d); 129,01, 129,05 (d); 129,39, 129,51 (d); 135,32, 135,53 (s); 139,55, 139,71 (s); 162,86, 163,07 (s).

### (2R)-1-(4-Benzyl-piperazin-1-yl)-2-isocyano-3-phenyl-propan-1-on 4e (NR⁷R⁸ = 4-Benzylpiperazinyl)

¹³C-NMR (75 MHz, CDCl₃): δ = 38,95 (t); 42,73 (t); 45,93 (t); 52,32 (t); 55,33 (d); 62,66 (t); 127,37 (d); 127,67 (d); 128,38 (d); 128,84 (d); 129,07 (d); 129,41 (d); 135,25 (s); 137,36 (s); 163,12 (s).

### (2R)-2-Isocyano-1-(4-methyl-piperidin-1-yl)-3-phenyl-propan-1-on 4f (NR⁷R⁸ = 4-Methylpiperidinyl)

¹³C-NMR (75 MHz, CDCl₃) beide Diastereomere: δ = 21,47 (q); 21,51 (q); 30,68 (d); 30,96 (d); 33,42 (t); 33,82 (t); 33,94 (t); 38,83 (t); 39,20 (t); 43,26 (t); 43,29 (t); 46,17 (t); 46,37 (t); 55,18 (d); 55,64 (d); 127,50 (d); 127,57 (d); 128,75 (d); 128,84 (d); 129,40 (d); 129,47 (d); 135,36 (s); 135,58 (s); 162,88 (s); 163,07 (s).

### (2R)-N-benzyl-2-isocyano-N-methyl-3-phenylpropanamid 4g (R⁷ = Methyl, R⁸ = Benzyl)

¹H-NMR (300 MHz, CDCl3): δ = 2,85 (s, 3 H); 3,11 - 3,40 (m, 2 H); 4,30 - 4,66 (m, 3 H); 7,12-7,21 (m, 2 H); 7,24-7.39 (m, 8 H).

### (2R)-2-Isocyano-3-phenyl-1-(4-phenyl-piperazin-1-yl)-propan-1-on 4h (NR⁷R⁸ = 4-Phenylpiperazinyl)

¹H-NMR (300 MHz, CDCl₃): δ = 3,06 - 3,90 (m, 10 H); 4,60 (dd, 1H, *J* = 6,78 Hz, *J* = 6,0 Hz); 6,83 - 6,98 (m, 3 H); 7,22 - 7,42 (m, 7 H).

### 1-(3,5-Dimethyl-piperidin-1-yl)-2-isocyano-3-phenyl-propan-1-on 4i (NR⁷R⁸ = 3,5-Dimethylpiperidinyl)

¹H-NMR (300 MHz, CDCl₃) beide Diastereomere: δ = 0,68 - 0,80 (m, 2H); 0,80-0,96 (m, 12H); 0,99 - 1,14 (m, 1H); 1,38 - 1,53 (m, 1H); 1,63 - 1,90 (m, 4H); 1,97 - 2,13 (m, 2H); 2,31 - 2,44 (m, 1H); 2,49 - 2,61 (m, 1H); 3,10 - 3,67 (m, 6H); 4,48 - 4,63 (m, 4H); 7,21 - 7,40 (m, 10H).

### (2R)-2-Isocyano-N-methyl-N-phenethyl-3-phenyl-propionamid 4j (R⁷ = Methyl, R⁸ = 2-Phenylethyl)

¹³C-NMR (75 MHz, CDCl₃) beide Rotamere: δ = 33,31 (t); 36,18 (q); 38,66 (t); 38,82 (t); 51,04 (t); 51,36 (t); 54,97 (d); 55,66 (d); 126,59 (d); 127,24 (d); 127,45 (d); 127,59 (d); 128,64 (d); 128,78 (d); 128,83 (d); 129,10 (d); 129,36 (d); 129,41 (d); 135,35 (s); 137,51(s); 138,41 (s); 164,36 (s); 164,82 (s).

### (2R)-N,N-Diethyl-2-isocyano-3-phenyl-propionamid 4k (R⁷ = Ethyl, R⁸ = Ethyl)

¹H-NMR (300 MHz, CDCl₃): δ = 1,10 (t, 3 H, *J* = 7,16 Hz); 1,11 (t, 3 H, *J* = 7,16 Hz); 3,09- 3,47 (m, 6 H); 4,49 (t, 1H, *J* = 7,16 Hz); 7,22- 7,38 (m, 5 H).

### 4-[(2R)-2-isocyano-3-phenylpropanoyl]thiomorpholin 4l (NR⁷R⁸ =Thiomorpholinyl)

¹H-NMR (300 MHz, CDCl₃): δ = 2,24 - 2,40 (m, 1H), 2,43 - 2,65 (m, 3 H); 2,94 - 3,17 (m, 2 H); 3,64 - 3,81 (m, 4 H); 5,28 (dd, 1H, *J* = 8,67 Hz, *J* = 6,03 Hz); 7,24 - 7,39 (m, 5 H).

### 1-[(2S)-2-isocyano-3-phenylpropanoyl]-4-(4-methoxyphenyl) piperazin 4m (NR7³R⁸ =1-(4-Methoxy-phenyl)piperazinyl)

¹H-NMR (300 MHz, CDCl₃): δ = 2,74 - 2,83 (m, 1H); 2,93 - 3,10 (m, 3 H); 3,13 - 3,88 (m, 7 H); 3,77 (s, 3 H); 4,60 (dd, 1H, *J* = 8,10 Hz, *J* = 6,59 Hz); 6,84 - 6,86 (m, 4 H); 7,26 - 7,38 (m, 5 H).

### 1-[2-isocyano-3-phenylpropanoyl]-3-methylpiperidin 4n (NR⁷R⁸ =3-Methylpiperidinyl)

¹H-NMR (300 MHz, CDCl₃) Diasteromere und Rotamere: δ = 0,82 - 0,94 (m, 3 H); 1,01 - 1,91 (m, 5 H); 2,20 - 3,76 (m, 4 H); 4,28 - 4,65 (m, 2 H); 7,23 - 7,39 (m, 5 H).

### 2-Isocyano-1-morpholin-4-yl-3-phenyl-propan-1-on 4o (NR⁷R⁸ = 2,6-Dimethylmorpholinyl)

¹H-NMR (300 MHz, CDCl₃) Diasteromere und Rotamere: δ = 1,04 - 1,27 (m, 6 H); 2,26 - 3,80 (m, 8 H); 4,34 - 4,63 (m, 2 H); 7,16 - 7,39 (m, 5 H).

### 1-[4-(2-Fluor-phenyl)-piperazin-1-yl]-2-isocyano-3-phenyl-propan-1-on 4p (NR⁷R⁸ = 1-(2-Fluor-phenyl)piperazinyl)

¹H-NMR (300 MHz, CDCl₃): δ = 2,77 - 2,85 (m, 1H); 2,96 - 3,10 (m, 3H); H); 3,16 - 3,37 (m, 2 H); 3,38 - 3, 50 (m, 1H); 3,59 - 3,77 (m, 2 H); 3,83 - 3,92 (m, 1H); 4,60 (dd, 1H, *J* = 8,10 Hz, *J* = 6,59 Hz), 6,84 - 6,91 (m, 1H); 6,96 - 7,10 (m, 3 H); 7,26 - 7,39 (m, 5 H).

### 1-[4-(4-Fluor-phenyl)-piperazin-1-yl]-2-isocyano-3-phenyl-propan-1-on 4q (NR⁷R⁸ = 1-(4-Fluor-phenyl) piperazinyl)

¹H-NMR (300 MHz, CDCl₃): δ = 2,77 - 2,85 (m, 1H); 3,00 - 3,11 (m, 3 H); 3,17 - 3,46 (m, 3 H); 3,57 - 3,77 (m, 2 H); 3,80 - 3,89 (m, 1H); 4,60 (dd, 1H, *J* = 8,10 Hz, *J* = 6,78 Hz), 6,82 - 6,88 (m, 2 H); 6,94 - 7,01 (m, 2 H); 7,25 - 7,39 (m, 5 H).

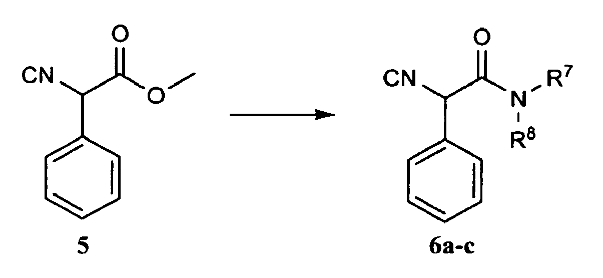

### Allgemeine Vorschrift:

Methyl 2-isocyano-2-phenylacetat **5** (13g, 68,71 mmol) und das entsprechende Amin (137,42 mmol) wurden zusammengegeben und bei Raumtemperatur gerührt. Nach abgeschlossener Reaktion (DC-Kontrolle) wurde zunächst am Rotationsverdampfer eingeengt und anschließend säulenchromatographisch aufgereinigt (Laufmittel: Gradient: Hexan bis Ether:Hexan = 1 : 1)

### 2-Isocyano-2-phenyl-1-pyrrolidin-1-yl-ethanon 6a (NR⁷R⁸ = Pyrrolidinyl)

¹H-NMR (300 MHz, CDCl₃): δ = 1,74 - 1,99 (m, 4 H); 3,25 - 3,63 (m, 4 H); 5,51 (s, 1 H); 7,38 - 7,53 (m, 5 H).

### 2-Isocyano-1-morpholin-4-yl-2-phenyl-ethanon 6b (NR⁷R⁸ = Morpholinyl)

¹H-NMR (300 MHz, CDCl₃): δ = 3,30 - 3,48 (m, 4 H); 3,59 - 3,70 (m, 4 H); 5,66 (s, 1 H); 7,37 - 7,50 (m, 5 H).

### 2-Isocyano-2-phenyl-1-(piperidin-1-yl)ethanon 6c (NR⁷R⁸ = Piperidinyl)

¹H-NMR (300 MHz, CDCl₃): δ = 1,22 - 1,36 (m, 2 H); 1,47 - 1,65 (m, 4 H); 3,29 (t, *J* = 4,90 Hz, 2 H); 3, 50 - 3,65 (m, 2 H); 5,67 (s, 1 H); 7,36 - 7,46 (m, 5 H).

### 3-(4-Chlor-phenyl)-2-isocyano-1-piperidin-1-yl-propan-1-on 8

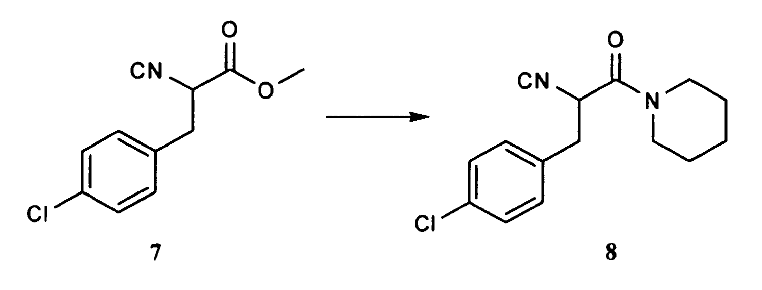

Methyl 2-isocyano-2-(4-chlorphenyl)propionat 7 (Priaton, Bestell-Nr. 224, 8 g, 35.87 mmol) und Piperidin (6,11g, 71,74 mmol) wurden zusammengegeben und bei Raumtemperatur gerührt. Nach abgeschlossener Reaktion (DC-Kontrolle) wurde zunächst am Rotationsverdampfer eingeengt und anschließend säulenchromatographisch aufgereinigt (Laufmittel: Gradient: Hexan bis Ether:Hexan =1:1)
¹H-NMR (300 MHz, CDCl₃): δ = 1,38 - 1,77 (m, 6 H); 3,08 - 3,35 (m, 3 H); 3,41 - 3,53 (m, 2 H); 3,61 - 3,73 (m, 1 H); 4,50 (dd, 1 H, *J* = 8,29 Hz, *J* = 6,03 Hz); 7,19 - 7,34 (m, 4 H).

### Synthese der Aldehyde

Die Aldehyde **15a-e** und **16a-e** wurden auf dem unten beschriebenen Weg in einer mehrstufigen Synthese aus dem kommerziell erhältlichen 4-Oxo-cyclohexancarbonsäureethylester erhalten. Die Ausbeuten der hergestellten Verbindungen sind nicht optimiert. Alle Temperaturen sind unkorrigiert.

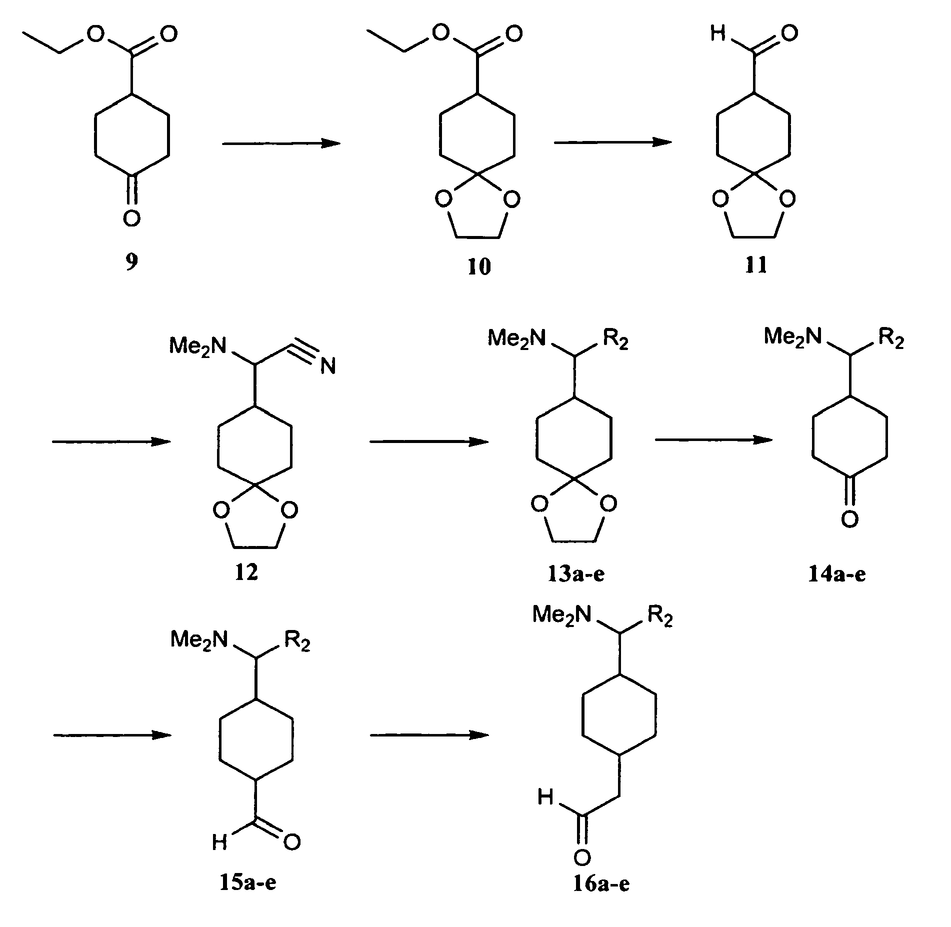

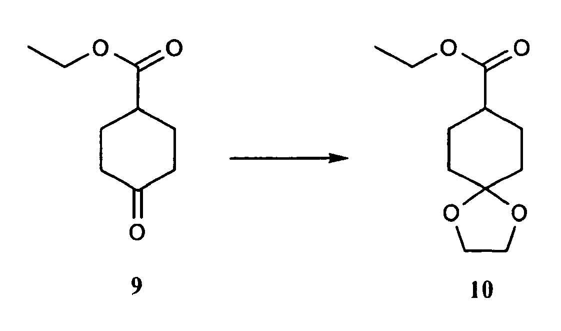

### 1,4-Dioxa-spiro[4.5]decan-8-carbonsäure-ethylester 10

4-Oxo-cyclohexancarbonsäure-ethylester 9 (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet (Na₂SO₄), i. Vak. eingeengt und die verbliebene farblose Flüssigkeit ohne Reinigung weiter verarbeitet.
Ausbeute: 66,5 g (100 %)
¹H-NMR (CDCl₃): 1,24 (t, 3 H); 1,53 (m, 2 H); 1,76 (m, 4 H); 1,92 (m, 2 H); 2,31 (m, 1 H); 3,91 (s, 4 H); 4,11 (q, 2 H).
¹³C-NMR (CDCl₃): 14,28 (q); 26,32 (t); 33,76 (t); 41,59 (d); 60,14 (t); 64,21 (t); 107,90 (d); 174,77 (s).

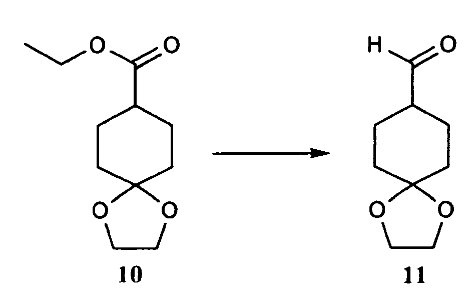

### 1,4-Dioxa-spiro[4.5]decane-8-carbaldehyd 11

Eine Lösung aus 1,4-Dioxa-spiro[4.5]decan-8-carbonsäureethylester **10** (32,13 g, 150 mmol) in absol. Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Kiesegur wurde zweimal mit Essigester gewaschen, die wäßrige Lösung abgetrennt und zweimal mit Essigester extrahiert. Die vereinigten organischen Extrakte wurden mit gesättigter Natriumchlorid-Lösung gewaschen, über Natriumsulfat getrocknet und i. Vak. eingeengt.
Ausbeute: 24,01 g (94 %), gelbes Öl
¹H-NMR (CDCl₃): 1,54 (m, 2 H); 1,74 (m, 4 H); 1,91 (m, 2 H); 2,21 (m, 1 H); 3,91 (s, 4 H); 9,60 (s, 1 H).
¹³C-NMR (CDCl₃): 23,35 (t); 33,37 (t); 48,18 (d); 64,30 (t); 107,89 (d); 203,51 (s).

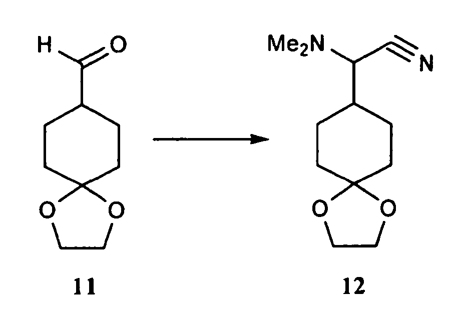

### Dimethylamino-(1,4-dioxa-spiro[4.5]dec-8-yl)-acetonitril 12

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxaspiro-[4.5]decan-8-carbaldehyd 11 (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.
Ausbeute: 25,2 g (81 %)
Schmelzpunkt: 48-51 °C.
¹H-NMR (CDCl₃): 1,23 - 2,03 (m, 9 H); 2,28 (s, 6 H); 3,16 (d, 1 H); 3,93 (m, 4 H). ¹³C-NMR (CDCl₃): 26,67 (t); 27,93 (t); 33,87 (t); 36,94 (d); 41.90 (q); 64.30 (t); 64.36 (t); 108.33 (d); 115.94 (s).

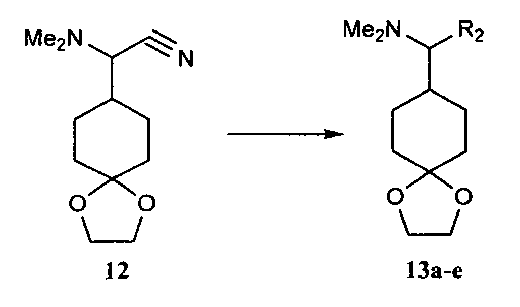

### [(1,4-Dioxa-spiro[4.5]dec-8-yl)-4-fluorphenyl-methyl]-dimethylamin 13a (R² = 4-Fluorphenyl)

Eine 1 M Lösung von 4-Fluorphenylmagnesiumbromid in THF (220 ml, 220 mmol) wurde unter Argon und Eiskühlung tropfenweise mit einer Lösung des Aminonitrils **12** (19,89 g, 88 mmol) in absol. THF (160 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet (Na₂SO₄) und eingeengt. Ausbeute: 31 g (>100 %)
¹³C-NMR (CDCl₃): 26,68 (t); 28,11 (t); 34,43 (t); 34,55 (t); 37,37 (d); 41,68 (q); 64,12 (t); 73,65 (d); 108,88 (d); 114,23 (d); 114,44 (d); 130,27; 130,35; 132,43; 160,36 (s); 162,78 (s).

### [(1,4-Dioxa-spiro[4.5]dec-8-yl)-3-fluorphenyl-methyl]-dimethyl-amin 13b (R² = 3-Fluorphenyl)

Eine 1 M Lösung von 3-Fluorphenylmagnesiumbromid in THF (208 ml, 208 mmol) wurde unter Argon und Eiskühlung tropfenweise mit einer Lösung des Aminonitrils **12** (23,45 g, 104 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,33 g (99 %).
¹H-NMR (CDCl₃): 1,12 (m, 1 H); 1,26 (m, 1 H); 1,46 - 1,81 (m, 7 H); 2,10 (s, 6 H); 3,10 (d, 1 H); 3,90 (m, 4 H); 6,85 (m, 3 H); 7,27 (m, 1 H).
¹³C-NMR (CDCl₃); 26,80 (t); 28,08 (t); 34,48 (t); 34,45 (t); 34,59 (t); 37,26 (d); 41,71 (q); 64,19 (t); 74,04 (t); 108,91 (d); 113,51 (d); 113,71 (d); 115,52 (d); 115,72 (d); 124,83 (d); 128,82 (d); 128,90 (d); 139,66 (s); 161,15 (s); 163,58 (s).

### [(4-Chlorphenyl)-(1,4-dioxa-spiro[4.5]dec-8-yl)-methyl]-dimethyl-amin 13c (R² = 4-Chlorphenyl)

Eine 1 M Lösung von 4-Chlorphenylmagnesiumbromid in Ether (200 ml, 200 mmol) wurde unter Argon und Eiskühlung tropfenweise mit einer Lösung des Aminonitrils **12** (22,43 g, 100 mmol) in absol. Ether (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,9 g (100 %)
¹³C-NMR (CDCl₃): 26,65 (t); 28,11 (t); 34,46 (t); 34,60 (t); 37,28 (d); 41,76 (q); 64,17 (t); 73,80 (d); 108,88 (s); 127,72 (d); 129,53 (d); 132,39 (d); 135,33 (d).

### [(1,4-Dioxa-spiro[4.5]dec-8-yl)-thiophen-2-yl-methyl]-dimethylamin 13d (R² = 2-Thienyl)

Eine 1M Lösung von Thiophen-2-yl-magnesiumbromid in THF (20 ml, 20 mmol) wurde unter Argon und Eiskühlung tropfenweise mit einer Lösung des Aminonitrils **12** (2,24 g, 10 mmol) in absol. THF (10 ml) versetzt und 20 h bei RT gerührt. Zur Aufarbeitung des Reaktionsgemisches wurde unter Eiskühlung gesättigte Ammoniumchlorid-Lösung (10 ml) und Wasser (10 ml) gegeben und mit Diethylether (3x10 ml) extrahiert. Die organische Phase wurde mit Wasser und gesättigter Natriumchlorid-Lösung gewaschen, getrocknet und eingeengt.
Ausbeute: 2,8 g (100 %)
¹³C-NMR (CDCl₃): 27,72 (t); 27,88 (t); 34,27 (t); 39,28 (d); 41,10 (q); 64,11 (t); 68,89 (d); 108,88 (s); 123,55 (d); 125,88 (d); 127,53 (d); 139,50 (s).

### [1-(1,4-Dioxa-spiro[4.5]dec-8-yl)-3-phenyl-propyl]-dimethylamin 13e (R² = Phenethyl)

Eine 1 M Lösung von Phenylethylmagnesiumchlorid in THF (242 ml, 242 mmol) wurde unter Argon und Eiskühlung tropfenweise mit einer Lösung des Aminonitrils **12** (21,93 g, 97 mmol) in absol. THF (180 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 34 g (>100 %).
¹³C-NMR (CDCl₃): 27,43 (t); 28,95 (t); 29,42 (t); 34,82 (t); 35,40 (t); 38,76 (d); 41,16 (q); 64,17 (t); 67,41 (d); 108,86 (s); 125,41 (d); 127,66 (d); 128,11 (d); 142,69 (s).

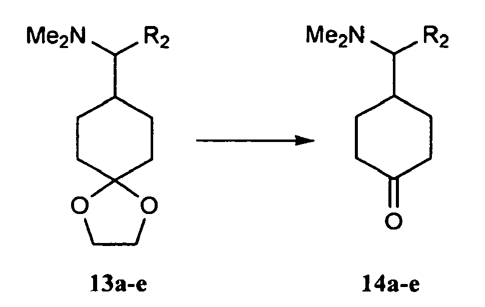

### 4-[Dimethylamino-(4-fluorphenyl)-methyl]-cyclohexanon 14a (R² = 4-Fluorphenyl)

Das Rohprodukt des Ketals **13a** (26 g, 88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt.
Ausbeute: 21,36 g (98 %)
¹³C-NMR (CDCl₃): 28,90 (t); 30,48 (t); 37,00 (t); 40,49 (t); 40,72 (t); 41,79 (q); 72,98 (d); 114,42 (d); 114,62 (d); 130,20 (d); 130,28 (d); 131,88 (s); 160,50 (s); 162,93 (s); 211,44 (s).

### 4-[Dimethylamino-(3-fluorphenyl)-methyl]-cyclohexanon 14b (R² = 3-Fluorphenyl)

Das Ketal **13b** (30,3 g, 103 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloser Feststoff isoliert.
Ausbeute: 22,4 g (87 %)
Schmelzpunkt: 72-75 °C.
¹³C-NMR (CDCl₃): 28,97 (t); 30,44 (t); 36,90 (t); 40,52 (t); 40,75 (t); 41,82 (q); 73,37 (d); 113,84; 114,06; 115,42; 115,62; 124,71; 129,03; 129,11; 139,00; 139,06; 161,16; 163,60; 211,40 (s).

### 4-[(4-Chlor-phenyl)-dimethylamino-methyl]-cyclohexanon 14c (R² = 4-Chlorphenyl)

Das Ketal 13c (30,98 g, 100 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als Öl isoliert.
Ausbeute: 21,9 g (82 %)
¹³C-NMR (CDCl₃): 28,88 (t); 30,45 (t); 36,89 (t); 40,49 (t); 40,74 (t); 41,83 (q); 73,12 (d); 127,87 (d); 130,16 (d); 132,75 (d); 13470 (s); 211,35 (s).

### 4-(Dimethylamino-thiophen-2-yl-methyl)-cyclohexanon 14d (R² = 2-Thienyl)

Das Ketal **13d** (2,80 g, 10 mmol) wurde in Wasser (4,4 ml) gelöst, mit konz. Salzsäure (6,4 ml) versetzt und 20 h bei RT gerührt. Das Reaktionsgemisch wurde mit Diethylether (2x10 ml) ausgeschüttelt, die wässrige Phase unter Eiskühlung mit 5N NaOH alkalisch gestellt, mit Dichlormethan (3x10 ml) extrahiert, getrocknet und eingeengt. Das Keton **14d** wurde als Öl isoliert.
Ausbeute: 1,79 g (75 %)
¹³C-NMR (CDCl₃): 30,02 (t); 30,18 (t); 38,84 (t); 40,29 (t); 39,28 (d); 41,17 (q); 68,24 (d); 123,88 (d); 126,01 (d); 126,34 (d); 138,77 (d); 211,49 (s).

### 4-(1-Dimethylamino-3-phenyl-propyl)-cyclohexanon 14e (R² = Phenethyl)

Das Rohprodukt des Ketals **13e** (29,6 g, 97 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.
Ausbeute: 16.9 g (58 %)
¹³C-NMR (CDCl₃): 29,40 (t); 30,02 (t); 30,97 (t); 35,34 (t); 38,71 (t); 40,79 (t); 41,01 (t); 41,23 (q); 66,65 (d); 125,66 (d); 128,12 (d); 128,19 (d); 142,27 (s); 211,70 (s).

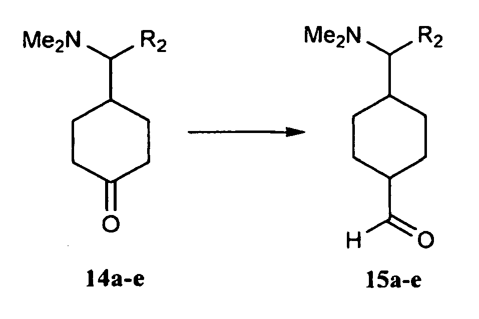

### 4-[Dimethylamino-(4-fluorphenyl)-methyl]-cyclohexan-carbaldehyd 15a (R² = 4-Fluorphenyl)

(Methoxymethyl)triphenylphosphoniumchlorid (25,7 g, 75 mmol) wurde in absol. THF (100 ml) unter Argon suspendiert, bei 0 °C tropfenweise mit Kalium-tert-butylat (8,42 g, 75 mmol), gelöst in absol. THF (70 ml) versetzt und anschließend 15 min bei 0 °C nachgerührt.
Bei RT wurde dann das Keton **14a** (12,44 g, 50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt. Ausbeute: 9,13 g (70 %).
¹H-NMR (DMSO, 600 MHz, ausgesuchte Signale): δ = 1,97 (s, 3 H); 1,99 (s, 3 H); 3,08 (d, 1 H, *J* = 9,06 Hz); 3,14 (d, 1 H, *J* = 9,82 Hz); 9,53 (s, 1 H); 9,56 (s, 1 H). ¹³C-NMR (CDCl₃, beide Diastereomere): δ = 23,97; 24,21; 25,85; 26,02; 26,17; 27,35; 28,00; 29,90; 37,26; 38,34; 41,50; 41,95; 47,36; 50,55; 72,75; 75,84; 114,25; 114,45; 130,33; 130,40; 132,61; 160,41; 162,83; 204,10; 204,93.

### 4-[Dimethylamino-(3-fluorphenyl)-methyl]-cyclohexan-carbaldehyd 15b (R² = 3-Fluorphenyl)

(Methoxymethyl)triphenylphosphoniumchlorid (15,42 g, 45 mmol) wurde in absol. THF (50 ml) unter Argon suspendiert, bei 0 °C tropfenweise mit Kalium-*tert*-butylat (5,05 g, 45 mmol), gelöst in absol. THF (50 ml), versetzt und anschließend 15 min bei 0 °C nachgerührt.
Bei RT wurde dann das Keton **14b** (7,48 g, 0.30 mmol), gelöst in absol.THF (50 ml), zur obigen Lösung zugetropft und über Nacht bei RT gerührt. Unter Eiswasserkühlung wurde tropfenweise mit Wasser (25 ml) und 6N HCl (75 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über Na₂SO₄ getrocknet und i. Vak. eingeengt. Das Rohprodukt wurde über durch Flashchromatographie mit Essigester/Cyclohexan (1:1) gereinigt. Ausbeute: 6.55 g (83 %).
Schmelzpunkt: 40-43 °C.
¹H-NMR (DMSO, 600 MHz, ausgesuchte Signale): δ = 1,99 (s, 3 H); 2,01 (s, 3 H); 3,10 (d, 1 H, *J* = 9,06 Hz); 3,18 (d, 1 H, *J* = 9,82 Hz); 9,54 (s, 1 H); 9,56 (s, 1 H). ¹³C-NMR (CDCl₃): 23,93; 24,12; 25,79; 25,95; 26,19; 27,19; 27,99; 29,77; 37,05; 38,16; 41,45; 41,91; 47,30; 50,49; 71,50; 74,78; 113,50; 115,37; 124,78; 128,24; 130,59;131,24; 131,67; 139,14; 139,76; 160,06; 163,50; 204,01; 204,85.

### 4-[(4-Chlor-phenyl)-dimethylamino-methyl]-cyclohexancarbaldehyd 15c (R² = 4-Chlorphenyl)

(Methoxymethyl)triphenylphosphoniumchlorid (68,55 g, 200 mmol) wurde in absol. THF (200 ml) unter Argon suspendiert, bei 0 °C tropfenweise mit Kalium-tert-butylat (22,44 g, 200 mmol), gelöst in absol. THF (300 ml), versetzt und anschließend 15 min bei 0 °C nachgerührt.
Bei RT wurde dann das Keton **14c** (38 g, 143 mmol), gelöst in absol. THF (200 ml), zur obigen Lösung getropft und über Nacht bei RT gerührt. Unter Eiswasserkühlung wurde tropfenweise mit Wasser (150 ml) und 6N HCl (450 ml) hydrolysiert. Nach 1 h Rühren bei RT wurde mit Ether (10 x 100 ml) extrahiert, die wässrige Phase mit 5N NaOH auf pH 11 gebracht, mit Essigester (3 x 100 ml) ausgeschüttelt, über Na₂SO₄ getrocknet und i. Vak. eingeengt. Das Rohprodukt wurde über zwei Kieselgelsäulen (400 g) mit Essigester/Cyclohexan (1:1) gereinigt.
Ausbeute: 32.17 g (80 %).
¹H-NMR (DMSO, 600 MHz, ausgesuchte Signale): δ = 1,97 (s, 3 H); 1,99 (s, 3 H); 3,07 (d, 1 H, *J* = 9,07 Hz); 3,14 (d, 1 H, *J* = 9,82 Hz); 9,53 (s, 1 H); 9,55 (s, 1 H). ¹³C-NMR (CDCl₃ beide Diastereomere): δ = 23,92; 24,16; 25,80; 25,97; 26,13; 27,25; 27,90; 29,81; 37,08; 38,19; 41,47; 41,96; 47,29; 50,48; 72,81; 74,54 ; 127,65 ; 130,28; 132,40; 134,78; 135,43; 203.98; 204.82.

### 4-(Dimethylamino-thiophen-2-yl-methyl)-cyclohexancarbaldehyd 15d (R² = 2-Thienyl)

(Methoxymethyl)triphenylphosphoniumchlorid (20,56 g, 60 mmol) wurde in absol. THF (70 ml) unter Argon suspendiert, bei 0 °C tropfenweise mit Kalium-tert-butylat (6,73 g, 60 mmol), gelöst in absol. THF (70 ml), versetzt und anschließend 15 min bei 0 °C nachgerührt Bei RT wurde dann das Keton **14d** (9,4 g, 40 mmol), gelöst in absol. THF (70 ml), zur obigen Lösung zugetropft und über Nacht bei RT gerührt. Unter Eiswasserkühlung wurde tropfenweise mit Wasser (60 ml) und 6 N HCl (180 ml) hydrolysiert. Nach 1 h Rühren bei RT wurde mit Ether (5 x 50 ml) extrahiert, die wässrige Phase mit 5 N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt. Ausbeute: 7.66 g (77 %).
¹H-NMR (DMSO, 600 MHz, ausgesuchte Signale): δ = 2,03 (s, 3 H); 2,05 (s, 3 H); 3,44 (d, 1 H, *J* = 9,82 Hz); 3,52 (d, 1 H, *J* = 10,58 Hz); 9,54 (s, 1 H); 9,58 (s, 1 H).
¹³C-NMR (CDCl₃, beide Diastereomere): δ= 23,74; 23,83; 25,80; 25,84; 26,98; 27,09; 29,15; 29,68; 39,13; 40,20; 40,98; 41,29 (N(CH₃)₂); 47,48; 50,49; 67,81; 69,79; 123,61; 123,70; 125,89; 126,20; 126,24; 139,14; 139,48; 204,07; 204,82.

### 4-(1-Dimethylamino-3-phenyl-propyl)-cyclohexan-carbaldehyd 15e (R² = Phenethyl)

(Methoxymethyl)triphenylphosphoniumchlorid (20,56 g, 60 mmol) wurde in absol. THF (85 ml) unter Argon suspendiert, bei 0 °C tropfenweise mit Kalium-tert-butylat (6,73 g, 60 mmol), gelöst in absol. THF (70 ml) versetzt und anschließend 15 min bei 0 °C nachgerührt.
Bei RT wurde dann das Keton **14e** (10,2 g, 40 mmol), gelöst in absol. THF (60 ml), zur obigen Lösung getropft und über Nacht bei RT gerührt. Unter Eiswasserkühlung wurde tropfenweise mit Wasser (35 ml) und 6N HCl (90 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über Na₂SO₄ getrocknet und i. Vak. eingeengt. Das Rohprodukt wurde durch Flashchromatographie mit Essigester/Cyclohexan (1:1) gereinigt.
Ausbeute: 6.73 g (63 %).
¹H-NMR (DMSO, 600 MHz, ausgesuchte Signale): δ = 2,18 (s, 3 H); 2,20 (s, 3 H); 9,54 (s, 1 H); 9,61 (s, 1 H).
¹³C-NMR (CDCl₃, beide Diastereomere): δ = 24,35; 24,49; 26,00; 26,09; 26,85; 27,79; 29,07; 29,13; 35,27; 39,02; 40,98; 41,19; 46,99; 50,33; 66,85; 67,85; 70,54; 71,42; 125,40; 125,44; 128,02; 128,13; 131,15; 131,17 ; 142,45; 204,10; 205,01.

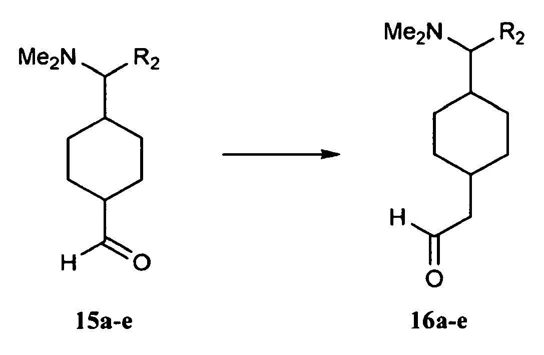

### {4-[Dimethylamino-(4-fluorphenyl)-methyl]-cyclohexyl}-acetaldehyd 16a (R² = 4-Fluorphenyl)

(Methoxymethyl)triphenylphosphoniumchlorid (43,53 g, 127 mmol) wurde in absol. THF (200 ml) unter Argon suspendiert, bei 0 °C tropfenweise mit Kalium-tert-butylat (14,25 g, 127 mmol), gelöst in absol. THF (130 ml), versetzt und anschließend 15 min bei 0 °C nachgerührt.
Bei RT wurde dann der Aldehyd **15a** (22,3 g, 85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Ether (je 100 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flash-chromatographie mit Essigester/Cyclohexan (1:1) gereinigt.
Ausbeute: 15,8 g (67 %)
¹³C-NMR (CDCl₃, beide Diastereomere): δ = 25,08; 25,87; 28,80; 29,10; 29,13; 29,62; 30,82; 32,90; 33,08; 36,19; 38,43; 41,36; 42,01; 47,94; 51,17; 71,11; 74,69; 114,11; 114,20; 114,32; 130,32; 130,40; 132,00; 132,92; 160,31; 162,74; 202,15; 202,23.

### {4-[Dimethylamino-(3-fluorphenyl)-methyl]-cyclohexyl}-acetaldehyd 16b (R² = 3-Fluorphenyl)

(Methoxymethyl)triphenylphosphoniumchlorid (26,73 g, 78 mmol) wurde in absol. THF (90 ml) unter Argon suspendiert, bei 0 °C tropfenweise mit Kalium-tert-butylat (8,75 g, 78 mmol), gelöst in absol. THF (90 ml), versetzt und anschließend 15 min bei 0 °C nachgerührt .
Bei RT wurde dann der Aldehyd **15b** (13,69 g, 52 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 12.61 g (87 %)
¹³C-NMR (CDCl₃, beide Diastereomere): δ = 25,19; 25,83; 28,90; 29,06; 29,14; 29,68; 30,77; 32,92; 32,98; 33,10; 36,05; 38,36; 41,39; 42,04; 48,02; 51,20; 71,48; 75,07; 113,43; 113,49; 113,64; 113,69; 115,55; 115,76; 124,89; 128,70; 128,78; 128,88; 139,24; 140,08; 140,14; 161,09; 163,52; 202,19; 202,27.

### {4-[(4-Chlorphenyl)-dimethylamino-methyl]-cyclohexyl}-acetaldehyd 16c (R² = 4-Chlorphenyl)

(Methoxymethyl)triphenylphosphoniumchlorid (25,02 g, 73 mmol) wurde in absol. THF (90 ml) unter Argon suspendiert, bei 0 °C tropfenweise mit Kalium-tert-butylat (8,19 g, 73 mmol), gelöst in absol. THF (90 ml), versetzt und anschließend 15 min bei 0 °C nachgerührt.
Bei RT wurde dann der Aldehyd **15c** (13,86 g, 49 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 12.07 g (84 %).
¹³C-NMR (CDCl₃, beide Diastereomere): δ = 25,06; 25,82; 28,74; 29,00; 29,13; 29,60; 30,77; 32,87; 32,94; 33,07; 36,06; 38,32; 41,38; 42,05; 47,95; 51,17; 71,23; 74,80; 127,58; 127,66; 130,31; 132,28; 132,34; 134,81; 135,77; 202,12; 202,20.

### {4-[Dimethylamino-thiophen-2-yl-methyl]-cyclohexyl}-acetaldehyd 16d (R² = 2-Thienyl)

(Methoxymethyl)triphenylphosphoniumchlorid (28,79 g, 84 mmol) wurde in absol. THF (100 ml) unter Argon suspendiert, bei 0 °C tropfenweise mit Kalium-tert-butylat (9,42 g, 84 mmol), gelöst in absol. THF (100 ml), versetzt und anschließend 15 min bei 0 °C nachgerührt.
Bei RT wurde dann der Aldehyd **15d** (14,08 g, 56 mmol), gelöst in absol. THF (100 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:2) gereinigt.
Ausbeute: 11.48 g (77 %).
¹³C-NMR (CDCl₃, beide Diastereomere): δ = 25,80; 25,88; 28,73; 29,95; 30,49, 32,23; 32,76; 37,89; 40,21; 40,88; 41,23; 48,36; 51,09; 66,02; 69,97; 123,19; 123,72; 125,95; 126,31; 139,42; 139,91; 202,61 .

### [4-(1-Dimethylamino-3-phenyl-propyl)-cyclohexyl]-acetaldehyd 16e (R² = Phenethyl)

(Methoxymethyl)triphenylphosphoniumchlorid (50,3 g, 147 mmol) wurde in absol. THF (150 ml) unter Argon suspendiert, bei 0 °C tropfenweise mit Kalium-tert-butylat (16,5 g, 147 mmol), gelöst in absol. THF (140 ml), versetzt und anschließend 15 min bei 0 °C nachgerührt.
Bei RT wurde dann der Aldehyd **15e** (27,0 g, 98 mmol), gelöst in absol. THF (150 ml), zugetropft und über Nacht bei RT gerührt. Unter Eiswasserkühlung wurde tropfenweise mit Wasser (102 ml) und 6N HCl (240 ml) hydrolysiert. Nach 1 h Rühren bei RT wurde fünfmal mit Ether (je 200 ml) extrahiert. Die wässrige Phase wurde unter Eiskühlung mit 5 N NaOH auf pH 11 gebracht, dreimal mit Essigester (je 200 ml) ausgeschüttelt, über Na₂SO₄ getrocknet und i. Vak. eingeengt. Das Rohprodukt wurde durch Flash-chromatographie mit Essigester/Cyclohexan (1:1) gereinigt.
Ausbeute: 18,1 g (64 %)
¹³C-NMR (CDCl₃, beide Diastereomere): δ = 25,55; 26,19; 29,04; 29,15; 29,35; 29,85; 31,00; 32,87; 32,68; 33,04; 35,33; 38,49; 40,86; 41,13; 47,51; 51,15; 65,48; 68,09; 125,58; 128,20; 142,79; 202,69.

### Automatisierte Synthese

Die Verbindungen wurden mit Hilfe der automatisierten Synthese mit Hilfe des Accelerators SLT106 der Firma Chemspeed Ltd. nach der folgenden Versuchsvorschrift hergestellt:
In einen trockenen 13 ml double-jacket Glasreaktor wurden bei Raumtemperatur 120 µmol (1.2ml, 0,1 M in Methanol) Aldehydlösung (Lösung II), 130 µmol (1.3ml, 0.1 M in Methanol) Aminlösung (Lösung III) und 100 µmol (1 ml, 0,1 M in Methanol) Isonitrilamid-Derivat (Lösung I) gegeben. Die Reaktionslösung wurde 6 h bei 60°C erhitzt und geschüttelt. Nach beendeter Reaktion wurde die Reaktionslösung in einer GeneVac eingeengt. Die Aufreinigung erfolgte über HPLC.

Die folgenden Verbindungen wurden nach der vorstehenden allgemeinen Vorschrift hergestellt. Die Analytik erfolgte über HPLC-MS. In allen Fällen wurde die exakte Masse als M+1 gefunden.

| **Nr.** | **Name** | **Masse** |
|---|---|---|
| 17. | [{4-[(4-Benzyl-5-pyrrolidin-1-yl-oxazol-2-yl)-piperidin-1-yl-methyl]-cyclohexyl}-(4-fluor-phenyl)-methyl]-dimethyl-amin | 558,37 |
| 18. | ((4-Benzyl-5-morpholin-4-yl-oxazol-2-yl)-{4-[dimethylamino-(4-fluor-phenyl)-methyl]-cyclohexyl}-methyl)-methyl-phenyl-amin | 596,35 |
| 19. | ([4-Benzyl-5-(4-methyl-piperidin-1-yl)-oxazol-2-yl]-{4-[dimethylamino-(4-fluor-phenyl)-methyl]-cyclohexyl}-methyl)-methyl-phenyl-amin | 608,39 |
| 20. | ([4-Benzyl-5-(4-methyl-piperidin-1-yl)-oxazol-2-yl]-{4-[(4-chlor-phenyl)-dimethylamino-methyl]-cyclohexyl}-methyl)-diethyl-amin | 590,38 |
| 21. | Benzyl-[4-benzyl-2-({4-[(4-chlor-phenyl)-dimethylamino-methyl]-cyclohexyl}-diethylamino-methyl)-oxazol-5-yl]-methyl-amin | 612,36 |
| 22. | Benzyl-{4-benzyl-2-[{4-[(4-chlor-phenyl)-dimethylamino-methyl]-cyclohexyl}-(methyl-phenyl-amino)-methyl]-oxazol-5-yl}-methyl-amin | 646,34 |
| 23. | [(4-{[4-Benzyl-5-(4-benzyl-piperidin-1-yl)-oxazol-2-yl]-piperidin-1-yl-methyl}-cyclohexyl)-thiophen-2-yl-methyl]-dimethyl-amin | 650,40 |
| 24. | [(4-{[4-Benzyl-5-(4-methyl-piperidin-1-yl)-oxazol-2-yl]-piperidin-1-yl-methyl}-cydohexyl)-thiophen-2-yl-methyl]-dimethyl-amin | 574,37 |
| 25. | Benzyl-(4-benzyl-2-{[4-(dimethylamino-thiophen-2-yl-methyl)-cyclohexyl]-piperidin-1-yl-methyl}-oxazol-5-yl)-methyl-amin | 596,35 |
| 26. | [(4-{[4-Benzyl-5-(4-phenyl-piperazin-1-yl)-oxazol-2-yl]-piperidin-1-yl-methyl}-cyclohexyl)-thiophen-2-yl-methyl]-dimethyl-amin | 637,38 |
| 27. | {[4-Benzyl-5-(4-benzyl-piperidin-1-yl)-oxazol-2-yl]-[4-(dimethylamino-thiophen-2-yl-methyl)-cyclohexyl]-methyl}-diethyl-amin | 638,40 |
| 28. | {[4-Benzyl-5-(4-benzyl-piperazin-1-yl)-oxazol-2-yl]-[4-(dimethylamino-thiophen-2-yl-methyl)-cyclohexyl]-methyl}-diethyl-amin | 639,40 |
| 29. | {[4-Benzyl-5-(4-methyl-piperidin-1-yl)-oxazol-2-yl]-[4-(dimethylamino-thiophen-2-yl-methyl)-cyclohexyl]-methyl}-diethyl-amin | 562, 37 |
| 30. | Benzyl-(4-benzyl-2-{diethylamino-[4-(dimethylamino-thiophen-2-yl-methyl)-cyclohexyl]-methyl}-oxazol-5-yl)-methyl-amin | 584,35 |
| 31. | {[4-Benzyl-5-(4-phenyl-piperazin-1-yl)-oxazol-2-yl]-[4-(dimethylamino-thiophen-2-yl-methyl)-cyclohexyl]-methyl}-diethyl-amin | 625,38 |
| 32. | ({4-[[4-Benzyl-5-(4-benzyl-piperidin-1-yl)-oxazol-2-yl]-(4-benzyl-piperazin-1-yl)-methyl]-cyclohexyl}-thiophen-2-yl-methyl)-dimethyl-amin | 741,44 |
| 33. | [(4-{[4-Benzyl-5-(3-methyl-piperidin-1-yl)-oxazol-2-yl]-piperidin-1-yl-methyl}-cyclohexyl)-(4-fluor-phenyl)-methyl]-dimethyl-amin | 586,40 |
| 34. | [4-Benzyl-2-(diethylamino-{4-[dimethylamino-(4-fluor-phenyl)-methyl]-cyclohexyl}-methyl)-oxazol-5-yl]-methyl-phenethyl-amin | 610,40 |
| 35. | [4-Benzyl-2-(diethylamino-{4-[dimethylamino-(4-fluor-phenyl)-methyl]-cyclohexyl}-methyl)-oxazol-5-yl]-diethyl-amin | 548,39 |
| 36. | ([4-Benzyl-5-(3-methyl-piperidin-1-yl)-oxazol-2-yl]-{4-[dimethylamino-(4-fluor-phenyl)-methyl]-cyclohexyl}-methyl)-diethyl-amin | 574,40 |
| 37. | ([4-Benzyl-5-(3,5-dimethyl-piperidin-1-yl)-oxazol-2-yl]-{4-[dimethylamino-(4-fluor-phenyl)-methyl]-cyclohexyl}-methyl)-methyl-phenyl-amin | 622,40 |
| 38. | {4-Benzyl-2-[{4-[dimethylamino-(4-fluor-phenyl)-methyl]-cyclohexyl}-(methyl-phenyl-amino)-methyl]-oxazol-5-yl}-methyl-phenethyl-amin | 644,39 |
| 39. | [4-Benzyl-2-({4-[(4-chlor-phenyl)-dimethylamino-methyl]-cyclohexyl}-piperidin-1-yl-methyl)-oxazol-5-yl]-diethyl-amin | 576,36 |
| 40. | [(4-{[4-Benzyl-5-(3-methyl-piperidin-1-yl)-oxazol-2-yl]-piperidin-1-yl-methyl}-cyclohexyl)-(4-chlor-phenyl)-methyl]-dimethyl-amin | 602,38 |
| 41. | ([4-Benzyl-5-(3,5-dimethyl-piperidin-1-yl)-oxazol-2-yl]-{4-[(4-chlor-phenyl)-dimethylamino-methyl]-cyclohexyl}-methyl)-diethyl-amin | 604,39 |
| 42. | ([4-Benzyl-5-(3,5-dimethyl-piperidin-1-yl)-oxazol-2-yl]-{4-[(4-chlor-phenyl)-dimethylamino-methyl]-cyclohexyl}-methyl)-methyl-phenyl-amin | 638,38 |
| 43. | {4-Benzyl-2-[{4-[(4-chlor-phenyl)-dimethylamino-methyl]-cyclohexyl}-(methyl-phenyl-amino)-methyl]-oxazol-5-yl}-methyl-phenethyl-amin | 660,36 |
| 44. | [4-Benzyl-2-((4-benzyl-piperazin-1-yl)-{4-[(4-chlor-phenyl)-dimethylamino-methyl]-cyclohexyl}-methyl)-oxazol-5-yl]-diethyl-amin | 667,40 |
| 45. | [(4-{[4-Benzyl-5-(3,5-dimethyl-piperidin-1-yl)-oxazol-2-yl]-piperidin-1-yl-methyl}-cyclohexyl)-thiophen-2-yl-methyl]-dimethyl-amin | 588, 39 |
| 46. | (4-Benzyl-2-{[4-(dimethylamino-thiophen-2-yl-methyl)-cyclohexyl]-piperidin-1-yl-methyl}-oxazol-5-yl)-methyl-phenethyl-amin | 610,37 |
| 47. | (4-Benzyl-2-{[4-(dimethylamino-thiophen-2-yl-methyl)-cyclohexyl]-piperidin-1-yl-methyl}-oxazol-5-yl)-diethyl-amin | 548,35 |
| 48. | ({4-[(4-Benzyl-5-thiomorpholin-4-yl-oxazol-2-yl)-piperidin-1-yl-methyl]-cyclohexyl}-thiophen-2-yl-methyl)-dimethyl-amin | 578,31 |
| 49. | (4-Benzyl-2-{diethylamino-[4-(dimethytamino-thiophen-2-yl-methyl)-cyclohexyl]-methyl}-oxazol-5-yl)-methyl-phenethyl-amin | 598, 37 |
| 50. | (4-Benzyl-2-{diethylamino-[4-(dimethylamino-thiophen-2-yl-methyl)-cyclohexyl]-methyl}-oxazol-5-yl)-diethyl-amin | 536,35 |
| 51. | {[4-Benzyl-5-(3-methyl-piperidin-1-yl)-oxazol-2-yl]-[4-(dimethylamino-thiophen-2-yl-methyl)-cyclohexyl]-methyl}-diethyl-amin | 562,37 |
| 52. | {(4-Benzyl-5-thiomorpholin-4-yl-oxazol-2-yl)-[4-(dimethylamino-thiophen-2-yl-methyl)-cyclohexyl]-methyl}-methyl-phenyl-amin | 600,30 |
| 53. | ({4-[[4-Benzyl-5-(3,5-dimethyl-piperidin-1-yl)-oxazol-2-yl]-(4-benzyl-piperazin-1-yl)-methyl]-cyclohexyl}-thiophen-2-yl-methyl)-dimethyl-amin | 679,43 |
| 54. | (4-Benzyl-2-{(4-benzyl-piperazin-1-yl)-[4-(dimethylamino-thiophen-2-yl-methyl)-cyclohexyl]-methyl}-oxazol-5-yl)-methyl-phenethyl-amin | 701,41 |
| 55. | ({4-[(4-Benzyl-piperazin-1-yl)-(4-benzyl-5-thiomorpholin-4-yl-oxazol-2-yl)-methyl]-cyclohexyl}-thiophen-2-yl-methyl)-dimethyl-amin | 669,35 |
| 56. | [(4-{2-[4-Benzyl-5-(4-benzyl-piperazin-1-yl)-oxazol-2-yl]-2-piperidin-1-yl-ethyl}-cyclohexyl)-(4-chlor-phenyl)-methyl]-dimethyl-amin | 693,42 |
| 57. | [(4-{2-[4-Benzyl-5-(4-methyl-piperidin-1-yl)-oxazol-2-yl]-2-piperidin-1-yl-ethyl}-cyclohexyl)-(4-chlor-phenyl)-methyl]-dimethyl-amin | 616,39 |
| 58. | Benzyl-[4-benzyl-2-(2-{4-[(4-chlor-phenyl)-dimethylamino-methyl]-cyclohexyl}-1-piperidin-1-yl-ethyl)-oxazol-5-yl]-methyl-amin | 638,38 |
| 59. | (1-[4-Benzyl-5-(4-benzyl-piperidin-1-yl)-oxazol-2-yl]-2-{4-[(4-chlor-phenyl)-dimethylamino-methyl]-cyclohexyl}-ethyl)-diethyl-amin | 680,42 |
| 60. | (1-[4-Benzyl-5-(4-methyl-piperidin-1-yl)-oxazol-2-yl]-2-{4-[(4-chlor-phenyl)-dimethylamino-methyl]-cyclohexyl}-ethyl)-diethyl-amin | 604,39 |
| 61. | Benzyl-[4-benzyl-2-(2-{4-[(4-chlor-phenyl)-dimethylamino-methyl]-cyclohexyl}-1-diethylamino-ethyl)-oxazol-5-yl]-methyl-amin | 626,38 |
| 62. | [(4-[2-[4-Benzyl-5-(4-benzyl-piperidin-1-yl)-oxazol-2-yl]-2-(4-benzyl-piperazin-1-yl)-ethyl]-cyclohexyl}-(4-chlor-phenyl)-methyl]-dimethyl-amin | 783,46 |
| 63. | [{4-[2-[4-Benzyl-5-(4-benzyl-piperazin-1-yl)-oxazol-2-yl]-2-(4-benzyl-piperazin-1-yl)-ethyl]-cyclohexyl}-(4-chlor-phenyl)-methyl]-dimethyl-amin | 784,46 |
| 64. | [{4-[2-[4-Benzyl-5-(4-methyl-piperidin-1-yl)-oxazol-2-yl]-2-(4-benzyl-piperazin-1-yl)-ethyl]-cyclohexyl}-(4-chlor-phenyl)-methyl]-dimethyl-amin | 707,43 |
| 65. | [(4-{2-[4-Benzyl-5-(4-methyl-piperidin-1-yl)-oxazol-2-yl]-2-piperidin-1-yl-ethyl}-cyclohexyl)-(4-fluor-phenyl)-methyl]-dimethyl-amin | 600,42 |
| 66. | Benzyl-[4-benzyl-2-(2-{4-[dimethylamino-(4-fluor-phenyl)-methyl]-cyclohexyl}-1-piperidin-1-yl-ethyl)-oxazol-5-yl]-methyl-amin | 622,40 |
| 67. | (1-[4-Benzyl-5-(4-benzyl-piperidin-1-yl)-oxazol-2-yl]-2-{4-[dimethylamino-(4-fluor-phenyl)-methyl]-cyclohexyl}-ethyl)-diethyl-amin | 664,45 |
| 68. | (1-[4-Benzyl-5-(4-benzyl-piperazin-1-yl)-oxazol-2-yl]-2-{4-[dimethylamino-(4-fluor-phenyl)-methyl]-cyclohexyl}-ethyl)-diethyl-amin | 665,45 |
| 69. | (1-[4-Benzyl-5-(4-methyl-piperidin-1-yl)-oxazol-2-yl]-2-{4-[dimethylamino-(4-fluor-phenyl)-methyl]-cyclohexyl}-ethyl)-diethyl-amin | 588,42 |
| 70. | Benzyl-[4-benzyl-2-(1-diethylamino-2-{4-[dimethylamino-(4-fluor-phenyl)-methyl]-cyclohexyl}-ethyl)-oxazol-5-yl]-methyl-amin | 610,40 |
| 71. | [{4-[2-[4-Benzyl-5-(4-methyl-piperidin-1-yl)-oxazol-2-yl]-2-(4-benzyl-piperazin-1-yl)-ethyl]-cyclohexyl}-(4-fluor-phenyl)-methyl]-dimethyl-amin | 691,46 |
| 72. | [(4-{2-[4-Benzyl-5-(4-benzyl-piperidin-1-yl)-oxazol-2-yl]-2-piperidin-1-yl-ethyl}-cyclohexyl)-thiophen-2-yl-methyl]-dimethyl-amin | 664,42 |
| 73. | [(4-{2-[4-Benzyl-5-(4-benzyl-piperazin-1-yl)-oxazol-2-yl]-2-piperidin-1-yl-ethyl}-cyclohexyl)-thiophen-2-yl-methyl]-dimethyl-amin | 665,41 |
| 74. | [(4-{2-[4-Benzyl-5-(4-methyl-piperidin-1-yl)-oxazol-2-yl]-2-piperidin-1-yl-ethyl}-cyclohexyl)-thiophen-2-yl-methyl]-dimethyl-amin | 588,39 |
| 75. | Benzyl-(4-benzyl-2-{2-[4-(dimethylamino-thiophen-2-yl-methyl)-cyclohexyl]-1-piperidin-1-yl-ethyl}-oxazol-5-yl)-methyl-amin | 610,37 |
| 76. | [(4-{2-[4-Benzyl-5-(4-phenyl-piperazin-1-yl)-oxazol-2-yl]-2-piperidin-1-yl ethyl}-cyclohexyl)-thiophen-2-yl-methyl]-dimethyl-amin | 651,40 |
| 77. | {1-[4-Benzyl-5-(4-benzyl-piperidin-1-yl)-oxazol-2-yl]-2-[4-(dimethylamino-thiophen-2-yl-methyl)-cyclohexyl]-ethyl}-diethyl-amin | 652,42 |
| 78. | {1-[4-Benzyl-5-(4-benzyl-piperazin-1-yl)-oxazol-2-yl]-2-[4-(dimethylamino-thiophen-2-yl-methyl)-cyclohexyl]-ethyl}-diethyl-amin | 653,41 |
| 79. | {1-[4-Benzyl-5-(4-methyl-piperidin-1-yl)-oxazol-2-yl]-2-[4-(dimethylamino-thiophen-2-yl-methyl)-cyclohexyl]-ethyl}-diethyl-amin | 576,39 |
| 80. | Benzyl-(4-benzyl-2-{1-diethylamino-2-[4-(dimethylamino-thiophen-2-yl-methyl)-cyclohexyl]-ethyl}-oxazol-5-yl)-methyl-amin | 598,37 |
| 81. | ({4-[2-[4-Benzyl-5-(4-benzyl-piperidin-1-yl)-oxazol-2-yl]-2-(4-benzyl-piperazin-1-yl)-ethyl]-cyclohexyl}-thiophen-2-yl-methyl)-dimethyl-amin | 755,46 |
| 82. | ({4-[2-[4-Benzyl-5-(4-benzyl-piperazin-1-yl)-oxazol-2-yl]-2-(4-benzyl-piperazin-1-yl)-ethyl]-cyclohexyl}-thiophen-2-yl-methyl)-dimethyl-amin | 756,45 |
| 83. | ({4-[2-[4-Benzyl-5-(4-methyl-piperidin-1-yl)-oxazol-2-yl]-2-(4-benzyl-piperazin-1-yl)-ethyl]-cyclohexyl}-thiophen-2-yl-methyl)-dimethyl-amin | 679,43 |
| 84. | Benzyl-(4-benzyl-2-{1-(4-benzyl-piperazin-1-yl)-2-[4-(dimethylamino-thiophen-2-yl-methyl)-cyclohexyl]-ethyl}-oxazol-5-yl)-methyl-amin | 701,41 |
| 85. | ({4-[2-[4-Benzyl-5-(4-phenyl-piperazin-1-yl)-oxazol-2-yl]-2-(4-benzyl-piperazin-1-yl)-ethyl]-cyclohexyl}-thiophen-2-yl-methyl)-dimethyl-amin | 742,44 |
| 86. | [4-Benzyl-2-(2-{4-[(4-chlor-phenyl)-dimethylamino-methyl]-cyclohexyl}-1-piperidin-1-yl-ethyl)-oxazol-5-yl]-methyl-phenethyl-amin | 652,39 |
| 87. | [4-Benzyl-2-(2-{4-[(4-chlor-phenyl)-dimethylamino-methyl]-cyclohexyl}-1-piperidin-1-yl-ethyl)-oxazol-5-yl]-diethyl-amin | 590,38 |
| 88. | (1-[4-Benzyl-5-(3,5-dimethyl-piperidin-1-yl)-oxazol-2-yl]-2-{4-[(4-chlor-phenyl)-dimethylamino-methyl]-cyclohexyl}-ethyl)-diethyl-amin | 618,41 |
| 89. | [4-Benzyl-2-(2-{4-[(4-chlor-phenyl)-dimethylamino-methyl]-cyclohexyl}-1-diethylamino-ethyl)-oxazol-5-yl]-methyl-phenethyl-amin | 640,39 |
| 90. | [4-Benzyl-2-(2-{4-[(4-chlor-phenyl)-dimethylamino-methyl]-cyclohexyl}-1-diethylamino-ethyl)-oxazol-5-yl]-diethyl-amin | 578,38 |
| 91. | (1-(4-Benzyl-5-thiomorpholin-4-yl-oxazol-2-yl)-2-{4-[(4-chlor-phenyl)-dimethylamino-methyl]-cyclohexyl}-ethyl)-diethyl-amin | 608,33 |
| 92. | (1-(4-Benzyl-5-thiomorpholin-4-yl-oxazol-2-yl)-2-{4-[(4-chlor-phenyl)-dimethylamino-methyl]-cyclohexyl}-ethyl)-methyl-phenyl-amin | 642,32 |
| 93. | [4-Benzyl-2-(1-(4-benzyl-piperazin-1-yl)-2-{4-[(4-chlor-phenyl)-dimethylamino-methyl]-cyclohexyl}-ethyl)-oxazol-5-yl]-methyl-phenethyl-amin | 743,43 |
| 94. | [4-Benzyl-2-(1-(4-benzyl-piperazin-1-yl)-2-{4-[(4-chlor-phenyl)-dimethylamino-methyl]-cyclohexyl}-ethyl)-oxazol-5-yl]-diethyl-amin | 681,42 |
| 95. | [4-Benzyl-2-(2-{4-[dimethylamino-(4-fluor-phenyl)-methyl]-cyclohexyl}-1-piperidin-1-yl-ethyl)-oxazol-5-yl]-methyl-phenethyl-amin | 636,42 |
| 96. | (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-diethyl-amin | 602,44 |
| 97. | [4-Benzyl-2-(1-diethylamino-2-{4-[dimethylamino-(4-fluor-phenyl)-methyl]-cyclohexyl}-ethyl)-oxazol-5-yl]-methyl-phenethyl-amin | 624,42 |
| 98. | [4-Benzyl-2-(1-diethylamino-2-{4-[dimethylamino-(4-fluor-phenyl)-methyl]-cyclohexyl}-ethyl)-oxazol-5-yl]-diethyl-amin | 562,40 |
| 99. | (1-(4-Benzyl-5-thiomorpholin-4-yl-oxazol-2-yl)-2-{4-[dimethylamino-(4-fluor-phenyl)-methyl]-cyclohexyl}-ethyl)-diethyl-amin | 592,36 |
| 100. | (1-[4-Benzyl-5-(3-methyl-piperidin-1-yl)-oxazol-2-yl]-2-{4-[dimethylamino-(4-fluor-phenyl)-methyl]-cyclohexyl}-ethyl)-diethyl-amin | 588,42 |
| 101. | (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-methyl-phenyl-amin | 636,42 |
| 102. | {4-Benzyl-2-[2-{4-[dimethylamino-(4-fluor-phenyl)-methyl]-cyclohexyl}-1-(methyl-phenyl-amino)-ethyl]-oxazol-5-yl}-methyl-phenethyl-amin | 658,40 |
| 103. | {4-Benzyl-2-[2-{4-[dimethylamino-(4-fluor-phenyl)-methyl]-cyclohexyl}-1-(methyl-phenyl-amino)-ethyl]-oxazol-5-yl}-diethyl-amin | 596,39 |
| 104. | (1-(4-Benzyl-5-thiomorpholin-4-yl-oxazol-2-yl)-2-{4-[dimethylamino-(4-fluor-phenyl)-methyl]-cyclohexyl}-ethyl)-methyl-phenyl-amin | 626,35 |
| 105. | (1-[4-Benzyl-5-(3-methyl-piperidin-1-yl)-oxazol-2-yl]-2-{4-[dimethylamino-(4-fluor-phenyl)-methyl]-cyclohexyl}-ethyl)-methyl-phenyl-amin | 622,40 |
| 106. | [{4-[2-[4-Benzyl-5-(3-methyl-piperidin-1-yl)-oxazol-2-yl]-2-(4-benzyl-piperazin-1-yl)-ethyl]-cyclohexyl}-(4-fluor-phenyl)-methyl]-dimethyl-amin | 691,46 |
| 107. | [(4-{2-[4-Benzyl-5-(3,5-dimethyl-piperidin-1-yl)-oxazol-2-yl]-2-piperidin-1-yl-ethyl}-cyclohexyl)-thiophen-2-yl-methyl]-dimethyl-amin | 602,40 |
| 108. | (4-Benzyl-2-{2-[4-(dimethylamino-thiophen-2-yl-methyl)-cyclohexyl]-1-piperidin-1-yl-ethyl}-oxazol-5-yl)-methyl-phenethyl-amin | 624,39 |
| 109. | (4-Benzyl-2-{2-(4-(dimethylamino-thiophen-2-yl-methyl)-cyclohexyl)-1-piperidin-1-yl-ethyl}-oxazol-5-yl)-diethyl-amin | 562,37 |
| 110. | ({4-[2-(4-Benzyl-5-thiomorpholin-4-yl-oxazol-2-yl)-2-piperidin-1-yl-ethyl]-cyclohexyl}-thiophen-2-yl-methyl)-dimethyl-amin | 592,33 |
| 111. | [(4-{2-[4-Benzyl-5-(3-methyl-piperidin-1-yl)-oxazol-2-yl]-2-piperidin-1-yl-ethyl}-cyclohexyl)-thiophen-2-yl-methyl]-dimethyl-amin | 588,39 |
| 112. | {1-[4-Benzyl-5-(3,5-dimethyl-piperidin-1-yl)-oxazol-2-yl]-2-[4-(dimethylamino-thiophen-2-yl-methyl)-cyclohexyl]-ethyl}-diethyl-amin | 590,40 |
| 113. | (4-Benzyl-2-{1-diethylamino-2-[4-(dimethylamino-thiophen-2-yl-methyl)-cyclohexyl]-ethyl}-oxazol-5-yl)-methyl-phenethyl-amin | 612,39 |
| 114. | (4-Benzyl-2-{1-diethylamino-2-[4-(dimethylamino-thiophen-2-yl-methyl)-cyclohexyl]-ethyl}-oxazol-5-yl)-diethyl-amin | 550,37 |
| 115. | {1-(4-Benzyl-5-thiomorpholin-4-yl-oxazol-2-yl)-2-[4-(dimethylamino-thiophen-2-yl-methyl)-cyclohexyl]-ethyl}-diethyl-amin | 580, 33 |
| 116. | {1-[4-Benzyl-5-(3-methyl-piperidin-1-yl)-oxazol-2-yl]-2-[4-(dimethylamino-thiophen-2-yl-methyl)-cyclohexyl]-ethyl}-diethyl-amin | 576,39 |
| 117. | {1-[4-Benzyl-5-(3-methyl-piperidin-1-yl)-oxazol-2-yl]-2-[4-(dimethylamino-thiophen-2-yl-methyl)-cyclohexyl]-ethyl}-methyl-phenyl-amin | 610, 37 |
| 118. | ({4-[2-(4-Benzy)-piperazin-1-yl)-2-(4-benzy)-5-thiomorphotin-4-yl-oxazol-2-yl)-ethyl]-cyclohexyl}-thiophen-2-yl-methyl)-dimethyl-amin | 683,37 |
| 119. | ({4-[2-[4-Benzyl-5-(3-methyl-piperidin-1-yl)-oxazol-2-yl]-2-(4-benzyl-piperazin-1-yl)-ethyl]-cyclohexyl}-thiophen-2-yl-methyl)-dimethyl-amin | 679,43 |
| 120. | [{4-[(4-Benzyl-5-pyrrolidin-1-yl-oxazol-2-yl)-morpholin-4-yl-methyl]-cyclohexyl}-(3-fluor-phenyl)-methyl]-dimethyl-amin | 560,35 |
| 121. | [{4-[(4-Benzyl-5-morpholin-4-yl-oxazol-2-yl)-morpholin-4-yl-methyl]-cyclohexyl}-(3-fluor-phenyl)-methyl]-dimethyl-amin | 576,35 |
| 122. | [(4-{(4-Benzyl-5-pyrrolidin-1-yl-oxazol-2-yl)-[4-(4-fluor-phenyl)-piperazin-1-yl]-methyl)-cyclohexyl)-(3-fluor-phenyl)-methyl]-dimethyl-amin | 653,39 |
| 123. | [(4-{(4-Benzyl-5-pyrrolidin-1-yl-oxazol-2-yl)-[4-(4-methoxy-phenyl)-piperazin-1-yl]-methyl}-cyclohexyl)-(3-fluor-phenyl)-methyl]-dimethyl-amin | 665,41 |
| 124. | [(4-{(4-Benzyl-5-morpholin-4-yl-oxazol-2-yl)-[4-(4-methoxy-phenyl)-piperazin-1-yl]-methyl}-cyclohexyl)-(3-fluor-phenyl)-methyl]-dimethyl-amin | 681,41 |
| 125. | [{4-[(4-Benzyl-5-pyrrolidin-1-yl-oxazol-2-yl)-(3,4-dihydro-1 H-isochinolin-2-yl)-methyl]-cyclohexyl}-(3-fluor-phenyl)-methyl]-dimethyl-amin | 606,37 |
| 126. | [{4-[(4-Benzyl-5-morpholin-4-yl-oxazol-2-yl)-(3,4-dihydro-1H-isochinolin-2-yl)-methyl]-cyclohexyl}-(3-fluor-phenyl)-methyl]-dimethyl-amin | 622,37 |
| 127. | [{4-[{4-Benzyl-5-[4-(4-methoxy-phenyl)-piperazin-1-yl]-oxazol-2-yl}-(3,4-dihydro-1H-isochinolin-2-yl)-methyl]-cyclohexyl}-(3-fluor-phenyl)-methyl]-dimethyl-amin | 727,43 |
| 128. | [{4-[[4-Benzyl-5-(2,6-dimethyl-morpholin-4-yl)-oxazol-2-yl]-(3,4-dihydro-1H-isochinolin-2-yl)-methyl]-cyclohexyl}-(3-fluor-phenyl)-methyl]-dimethyl-amin | 650,40 |
| 129. | [{4-[(4-Benzyl-5-pyrrolidin-1-yl-oxazol-2-yl)-morpholin-4-yl-methyl]-cyclohexyl}-(4-chlor-phenyl)-methyl]-dimethyl-amin | 576,32 |
| 130. | [{4-[(4-Benzyl-5-morpholin-4-yl-oxazol-2-yl)-morpholin-4-yl-methyl]-cyclohexyl}-(4-chlor-phenyl)-methyl]-dimethyl-amin | 592,32 |
| 131. | [[4-({4-Benzyl-5-[4-(4-methoxy-phenyl)-piperazin-1-yl]-oxazol-2-yl}-morpholin-4-yl-methyl)-cyclohexyl]-(4-chlor-phenyl)-methyl]-dimethyl-amin | 697,38 |
| 132. | [(4-{[4-Benzyl-5-(2,6-dimethyl-morpholin-4-yl)-oxazol-2-yl]-morpholin-4-yl-methyl}-cyclohexyl)-(4-chlor-phenyl)-methyl]-dimethyl-amin | 620,35 |
| 133. | [(4-{(4-Benzyl-5-pyrrolidin-1-yl-oxazol-2-yl)-[4-(4-fluor-phenyl)-piperazin-1-yl]-methyl}-cyclohexyl)-(4-chlor-phenyl)-methyl]-dimethyl-amin | 669,36 |
| 134. | [(4-{(4-Benzyl-5-pyrrolidin-1-yl-oxazol-2-yl)-[4-(4-methoxy-phenyl)-piperazin-1-yl]-methyl}-cyclohexyl)-(4-chlor-phenyl)-methyl]-dimethyl-amin | 681,38 |
| 135. | [(4-{(4-Benzyl-5-morpholin-4-yl-oxazol-2-yl)-[4-(4-methoxy-phenyl)-piperazin-1-yl]-methyl}-cyclohexyl)-(4-chlor-phenyl)-methyl]-dimethyl-amin | 697,38 |
| 136. | [(4-{[4-Benzyl-5-(2,6-dimethyl-morpholin-4-yl)-oxazol-2-yl]-[4-(4-methoxy-phenyl)-piperazin-1-yl]-methyl}-cyclohexyl)-(4-chlor-phenyl)-methyl]-dimethyl-amin | 725,41 |
| 137. | [{4-[(4-Benzyl-5-pyrrolidin-1-yl-oxazol-2-yl)-(3,4-dihydro-1 H-isochinolin-2-yl)-methyl]-cyclohexyl}-(4-chlor-phenyl)-methyl]-dimethyl-amin | 622,34 |
| 138. | ({4-[(4-Benzyl-5-pyrrolidin-1-yl-oxazol-2-yl)-morpholin-4-yl-methyl]-cyclohexyl}-thiophen-2-yl-methyl)-dimethyl-amin | 548,32 |
| 139. | ({4-[(4-Benzyl-5-morpholin-4-yl-oxazol-2-yl)-morpholin-4-yl-methyl]-cyclohexyl}-thiophen-2-yl-methyl)-dimethyl-amin | 564,31 |
| 140. | {[4-({4-Benzyl-5-[4-(4-methoxy-phenyl)-piperazin-1-yl]-oxazol-2-yl}-morpholin-4-yl-methyl)-cyclohexyl]-thiophen-2-yl-methyl}-dimethyl-amin | 669,37 |
| 141. | [(4-{[4-Benzyl-5-(2,6-dimethyl-morpholin-4-yl)-oxazol-2-yl]-morpholin-4-yl-methyl}-cyclohexyl)-thiophen-2-yl-methyl]-dimethyl-amin | 592,34 |
| 142. | [(4-{(4-Benzyl-5-pyrrolidin-1-yl-oxazol-2-yl)-[4-(4-fluor-phenyl)-piperazin-1-yl]-methyl}-cyclohexyl)-thiophen-2-yl-methyl]-dimethyl-amin | 641,36 |
| 143. | [(4-{(4-Benzyl-5-pyrrolidin-1-yl-oxazol-2-yl)-[4-(4-methoxy-phenyl)-piperazin-1-yl]-methyl}-cyclohexyl)-thiophen-2-yl-methyl]-dimethyl-amin | 653,38 |
| 144. | [(4-{(4-Benzyl-5-morpholin-4-yl-oxazol-2-yl)-[4-(4-methoxy-phenyl)-piperazin-1-yl]-methyl}-cyclohexyl)-thiophen-2-yl-methyl]-dimethyl-amin | 669,37 |
| 145. | [(4-{[4-Benzyl-5-(2,6-dimethyl-morpholin-4-yl)-oxazol-2-yl]-[4-(4-methoxy-phenyl)-piperazin-1-yl]-methyl}-cyclohexyl)-thiophen-2-yl-methyl]-dimethyl-amin | 697,40 |
| 146. | ({4-[(4-Benzyl-5-pyrrolidin-1-yl-oxazol-2-yl)-(3,4-dihydro-1H-isochinolin-2-yl)-methyl]-cyclohexyl}-thiophen-2-yl-methyl)-dimethyl-amin | 594,34 |
| 147. | ({4-[{4-Benzyl-5-[4-(4-methoxy-phenyl)-piperazin-1-yl]-oxazol-2-yl}-(3,4-dihydro-1H-isochinolin-2-yl)-methyl]-cyclohexyl}-thiophen-2-yl-methyl)-dimethyl-amin | 715,39 |
| 148. | ({4-[[4-Benzyl-5-(2,6-dimethyl-morpholin-4-yl)-oxazol-2-yl]-(3,4-dihydro-1 H-isochinolin-2-yl)-methyl]-cyclohexyl}-thiophen-2-yl-methyl)-dimethyl-amin | 638,37 |
| 149. | [{4-[2-(4-Benzyl-5-pyrrolidin-1-yl-oxazol-2-yl)-2-morpholin-4-yl-ethyl]-cyclohexyl}-(3-fluor-phenyl)-methyl]-dimethyl-amin | 574,37 |
| 150. | [{4-[2-(4-Benzyl-5-morpholin-4-yl-oxazol-2-yl)-2-morpholin-4-yl-ethyl]-cyclohexyl}-(3-fluor-phenyl)-methyl]-dimethyl-amin | 590,36 |
| 151. | [[4-(2-{4-Benzyl-5-[4-(4-methoxy-phenyl)-piperazin-1-yl]-oxazol-2-yl}-2-morpholin-4-yl-ethyl)-cyclohexylJ-(3-fluor-phenyl)-methyl]-dimethyl-amin | 695,42 |
| 152. | [(4-{2-[4-Benzyl-5-(2,6-dimethyl-morpholin-4-yl)-oxazol-2-yl]-2-morpholin-4-yl-ethyl}-cyclohexyl)-(3-fluor-phenyl)-methyl]-dimethyl-amin | 618,39 |
| 153. | [(4-{2-[4-Benzyl-5-(2,6-dimethyl-morpholin-4-yl)-oxazol-2-yl]-2-[4-(4-fluor-phenyl)-piperazin-1-yl]-ethyl}-cyclohexyl)-(3-fluor-phenyl)-methyl]-dimethyl-amin | 711,43 |
| 154. | [(4-{2-(4-Benzyl-5-morpholin-4-yl-oxazol-2-yl)-2-[4-(4-methoxy-phenyl)-piperazin-1-yl]-ethyl}-cyclohexyl)-(3-fluor-phenyl)-methyl]-dimethyl-amin | 695,42 |
| 155. | [(4-{2-(4-Benzyl-5-[4-(4-methoxy-phenyl)-piperazin-1-yl]-oxazol-2-yl)-2-[4-(4-methoxy-phenyl)-piperazin-1-yl]-ethyl}-cyclohexyl)-(3-fluor-phenyl)-methyl]-dimethyl-amin | 800,48 |
| 156. | [(4-{2-[4-Benzyl-5-(2,6-dimethyl-morpholin-4-yl)-oxazol-2-yl]-2-[4-(4-methoxy-phenyl)-piperazin-1-yl]-ethyl}-cyclohexyl)-(3-fluor-phenyl)-methyl]-dimethyl-amin | 723,45 |
| 157. | [{4-[2-{4-Benzyl-5-[4-(4-methoxy-phenyl)-piperazin-1-yl]-oxazol-2-yl}-2-(3,4-dihydro-1H-isochinolin-2-yl)-ethyl]-cyclohexyl}-(3-fluor-phenyl)-methyl]-dimethyl-amin | 741,44 |
| 158. | ({4-[2-(4-Benzyl-5-morpholin-4-yl-oxazol-2-yl)-2-morpholin-4-yl-ethyl]-cyclohexyl}-thiophen-2-yl-methyl)-dimethyl-amin | 578,33 |
| 159. | {[4-(2-{4-Benzyl-5-[4-(4-methoxy-phenyl)-piperazin-1-yl]-oxazol-2-yl}-2-morpholin-4-yl-ethyl)-cyclohexyl]-thiophen-2-yl-methyl}-dimethyl-amin | 683,39 |
| 160. | [(4-{2-[4-Benzyl-5-(2,6-dimethyl-morpholin-4-yl)-oxazol-2-yl]-2-morpholin-4-yl-ethyl}-cyclohexyl)-thiophen-2-yl-methyl]-dimethyl-amin | 606,36 |
| 161. | [(4-{2-(4-Benzyl-5-morpholin-4-yl-oxazol-2-yl)-2-[4-(4-fluor-phenyl)-piperazin-1-yl]-ethyl}-cyclohexyl)-thiophen-2-yl-methyl]-dimethyl-amin | 671,37 |
| 162. | [(4-{2-{4-Benzyl-5-[4-(4-methoxy-phenyl)-piperazin-1-yl]-oxazol-2-yl}-2-[4-(4-fluor-phenyl)-piperazin-1 -yl]-ethyl}-cyclohexyl)-thiophen-2-yl-methyl]-dimethyl-amin | 776,42 |
| 163. | [(4-{2-(4-Benzyl-5-pyrrolidin-1-yl-oxazol-2-yl)-2-[4-(4-methoxy-phenyl)-piperazin-1-yl]-ethyl}-cyclohexyl)-thiophen-2-yl-methyl]-dimethyl-amin | 667,39 |
| 164. | [(4-{2-{4-Benzyl-5-[4-(4-methoxy-phenyl)-piperazin-1-yl]-oxazol-2-yl}-2-[4-(4-methoxy-phenyl)-piperazin-1-yl]-ethyl}-cyclohexyl)-thiophen-2-yl-methyl]-dimethyl-amin | 788,44 |
| 165. | [(4-{2-[4-Benzyl-5-(2,6-dimethyl-morpholin-4-yl)-oxazol-2-yl]-2-[4-(4-methoxy-phenyl)-piperazin-1-yl]-ethyl}-cyclohexyl)-thiophen-2-yl-methyl]-dimethyl-amin | 711,42 |
| 166. | ({4-[2-{4-Benzyl-5-[4-(4-methoxy-phenyl)-piperazin-1-yl]-oxazol-2-yl}-2-(3,4-dihydro-1 H-isochinolin-2-yl)-ethyl]-cyclohexyl}-thiophen-2-yl-methyl)-dimethyl-amin | 729,41 |

### Untersuchungen zur Wirksamkeit der erfindungsgemäßen Verbindungen

Die in den folgenden Assays erhobenen Daten sind in der Tabelle 1 zusammengefaßt.

### Methode zur Bestimmung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15 - 40 µg Protein / 250 µl Inkubationsansatz) von CHO-K1-Zellen, welche den humanen µ-Opiatrezeptor exprimieren (RB-HOM-Rezeptormembran-Präparation von Fa. NEN, Zaventem, Belgien) in Gegenwart von 1 nmol/l des radioaktiven Liganden [³H]-Naloxon (NET719, Fa. NEN, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 % Natriumazid und mit 0,06 % bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem ß-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben.

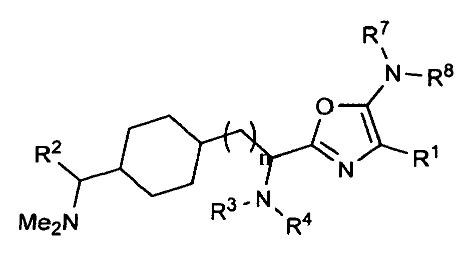

| | n | R² | R³ | R⁴ | R¹ | R⁷ | R⁸ | µ-Opioid-Rezeptor, %Hemmung [1µM] |
|---|---|---|---|---|---|---|---|---|
| 17 | 0 | 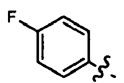 | ]-(CH₂)₅-[ | | CH₂Ph | ]-(CH₂)₄-[ | | 76 |
| 18 | 0 | 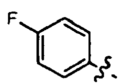 | Me | Ph | CH₂Ph | ]-(CH₂)₂-O-(CH₂)₂-[ | | 65 |
| 19 | 0 | 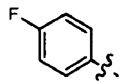 | Me | Ph | CH₂Ph | 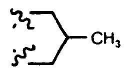 | | 63 |
| 20 | 0 | 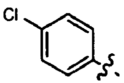 | Et | Et | CH₂Ph | 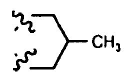 | | 63 |
| 21 | 0 | 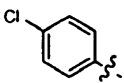 | Et | Et | CH₂Ph | Me | CH₂Ph | 72 |
| 22 | 0 | 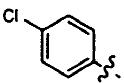 | Me | CH₂Ph | CH₂Ph | Me | CH₂Ph | 64 |
| 23 | 0 | 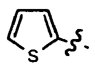 | ]-(CH₂)₅-[ | | CH₂Ph | 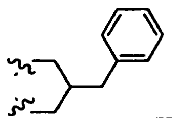 | | 70 |
| 24 | 0 | 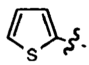 | ]-(CH₂)₅-[ | | CH₂Ph | 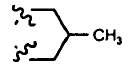 | | 70 |
| 25 | 0 | 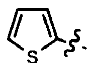 | ]-(-CH₂)₅-[ | | CH₂Ph | Me | CH₂Ph | 80 |
| 26 | 0 | 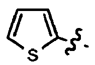 | ]-(CH₂)₅-[ | | CH₂Ph | 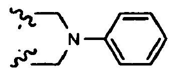 | | 85 |
| 27 | 0 | 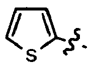 | Et | Et | CH₂Ph | 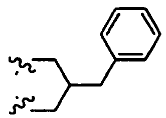 | | 78 |
| 28 | 0 | 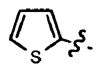 | Et | Et | CH₂Ph | 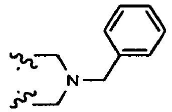 | | 72 |
| 29 | 0 | 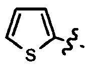 | Et | Et | CH₂Ph | 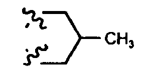 | | 95 |
| 30 | 0 | 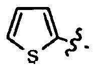 | Et | Et | CH₂Ph | Me | CH₂Ph | 92 |
| 31 | 0 | 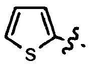 | Et | Et | CH₂Ph | 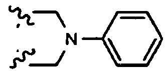 | | 62 |
| 32 | 0 | 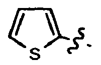 | 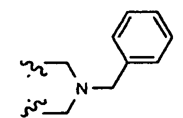 | | CH₂Ph | 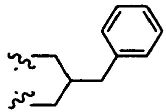 | | 75 |
| 33 | 0 | 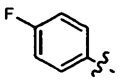 | ]-(CH₂)₅-[ | | CH₂Ph | 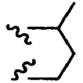 | | 65 |
| 34 | 0 | 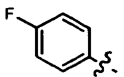 | Et | Et | CH₂Ph | Me | (CH₂)₂P h | 84 |
| 35 | 0 | 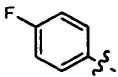 | Et | Et | CH₂Ph | Et | Et | 61 |
| 36 | 0 | 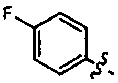 | Et | Et | CH₂Ph | 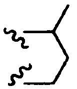 | | 69 |
| 37 | 0 | 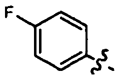 | Me | Ph | CH₂Ph | 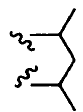 | | 68 |
| 38 | 0 | 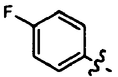 | Me | Ph | CH₂Ph | Me | (CH₂)₂P h | 62 |
| 39 | 0 | 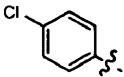 | ]-(CH₂)₅-[ | | CH₂Ph | Et | Et | 75 |
| 40 | 0 | 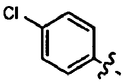 | ]-(CH₂)₅-[ | | CH₂Ph | 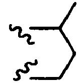 | | 60 |
| 41 | 0 | 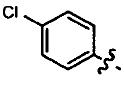 | Et | Et | CH₂Ph | 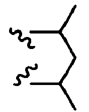 | | 64 |
| 42 | 0 | 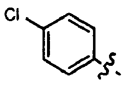 | Me | Ph | CH₂Ph | 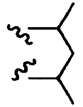 | | 79 |
| 43 | 0 | 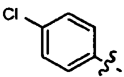 | Me | Ph | CH₂Ph | Me | (CH₂)₂P h | 72 |
| 44 | 0 | 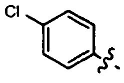 | 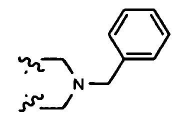 | | CH₂Ph | Et | Et | 67 |
| 45 | 0 | 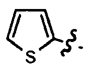 | ]-(CH₂)₅-[ | | CH₂Ph | 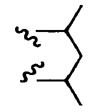 | | 71 |
| 46 | 0 | 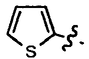 | ]-(CH₂)₅-[ | | CH₂Ph | Me | (CH₂)₂P h | 80 |
| 47 | 0 | 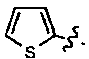 | ]-(CH₂)₅-[ | | CH₂Ph | Et | Et | 73 |
| 48 | 0 | 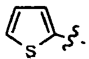 | ]-(CH₂)₅-[ | | CH₂Ph | ]-(CH₂)₂-S-(CH₂)₂-[ | | 60 |
| 49 | 0 | 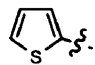 | Et | Et | CH₂Ph | Me | (CH₂)₂P h | 90 |
| 50 | 0 | 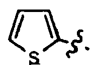 | Et | Et | CH₂Ph | Et | Et | 83 |
| 51 | 0 | 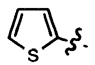 | Et | Et | CH₂Ph | 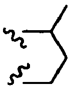 | | 100 |
| 52 | 0 | 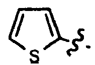 | Me | Ph | CH₂Ph | ]-(CH₂)₂-S-(CH₂)₂-[ | | 92 |
| 53 | 0 | 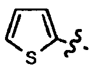 | 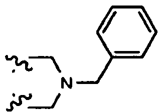 | | CH₂Ph | 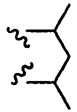 | | 86 |
| 54 | 0 | 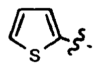 | 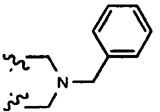 | | CH₂Ph | Me | (CH₂)₂P h | 74 |
| 55 | 0 | 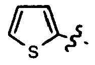 | 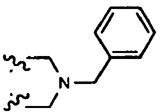 | | CH₂Ph | ]-(CH₂)₂-S-(CH₂)₂-[ | | 63 |
| 56 | 1 | 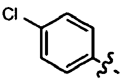 | ]-(CH₂)₅-[ | | CH₂Ph | 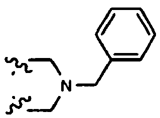 | | 62 |
| 57 | 1 | 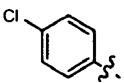 | ]-(CH₂)₅-[ | | CH₂Ph | 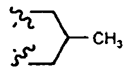 | | 64 |
| 58 | 1 | 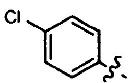 | ]-(CH₂)₅-[ | | CH₂Ph | Me | CH₂Ph | 73 |
| 59 | 1 | 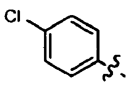 | Et | Et | CH₂Ph | 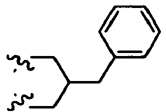 | | 60 |
| 60 | 1 | 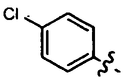 | Et | Et | CH₂Ph | 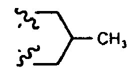 | | 68 |
| 61 | 1 | 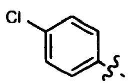 | Et | Et | CH₂Ph | Me | CH₂Ph | 76 |
| 62 | 1 | 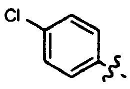 | 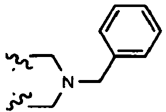 | | CH₂Ph | 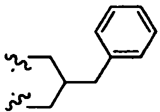 | | 87 |
| 63 | 1 | 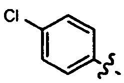 | 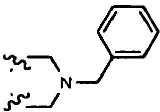 | | CH₂Ph | 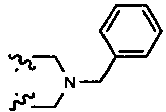 | | 78 |
| 64 | 1 | 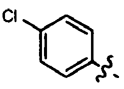 | 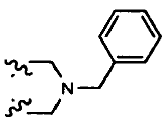 | | CH₂Ph | 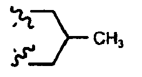 | | 69 |
| 65 | 1 | 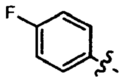 | ]-(CH₂)₅-[ | | CH₂Ph | 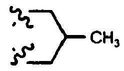 | | 61 |
| 66 | 1 | 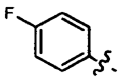 | ]-(CH₂)₅-[ | | CH₂Ph | Me | CH₂Ph | 65 |
| 67 | 1 | 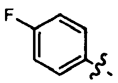 | Et | Et | CH₂Ph | 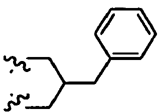 | | 64 |
| 68 | 1 | 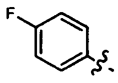 | Et | Et | CH₂Ph | 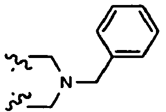 | | 60 |
| 69 | 1 | 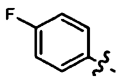 | Et | Et | CH₂Ph | 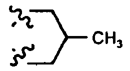 | | 82 |
| 70 | 1 | 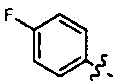 | Et | Et | CH₂Ph | Me | CH₂Ph | 60 |
| 71 | 1 | 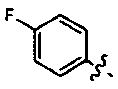 | 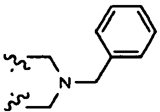 | | CH₂Ph | 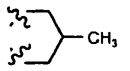 | | 67 |
| 72 | 1 | 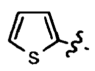 | ]-(CH₂)₅-[ | | CH₂Ph | 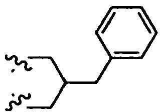 | | 69 |
| 73 | 1 | 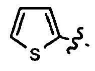 | ]-(CH₂)₅-[ | | CH₂Ph | 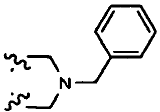 | | 94 |
| 74 | 1 | 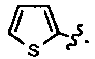 | ]-(CH₂)₅-[ | | CH₂Ph | 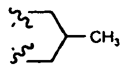 | | 79 |
| 75 | 1 | 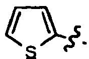 | ]-(CH₂)₅-[ | | CH₂Ph | Me | CH₂Ph | 90 |
| 76 | 1 | 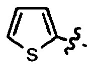 | ]-(CH₂)₅-[ | | CH₂Ph | 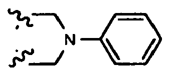 | | 94 |
| 77 | 1 | 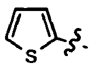 | Et | Et | CH₂Ph | 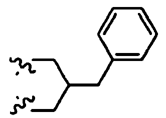 | | 91 |
| 78 | 1 | 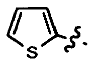 | Et | Et | CH₂Ph | 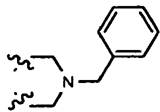 | | 100 |
| 79 | 1 | 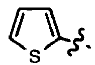 | Et | Et | CH₂Ph | 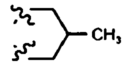 | | 97 |
| 80 | 1 | 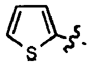 | Et | Et | CH₂Ph | Me | CH₂Ph | 93 |
| 81 | 1 | 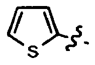 | 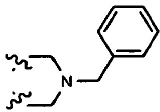 | | CH₂Ph | 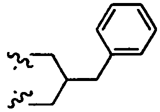 | | 81 |
| 82 | 1 | 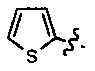 | 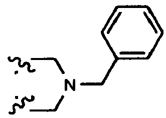 | | CH₂Ph | 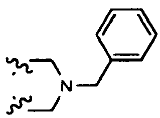 | | 83 |
| 83 | 1 | 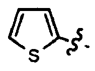 | 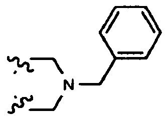 | | CH₂Ph | 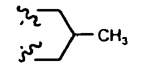 | | 97 |
| 84 | 1 | 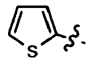 | 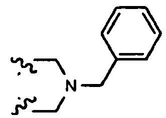 | | CH₂Ph | Me | CH₂Ph | 88 |
| 85 | 1 | 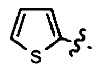 | 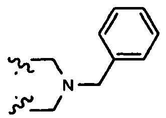 | | CH₂Ph | 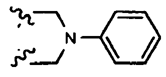 | | 91 |
| 86 | 1 | 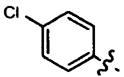 | ]-(CH₂)₅-[ | | CH₂Ph | Me | (CH₂)₂P h | 63 |
| 87 | 1 | 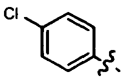 | ]-(CH₂)₅-[ | | CH₂Ph | Et | Et | 64 |
| 88 | 1 | 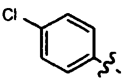 | Et | Et | CH₂Ph | 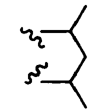 | | 79 |
| 89 | 1 | 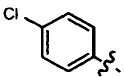 | Et | Et | CH₂Ph | Me | (CH₂)₂P h | 87 |
| 90 | 1 | 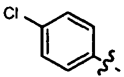 | Et | Et | CH₂Ph | Et | Et | 89 |
| 91 | 1 | 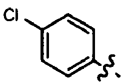 | Et | Et | CH₂Ph | ]-(CH2)₂-S-(CH₂)₂-[ | | 75 |
| 92 | 1 | 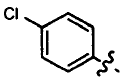 | Me | Ph | CH₂Ph | ]-(CH₂)₂-S-(CH₂)₂-[ | | 87 |
| 93 | 1 | 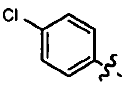 | 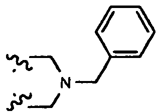 | | CH₂Ph | Me | (CH₂)₂P h | 66 |
| 94 | 1 | 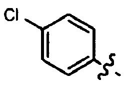 | 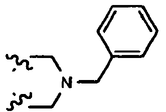 | | CH₂Ph | Et | Et | 74 |
| 95 | 1 | 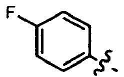 | ]-(CH₂)₅-[ | | CH₂Ph | Me | (CH₂)₂P h | 65 |
| 96 | 1 | 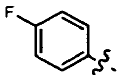 | Et | Et | CH₂Ph | 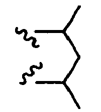 | | 80 |
| 97 | 1 | 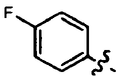 | Et | Et | CH₂Ph | Me | (CH₂)₂P h | 85 |
| 98 | 1 | 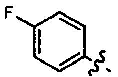 | Et | Et | CH₂Ph | Et | Et | 77 |
| 99 | 1 | 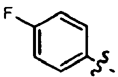 | Et | Et | CH₂Ph | ]-(CH₂)₂-S-(CH₂)₂-[ | | 62 |
| 100 | 1 | 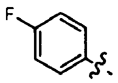 | Et | Et | CH₂Ph | 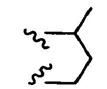 | | 86 |
| 101 | 1 | 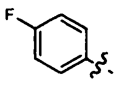 | Me | Ph | CH₂Ph | 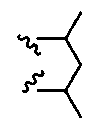 | | 68 |
| 102 | 1 | 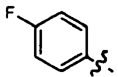 | Me | Ph | CH₂Ph | Me | (CH₂)₂P h | 77 |
| 103 | 1 | 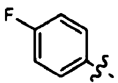 | Me | Ph | CH₂Ph | Et | Et | 79 |
| 104 | 1 | 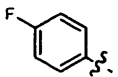 | Me | Ph | CH₂Ph | ]-(CH₂)₂-S-(CH₂)₂-[ | | 89 |
| 105 | 1 | 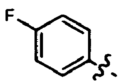 | Me | Ph | CH₂Ph | 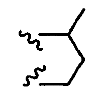 | | 71 |
| 106 | 1 | 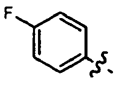 | 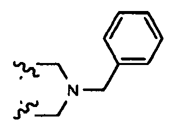 | | CH₂Ph | 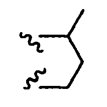 | | 61 |
| 107 | 1 | 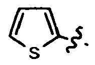 | ]-(CH₂)₅-[ | | CH₂Ph | 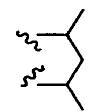 | | 84 |
| 108 | 1 | 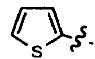 | ]-(CH₂)₅-[ | | CH₂Ph | Me | (CH₂)₂P h | 88 |
| 109 | 1 | 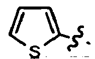 | ]-(CH₂)₅-[ | | CH₂Ph | Et | Et | 98 |
| 110 | 1 | 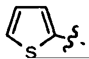 | ]-(CH₂)₅-[ | | CH₂Ph | ]-(CH₂)₂-S-(CH₂)₂-[ | | 97 |
| 111 | 1 | 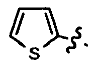 | ]-(CH₂)₅-[ | | CH₂Ph | 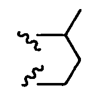 | | 92 |
| 112 | 1 | 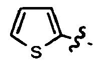 | Et | Et | CH₂Ph | 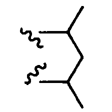 | | 89 |
| 113 | 1 | 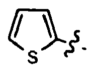 | Et | Et | CH₂Ph | Me | (CH₂)₂P h | 97 |
| 114 | 1 | 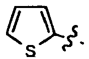 | Et | Et | CH₂Ph | Et | Et | 94 |
| 115 | 1 | 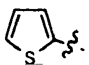 | Et | Et | CH₂Ph | ]-(CH₂)₂-S-(CH₂)₂-[ | | 93 |
| 116 | 1 | 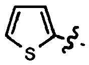 | Et | Et | CH₂Ph | 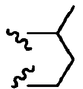 | | 94 |
| 117 | 1 | 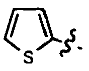 | Me | Ph | CH₂Ph | 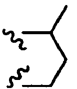 | | 99 |
| 118 | 1 | 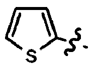 | 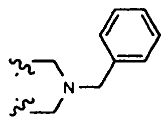 | | CH₂Ph | ]-(CH₂)₂-S-(CH₂)₂-[ | | 84 |
| 119 | 1 | 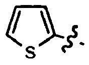 | 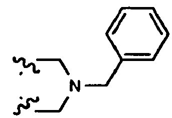 | | CH₂Ph | 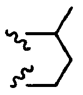 | | 99 |
| 120 | 0 | 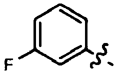 | ]-(CH₂)₂-O-(CH₂)₂-[ | | CH₂Ph | ]-(CH₂)₄-[ | | 75 |
| 121 | 0 | 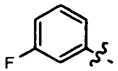 | ]-(CH₂)₂-O-(CH₂)₂-[ | | CH₂Ph | ]-(CH₂)₂-O-(CH₂)₂-[ | | 67 |
| 122 | 0 | 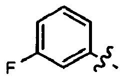 | 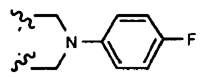 | | CH₂Ph | ]-(CH₂)₄-[ | | 72 |
| 123 | 0 | 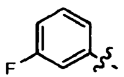 | 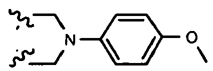 | | CH₂Ph | ]-(CH₂)₄-[ | | 66 |
| 124 | 0 | 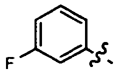 | 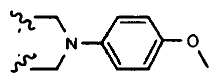 | | CH₂Ph | ]-(CH₂)₂-O-(CH₂)₂-[ | | 60 |
| 125 | 0 | 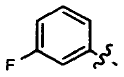 | 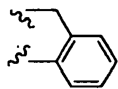 | | CH₂Ph | ]-(CH₂)₄-[ | | 83 |
| 126 | 0 | 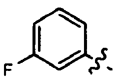 | 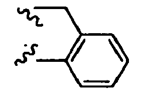 | | CH₂Ph | ]-(CH₂)₂-O-(CH₂)₂-[ | | 76 |
| 127 | 0 | 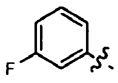 | 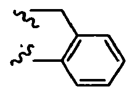 | | CH₂Ph | 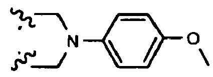 | | 73 |
| 128 | 0 | 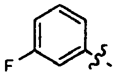 | 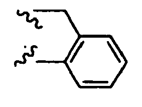 | | CH₂Ph | 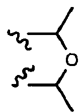 | | 71 |
| 129 | 0 | 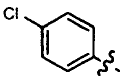 | ]-(CH₂)₂-O-(CH₂)₂- | | CH₂Ph | ]-(CH₂)₄-[ | | 75 |
| 130 | 0 | 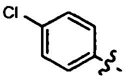 | ]-(CH₂)₂-O-(CH₂)₂-[ | | CH₂Ph | ]-(CH₂)₂-O-(CH₂)₂-[ | | 77 |
| 131 | 0 | 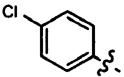 | ]-(CH₂)₂-O-(CH₂)₂-[ | | CH₂Ph | 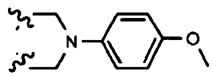 | | 64 |
| 132 | 0 | 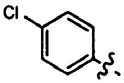 | ]-(CH₂)₂-O-(CH₂)₂-[ | | CH₂Ph | 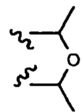 | | 64 |
| 133 | 0 | 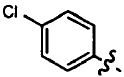 | 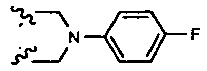 | | CH₂Ph | ]-(CH₂)₄-[ | | 64 |
| 134 | 0 | 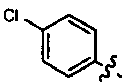 | 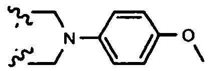 | | CH₂Ph | ]-(CH₂)₄-[ | | 72 |
| 135 | 0 | 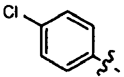 | 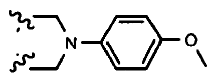 | | CH₂Ph | ]-(CH₂)₂-O-(CH₂)₂-[ | | 70 |
| 136 | 0 | 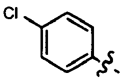 | 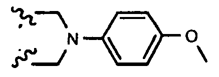 | | CH₂Ph | 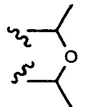 | | 62 |
| 137 | 0 | 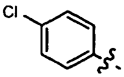 | 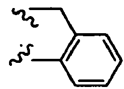 | | CH₂Ph | ]-(CH₂)₄-[ | | 85 |
| 138 | 0 | 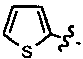 | ]-(CH₂)₂-O-(CH₂)₂-[ | | CH₂Ph | ]-(CH₂)₄-[ | | 91 |
| 139 | 0 | 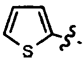 | ]-(CH₂)₂-O-(CH₂)₂-[ | | CH₂Ph | ]-(CH₂)₂-O-(CH₂)₂-[ | | 72 |
| 140 | 0 | 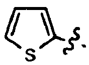 | ]-(CH₂)₂-O-(CH₂)₂-[ | | CH₂Ph | 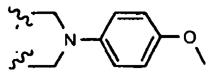 | | 67 |
| 141 | 0 | 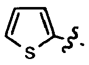 | ]-(CH₂)₂-O-(CH₂)₂-[ | | CH₂Ph | 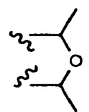 | | 65 |
| 142 | 0 | 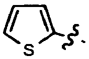 | 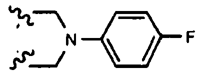 | | CH₂Ph | ]-(CH₂)₄-[ | | 85 |
| 143 | 0 | 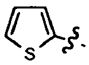 | 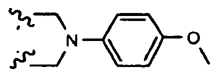 | | CH₂Ph | ]-(CH₂)₄-[ | | 77 |
| 144 | 0 | 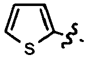 | 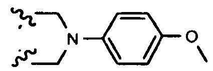 | | CH₂Ph | ]-(CH₂)₂-O-(CH₂)₂-[ | | 64 |
| 145 | 0 | 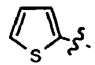 | 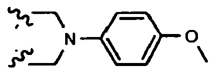 | | CH₂Ph | ]-(CH₂)₂-O-(CH₂)₂-[ | | 69 |
| 146 | 0 | 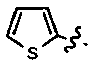 | 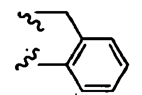 | | CH₂Ph | ]-(CH₂)₄-[ | | 92 |
| 147 | 0 | 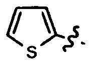 | 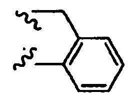 | | CH₂Ph | 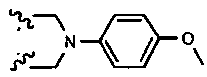 | | 91 |
| 148 | 0 | 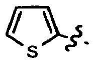 | 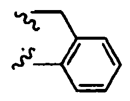 | | CH₂Ph | 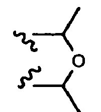 | | 84 |
| 149 | 1 | 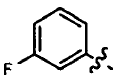 | ]-(CH₂)₂-O-(CH₂)₂-[ | | CH₂Ph | ]-(CH₂)₄-[ | | 87 |
| 150 | 1 | 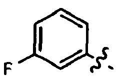 | ]-(CH₂)₂-O-(CH₂)₂-[ | | CH₂Ph | ]-(CH₂)₂-O-(CH₂)₂-[ | | 75 |
| 151 | 1 | 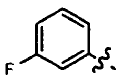 | ]-(CH₂)₂-O-(CH₂)₂-[ | | CH₂Ph | 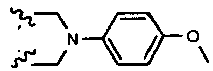 | | 89 |
| 152 | 1 | 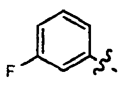 | ]-(CH₂)₂-O-(CH₂)₂-[ | | CH₂Ph | 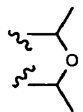 | | 65 |
| 153 | 1 | 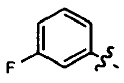 | 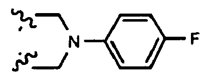 | | CH₂Ph | 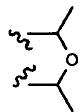 | | 71 |
| 154 | 1 | 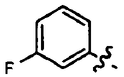 | 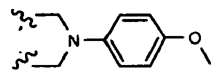 | | CH₂Ph | ]-(CH₂)₂-O-(CH₂)₂-[ | | 86 |
| 155 | 1 | 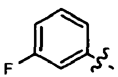 | 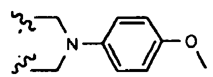 | | CH₂Ph | 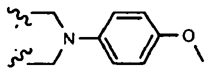 | | 82 |
| 156 | 1 | 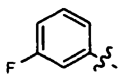 | 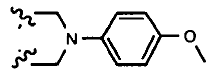 | | CH₂Ph | 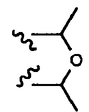 | | 74 |
| 157 | 1 | 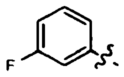 | 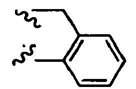 | | CH₂Ph | 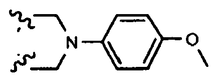 | | 84 |
| 158 | 1 | 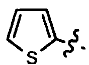 | ]-(CH₂)₂-O-(CH₂)₂-[ | | CH₂Ph | ]-(CH₂)₂-O-(CH₂)₂-[ | | 83 |
| 159 | 1 | 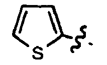 | ]-(CH₂)₂-O-(CH₂)₂-[ | | CH₂Ph | 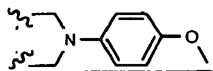 | | 94 |
| 160 | 1 | 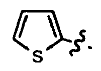 | ]-(CH₂)₂-O-(CH₂)₂-[ | | CH₂Ph | 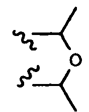 | | 79 |
| 161 | 1 | 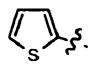 | 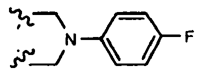 | | CH₂Ph | ]-(CH₂)₂-O-(CH₂)₂-[ | | 87 |
| 162 | 1 | 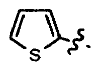 | 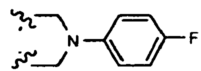 | | CH₂Ph | 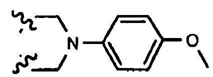 | | 96 |
| 163 | 1 | 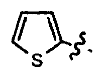 | 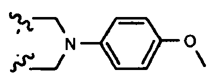 | | CH₂Ph | ]-(CH₂)₄-[ | | 83 |
| 164 | 1 | 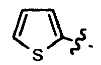 | 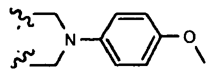 | | CH₂Ph | 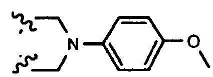 | | 93 |
| 165 | 1 | 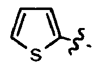 | 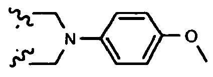 | | CH₂Ph | 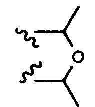 | | 93 |
| 166 | 1 | 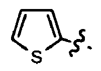 | 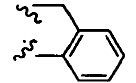 | | CH₂Ph | 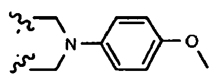 | | 78 |

Die Trennung von Diastereomeren und/oder Enantiomeren erfolgt nach dem Fachmann bekannten Methoden, beispielsweise durch Umkristallisation, Chromatographie oder insbesondere HPLC-Chromatographie bzw. Kristallisation mit einer gegebenenfalls chiralen Säure oder Base und Trennung der Salze oder chirale HPLC-Chromatographie (Fogassy et al., Optical resolution methods, Org. Biomol. Chem 2006, 4, 3011-3030).

## Patentansprüche

1. Substituierte Oxazol-Derivate der allgemeinen Formel I, 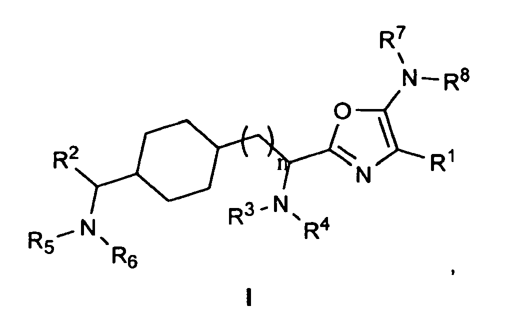 worin
n 0 oder 1 bedeutet,
R¹ Benzyl bedeutet,
R² Phenyl, unsubstituiert oder einfach substituiert mit Cl oder F oder Thienyl bedeutet,
R³ und R⁴ unabhängig voneinander Phenyl, Ethyl oder Methyl bedeuten oder die Reste R³ und R⁴ zusammen CH₂CH₂OCH₂CH₂, CH₂CH₂NR⁹CH₂CH₂, (CH₂)₄₋₅ oder 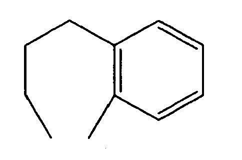 bedeuten, wobei R⁹ für Benzyl, 4-F-Phenyl oder 4-Methoxyphenyl steht,
R⁵ und R⁶ CH3 bedeuten,
R⁷ und R⁸ unabhängig voneinander Methyl, Ethyl, Benzyl oder Phenethyl bedeuten;
oder die Reste R⁷ und R⁸ CH₂CH₂SCH₂CH₂, CH₂CH₂OCH₂CH₂, (CH₂)₄ oder (CH₂)₅, CH₂CH₂NR¹⁰CH₂CH₂, wobei einzelne H-Atome durch Methyl oder Benzyl ersetzt sein können, und R¹⁰ Phenyl, 4-Methoxyphenyl oder Benzyl bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Substituierte Oxazol-Derivate gemäß Anspruch 1 aus der Gruppe
17. [{4-[(4-Benzy)-5-pyrrolidin-1-yl-oxazol-2-yl)-piperidin-1-yl-methyl]-cyclohexyl}-(4-fluor-phenyl)-methyl]-dimethyl-amin
18. ((4-Benzyl-5-morpholin-4-yl-oxazol-2-yl)-{4-[dimethylamino-(4-fluor-phenyl)-methyl]-cyclohexyl}-methyl)-methyl-phenyl-amin
19. ([4-Benzyl-5-(4-methyl-piperidin-1-yl)-oxazol-2-yl]-{4-[dimethylamino-(4-fluorphenyl)-methyl]-cyclohexyl}-methyl)-methyl-phenyl-amin
20. ([4-Benzyl-5-(4-methyl-piperidin-1-yl)-oxazol-2-yl]-{4-[(4-chlor-phenyl)-dimethylamino-methyl]-cyclohexyl}-methyl)-diethyl-amin
21. Benzyl-[4-benzyl-2-({4-[(4-chlor-phenyl)-dimethylamino-methyl]-cyclohexyl}-diethylamino-methyl)-oxazol-5-yl]-methyl-amin
22. Benzyl-{4-benzyl-2-[{4-[(4-chlor-phenyl)-dimethylamino-methyl]-cyclohexyl}-(methyl-phenyl-amino)-methyl]-oxazol-5-yl}-methyl-amin
23. [(4-{[4-Benzyl-5-(4-benzyl-piperidin-1-yl)-oxazol-2-yl]-piperidin-1-yl-methyl}-cyclohexyl)-thiophen-2-yl-methyl]-dimethyl-amin
24. [(4-{[4-Benzyl-5-(4-methyl-piperidin-1-yl)-oxazol-2-yl]-piperidin-1-yl-methyl}-cyclohexyl)-thiophen-2-yl-methyl]-dimethyl-amin
25. Benzyl-(4-benzyl-2-{[4-(dimethylamino-thiophen-2-yl-methyl)-cyclohexyl]-piperidin-1-yl-methyl}-oxazol-5-yl)-methyl-amin
26. [(4-{[4-Benzyl-5-(4-phenyl-piperazin-1-yl)-oxazol-2-yl]-piperidin-1-yl-methyl}-cyclohexyl)-thiophen-2-yl-methyl]-dimethyl-amin
27. {[4-Benzyl-5-(4-benzyl-piperidin-1-yl)-oxazol-2-yl]-[4-(dimethylamino-thiophen-2-yl-methyl)-cyclohexyl]-methyl}-diethyl-amin
28. {[4-Benzyl-5-(4-benzyl-piperazin-1-yl)-oxazol-2-yl]-[4-(dimethylamino-thiophen-2-yl-methyl)-cyclohexyl]-methyl}-diethyl-amin
29. {[4-Benzyl-5-(4-methyl-piperidin-1-yl)-oxazol-2-yl]-[4-(dimethylamino-thiophen-2-yl-methyl)-cyclohexyl]-methyl}-diethyl-amin
30. Benzyl-(4-benzyl-2-{diethylamino-[4-(dimethylamino-thiophen-2-yl-methyl)-cyclohexyl]-methyl}-oxazol-5-yl)-methyl-amin
31. {[4-Benzyl-5-(4-phenyl-piperazin-1-yl)-oxazol-2-yl]-[4-(dimethylamino-thiophen-2-yl-methyl)-cyclohexyl]-methyl}-diethyl-amin
32. ({4-[[4-Benzyl-5-(4-benzyl-piperidin-1-yl)-oxazol-2-yl]-(4-benzyl-piperazin-1-yl)-methyl]-cyclohexyl}-thiophen-2-yl-methyl)-dimethyl-amin
33. [(4-{[4-Benzyl-5-(3-methyl-piperidin-1-yl)-oxazol-2-yl]-piperidin-1-yl-methyl}-cyclohexyl)-(4-fluor-phenyl)-methyl]-dimethyl-amin
34. [4-Benzyl-2-(diethylamino-{4-[dimethylamino-(4-fluor-phenyl)-methyl]-cyclohexyl}-methyl)-oxazol-5-yl]-methyl-phenethyl-amin
35. [4-Benzyl-2-(diethylamino-{4-[dimethylamino-(4-fluor-phenyl)-methyl]-cyclohexyl}-methyl)-oxazol-5-yl]-diethyl-amin
36. ([4-Benzyl-5-(3-methyl-piperidin-1-yl)-oxazol-2-yl]-{4-[dimethylamino-(4-fluorphenyl)-methyl]-cyclohexyl}-methyl)-diethyl-amin
37. ([4-Benzyl-5-(3,5-dimethyl-piperidin-1-yl)-oxazol-2-yl]-{4-[dimethylamino-(4-fluorphenyl)-methyl]-cyclohexyl}-methyl)-methyl-phenyl-amin
38. {4-Benzyl-2-[{4-[dimethylamino-(4-fluor-phenyl)-methyl]-cyclohexyl}-(methylphenyl-amino)-methyl]-oxazol-5-yl}-methyl-phenethyl-amin
39. [4-Benzyl-2-({4-[(4-chlor-phenyl)-dimethylamino-methyl]-cyclohexyl}-piperidin-1-yl-methyl)-oxazol-5-yl]-diethyl-amin
40. [(4-{[4-Benzyl-5-(3-methyl-piperidin-1-yl)-oxazol-2-yl]-piperidin-1-yl-methyl}-cyclohexyl)-(4-chlor-phenyl)-methyl]-dimethyl-amin
41. ([4-Benzyl-5-(3,5-dimethyl-piperidin-1-yl)-oxazol-2-yl]-{4-[(4-chlor-phenyl)-dimethylamino-methyl]-cyclohexyl}-methyl)-diethyl-amin
42. ([4-Benzyl-5-(3,5-dimethyl-piperidin-1-yl)-oxazol-2-yl]-{4-[(4-chlor-phenyl)-dimethylamino-methyl]-cyclohexyl}-methyl)-methyl-phenyl-amin
43. {4-Benzyl-2-[{4-[(4-chlor-phenyl)-dimethylamino-methyl]-cyclohexyl}-(methylphenyl-amino)-methyl]-oxazol-5-yl}-methyl-phenethyl-amin
44. [4-Benzyl-2-((4-benzyl-piperazin-1-yl)-{4-[(4-chlor-phenyl)-dimethylaminomethyl]-cyclohexyl}-methyl)-oxazol-5-yl]-diethyl-amin
45. [(4-{[4-Benzyl-5-(3,5-dimethyl-piperidin-1-yl)-oxazol-2-yl]-piperidin-1-yl-methyl}-cyclohexyl)-thiophen-2-yl-methyl]-dimethyl-amin
46. (4-Benzyl-2-{[4-(dimethylamino-thiophen-2-yl-methyl)-cyclohexyl]-piperidin-1-yl-methyl}-oxazol-5-yl)-methyl-phenethyl-amin
47. (4-Benzyl-2-{[4-(dimethylamino-thiophen-2-yl-methyl)-cyclohexyl]-piperidin-1-yl-methyl}-oxazol-5-yl)-diethyl-amin
48. ({4-[(4-Benzyl-5-thiomorpholin-4-yl-oxazol-2-yl)-piperidin-1-yl-methyl]-cyclohexyl}-thiophen-2-yl-methyl)-dimethyl-amin
49. (4-Benzyl-2-{diethylamino-[4-(dimethylamino-thiophen-2-yl-methyl)-cyclohexyl]-methyl}-oxazol-5-yl)-methyl-phenethyl-amin
50. (4-Benzyl-2-{diethylamino-[4-(dimethylamino-thiophen-2-yl-methyl)-cyclohexyl]-methyl}-oxazol-5-yl)-diethyl-amin
51. {[4-Benzyl-5-(3-methyl-piperidin-1-yl)-oxazol-2-yl]-[4-(dimethylamino-thiophen-2-yl-methyl)-cyclohexyl]-methyl}-diethyl-amin
52. {(4-Benzyl-5-thiomorpholin-4-yl-oxazol-2-yl)-[4-(dimethylamino-thiophen-2-yl-methyl)-cyclohexyl]-methyl}-methyl-phenyl-amin
53. ({4-[[4-Benzyl-5-(3,5-dimethyl-piperidin-1-yl)-oxazol-2-yl]-(4-benzyl-piperazin-1-yl)-methyl]-cyclohexyl}-thiophen-2-yl-methyl)-dimethyl-amin
54. (4-Benzyl-2-{(4-benzyl-piperazin-1-yl)-[4-(dimethylamino-thiophen-2-yl-methyl)-cyclohexyl]-methyl}-oxazol-5-yl)-methyl-phenethyl-amin
55. ({4-[(4-Benzyl-piperazin-1-yl)-(4-benzyl-5-thiomorpholin-4-yl-oxazol-2-yl)-methyl]-cyclohexyl}-thiophen-2-yl-methyl)-dimethyl-amin
56. [(4-{2-[4-Benzyl-5-(4-benzyl-piperazin-1-yl)-oxazol-2-yl]-2-piperidin-1-yl-ethyl}-cyclohexyl)-(4-chlor-phenyl)-methyl]-dimethyl-amin
57. [(4-{2-[4-Benzyl-5-(4-methyl-piperidin-1-yl)-oxazol-2-yl]-2-piperidin-1-yl-ethyl}-cyclohexyl)-(4-chlor-phenyl)-methyl]-dimethyl-amin
58. Benzyl-[4-benzyl-2-(2-{4-[(4-chlor-phenyl)-dimethylamino-methyl]-cyclohexyl}-1-piperidin-1-yl-ethyl)-oxazol-5-yl]-methyl-amin
59. (1-[4-Benzyl-5-(4-benzyl-piperidin-1-yl)-oxazol)-2-yl]-2-{4-[(4-chlor-phenyl)-dimethylamino-methyl]-cyclohexyl}-ethyl)-diethyl-amin
60. (1-[4-Benzyl-5-(4-methyl-piperidin-1-yl)-oxazol-2-yl]-2-{4-[(4-chlor-phenyl)-dimethylamino-methyl]-cyclohexyl}-ethyl)-diethyl-amin
61. Benzyl-[4-benzyl-2-(2-{4-[(4-chlor-phenyl)-dimethylamino-methyl]-cyclohexyl}-1-diethylamino-ethyl)-oxazol-5-yl]-methyl-amin
62. [{4-[2-[4-Benzyl-5-(4-benzyl-piperidin-1-yl)-oxazol-2-yl]-2-(4-benzyl-piperazin-1-yl)-ethyl]-cyclohexyl}-(4-chlor-phenyl)-methyl]-dimethyl-amin
63. [{4-[2-[4-Benzyl-5-(4-benzyl-piperazin-1-yl)-oxazol-2-yl]-2-(4-benzyl-piperazin-1-yl)-ethyl]-cyclohexyl}-(4-chlor-phenyl)-methyl]-dimethyl-amin
64. [{4-[2-[4-Benzyl-5-(4-methyl-piperidin-1-yl)-oxazol-2-yl]-2-(4-benzyl-piperazin-1-yl)-ethyl]-cyclohexyl}-(4-chlor-phenyl)-methyl]-dimethyl-amin
65. [(4-{2-[4-Benzyl-5-(4-methyl-piperidin-1-yl)-oxazol-2-yl]-2-piperidin-1-yl-ethyl}-cyclohexyl)-(4-fluor-phenyl)-methyl]-dimethyl-amin
66. Benzyl-[4-benzyl-2-(2-{4-[dimethylamino-(4-fluor-phenyl)-methyl]-cyclohexyl}-1-piperidin-1-yl-ethyl)-oxazol-5-yl]-methyl-amin
67. (1-[4-Benzyl-5-(4-benzyl-piperidin-1-yl)-oxazol-2-yl]-2-{4-[dimethylamino-(4-fluor-phenyl)-methyl]-cyclohexyl}-ethyl)-diethyl-amin
68. (1-[4-Benzyl-5-(4-benzyl-piperazin-1-yl)-oxazol-2-yl]-2-{4-[dimethylamino-(4-fluor-phenyl)-methyl]-cyclohexyl}-ethyl)-diethyl-amin
69. (1-[4-Benzyl-5-(4-methyl-piperidin-1-yl)-oxazol-2-yl]-2-{4-[dimethylamino-(4-fluor-phenyl)-methyl]-cyclohexyl}-ethyl)-diethyl-amin
70. Benzyl-[4-benzyl-2-(1-diethylamino-2-{4-[dimethylamino-(4-fluor-phenyl)-methyl]-cyclohexyl}-ethyl)-oxazol-5-yl]-methyl-amin
71. [{4-[2-[4-Benzyl-5-(4-methyl-piperidin-1-yl)-oxazol-2-yl]-2-(4-benzyl-piperazin-1-yl)-ethyl]-cyclohexyl}-(4-fluor-phenyl)-methyl]-dimethyl-amin
72. [(4-{2-[4-Benzyl-5-(4-benzyl-piperidin-1-yl)-oxazol-2-yl]-2-piperidin-1-yl-ethyl}-cyclohexyl)-thiophen-2-yl-methyl]-dimethyl-amin
73. [(4-{2-[4-Benzyl-5-(4-benzyl-piperazin-1-yl)-oxazol-2-yl]-2-piperidin-1-yl-ethyl}-cyclohexyl)-thiophen-2-yl-methyl]-dimethyl-amin
74. [(4-{2-[4-Benzyl-5-(4-methyl-piperidin-1-yl)-oxazol-2-yl]-2-piperidin-1-yl-ethyl}-cyclohexyl)-thiophen-2-yl-methyl]-dimethyl-amin
75. Benzyl-(4-benzyl-2-{2-[4-(dimethylamino-thiophen-2-yl-methyl)-cyclohexyl]-1-piperidin-1-yl-ethyl}-oxazol-5-yl)-methyl-amin
76. [(4-{2-[4-Benzyl-5-(4-phenyl-piperazin-1-yl)-oxazol-2-yl]-2-piperidin-1-yl-ethyl}-cyclohexyl)-thiophen-2-yl-methyl]-dimethyl-amin
77. {1-[4-Benzyl-5-(4-benzyl-piperidin-1-yl)-oxazol-2-yl]-2-[4-(dimethylamino-thiophen-2-yl-methyl)-cyclohexyl]-ethyl}-diethyl-amin
78. {1-[4-Benzyl-5-(4-benzyl-piperazin-1-yl)-oxazol-2-yl]-2-[4-(dimethylamino-thiophen-2-yl-methyl)-cyclohexyl]-ethyl}-diethyl-amin
79. {1-[4-Benzyl-5-(4-methy)-piperidin-1-yl)-oxazol-2-yl]-2-[4-(dimethylamino-thiophen-2-yl-methyl)-cyclohexyl]-ethyl}-diethyl-amin
80. Benzyl-(4-benzyl-2-{1-diethylamino-2-[4-(dimethylamino-thiophen-2-yl-methyl)-cyclohexyl]-ethyl}-oxazol-5-yl)-methyl-amin
81. ({4-[2-[4-Benzyl-5-(4-benzyl-piperidin-1-yl)-oxazol-2-yl]-2-(4-benzyl-piperazin-1-yl)-ethyl]-cyclohexyl}-thiophen-2-yl-methyl)-dimethyl-amin
82. ({4-[2-[4-Benzyl-5-(4-benzyl-piperazin-1-yl)-oxazol-2-yl]-2-(4-benzyl-piperazin-1-yl)-ethyl]-cyclohexyl}-thiophen-2-yl-methyl)-dimethyl-amin
83. ({4-[2-[4-Benzyl-5-(4-methyl-piperidin-1-yl)-oxazol-2-yl]-2-(4-benzyl-piperazin-1-yl)-ethyl]-cyclohexyl}-thiophen-2-yl-methyl)-dimethyl-amin
84. Benzyl-(4-benzyl-2-{1-(4-benzyl-piperazin-1-yl)-2-[4-(dimethylamino-thiophen-2-yl-methyl)-cyclohexyl]-ethyl}-oxazol-5-yl)-methyl-amin
85. ({4-[2-[4-Benzyl-5-(4-phenyl-piperazin-1-yl)-oxazol-2-yl]-2-(4-benzyl-piperazin-1-yl)-ethyl]-cyclohexyl}-thiophen-2-yl-methyl)-dimethyl-amin
86. [4-Benzyl-2-(2-{4-[(4-chlor-phenyl)-dimethylamino-methyl]-cyclohexyl}-1-piperidin-1-yl-ethyl)-oxazol-5-yl]-methyl-phenethyl-amin
87. [4-Benzyl-2-(2-{4-[(4-chlor-phenyl)-dimethylamino-methyl]-cyclohexyl}-1-piperidin-1-yl-ethyl)-oxazol-5-yl]-diethyl-amin
88. (1-[4-Benzyl-5-(3,5-dimethyl-piperidin-1-yl)-oxazol-2-yl]-2-{4-[(4-chlor-phenyl)-dimethylamino-methyl]-cyclohexyl}-ethyl)-diethyl-amin
89. [4-Benzyl-2-(2-{4-[(4-chlor-phenyl)-dimethylamino-methyl]-cyclohexyl}-1-diethylamino-ethyl)-oxazol-5-yl]-methyl-phenethyl-amin
90. [4-Benzyl-2-(2-{4-[(4-chlor-phenyl)-dimethylamino-methyl]-cyclohexyl}-1-diethylamino-ethyl)-oxazol-5-yl]-diethyl-amin
91. (1-(4-Benzyl-5-thiomorpholin-4-yl-oxazol-2-yl)-2-{4-[(4-chlor-phenyl)-dimethylamino-methyl]-cyclohexyl}-ethyl)-diethyl-amin
92. (1-(4-Benzyl-5-thiomorpholin-4-yl-oxazol-2-yl)-2-{4-[(4-chlor-phenyl)-dimethylamino-methyl]-cyclohexyl}-ethyl)-methyl-phenyl-amin
93. [4-Benzyl-2-(1-(4-benzyl-piperazin-1-yl)-2-{4-[(4-chlor-phenyl)-dimethylaminomethyl]-cyclohexyl}-ethyl)-oxazol-5-yl]-methyl-phenethyl-amin
94. [4-Benzyl-2-(1-(4-benzyl-piperazin-1-yl)-2-{4-[(4-chlor-phenyl)-dimethylaminomethyl]-cyclohexyl}-ethyl)-oxazol-5-yl]-diethyl-amin
95. [4-Benzyl-2-(2-{4-[dimethylamino-(4-fluor-phenyl)-methyl]-cyclohexyl}-1-piperidin-1-yl-ethyl)-oxazol-5-yl]-methyl-phenethyl-amin
96. (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-diethyl-amin
97. [4-Benzyl-2-(1-diethylamino-2-{4-[dimethylamino-(4-fluor-phenyl)-methyl]-cyclohexyl}-ethyl)-oxazol-5-yl]-methyl-phenethyl-amin
98. [4-Benzyl-2-(1-diethylamino-2-{4-[dimethylamino-(4-fluor-phenyl)-methyl]-cyclohexyl}-ethyl)-oxazol-5-yl]-diethyl-amin
99. (1-(4-Benzyl-5-thiomorpholin-4-yl-oxazol-2-yl)-2-{4-[dimethylamino-(4-fluorphenyl)-methyl]-cyclohexyl}-ethyl)-diethyl-amin
100. (1-[4-Benzyl-5-(3-methyl-piperidin-1-yl)-oxazol-2-yl]-2-{4-[dimethylamino-(4-fluor-phenyl)-methyl]-cyclohexyl}-ethyl)-diethyl-amin
101. (1-[4-Benzyl-5-(3,5-dimethyl-piperidin-1-yl)-oxazol-2-yl]-2-{4-[dimethylamino-(4-fluor-phenyl)-methyl]-cyclohexyl}-ethyl)-methyl-phenyl-amin
102. {4-Benzyl-2-[2-{4-[dimethylamino-(4-fluor-phenyl)-methyl]-cyclohexyl}-1-(methylphenyl-amino)-ethyl]-oxazol-5-yl}-methyl-phenethyl-amin
103. {4-Benzyl-2-[2-{4-[dimethylamino-(4-fluor-phenyl)-methyl]-cyclohexyl}-1-(methylphenyl-amino)-ethyl]-oxazol-5-yl}-diethyl-amin
104. (1-(4-Benzyl-5-thiomorpholin-4-yl-oxazol-2-yl)-2-{4-[dimethylamino-(4-fluorphenyl)-methyl]-cyclohexyl}-ethyl)-methyl-phenyl-amin
105. (1-[4-Benzyl-5-(3-methyl-piperidin-1-yl)-oxazol-2-yl]-2-{4-[dimethylamino-(4-fluor-phenyl)-methyl]-cyclohexyl}-ethyl)-methyl-phenyl-amin
106. [{4-[2-[4-Benzyl-5-(3-methyl-piperidin-1-yl)-oxazol-2-yl]-2-(4-benzyl-piperazin-1-yl)-ethyl]-cyclohexyl}-(4-fluor-phenyl)-methyl]-dimethyl-amin
107. [(4-{2-[4-Benzyl-5-(3,5-dimethyl-piperidin-1-yl)-oxazol-2-yl]-2-piperidin-1-yl-ethyl}-cyclohexyl)-thiophen-2-yl-methyl]-dimethyl-amin
108. (4-Benzyl-2-{2-[4-(dimethylamino-thiophen-2-yl-methyl)-cyclohexyl]-1-piperidin-1-yl-ethyl}-oxazol-5-yl)-methyl-phenethyl-amin
109. (4-Benzyl-2-{2-[4-(dimethylamino-thiophen-2-yl-methyl)-cyclohexyl]-1-piperidin-1-yl-ethyl}-oxazol-5-yl)-diethyl-amin
110. ({4-[2-(4-Benzyl-5-thiomorpholin-4-yl-oxazol-2-yl)-2-piperidin-1-yl-ethyl]-cyclohexyl}-thiophen-2-yl-methyl)-dimethyl-amin
111. [(4-{2-[4-Benzyl-5-(3-methyl-piperidin-1-yl)-oxazol-2-yl]-2-piperidin-1-yl-ethyl}-cyclohexyl)-thiophen-2-yl-methyl]-dimethyl-amin
112. {1-[4-Benzyl-5-(3,5-dimethyl-piperidin-1-yl)-oxazol-2-yl]-2-[4-(dimethylamino-thiophen-2-yl-methyl)-cyclohexyl]-ethyl}-diethyl-amin
113. (4-Benzyl-2-{1-diethylamino-2-[4-(dimethylamino-thiophen-2-yl-methyl)-cyclohexyl]-ethyl}-oxazol-5-yl)-methyl-phenethyl-amin
114. (4-Benzyl-2-{1-diethylamino-2-[4-(dimethylamino-thiophen-2-yl-methyl)-cyclohexyl]-ethyl}-oxazol-5-yl)-diethyl-amin
115. {1-(4-Benzyl-5-thiomorpholin-4-yl-oxazol-2-yl)-2-[4-(dimethylamino-thiophen-2-yl-methyl)-cyclohexyl]-ethyl}-diethyl-amin
116. {1-[4-Benzyl-5-(3-methyl-piperidin-1-yl)-oxazol-2-yl]-2-[4-(dimethylamino-thiophen-2-yl-methyl)-cyclohexyl]-ethyl}-diethyl-amin
117. {1-[4-Benzyl-5-(3-methyl-piperidin-1-yl)-oxazol-2-yl]-2-[4-(dimethylamino-thiophen-2-yl-methyl)-cyclohexyl]-ethyl}-methyl-phenyl-amin
118. ({4-[2-(4-Benzyl-piperazin-1-yl)-2-(4-benzyl-5-thiomorpholin-4-yl-oxazol-2-yl)-ethyl]-cyclohexyl}-thiophen-2-yl-methyl)-dimethyl-amin
119. ({4-[2-[4-Benzyl-5-(3-methyl-piperidin-1-yl)-oxazol-2-yl]-2-(4-benzyl-piperazin-1-yl)-ethyl]-cyclohexyl}-thiophen-2-yl-methyl)-dimethyl-amin
120. [{4-[(4-Benzyl-5-pyrrolidin-1-yl-oxazol-2-yl)-morpholin-4-yl-methyl]-cyclohexyl}-(3-fluor-phenyl)-methyl]-dimethyl-amin
121. [{4-[(4-Benzyl-5-morpholin-4-yl-oxazol-2-yl)-morpholin-4-yl-methyl]-cyclohexyl}-(3-fluor-phenyl)-methyl]-dimethyl-amin
122. [(4-{(4-Benzyl-5-pyrrolidin-1-yl-oxazol-2-yl)-[4-(4-fluor-phenyl)-piperazin-1-yl]-methyl}-cyclohexyl)-(3-fluor-phenyl)-methyl]-dimethyl-amin
123. [(4-{(4-Benzyl-5-pyrrolidin-1-yl-oxazol-2-yl)-[4-(4-methoxy-phenyl)-piperazin-1-yl]-methyl}-cyclohexyl)-(3-fluor-phenyl)-methyl]-dimethyl-amin
124. [(4-{(4-Benzyl-5-morpholin-4-yl-oxazol-2-yl)-[4-(4-methoxy-phenyl)-piperazin-1-yl]-methyl}-cyclohexyl)-(3-fluor-phenyl)-methyl]-dimethyl-amin
125. [{4-[(4-Benzyl-5-pyrrolidin-1-yl-oxazol-2-yl)-(3,4-dihydro-1H-isochinolin-2-yl)-methyl]-cyclohexyl}-(3-fluor-phenyl)-methyl]-dimethyl-amin
126. [{4-[(4-Benzyl-5-morpholin-4-yl-oxazol-2-yl)-(3,4-dihydro-1H-isochinolin-2-yl)-methyl]-cyclohexyl}-(3-fluor-phenyl)-methyl]-dimethyl-amin
127. [{4-[{4-Benzyl-5-[4-(4-methoxy-phenyl)-piperazin-1-yl]-oxazol-2-yl}-(3,4-dihydro-1H-isochinolin-2-yl)-methyl]-cydohexyl}-(3-fluor-phenyl)-methyl]-dimethyl-amin
128. [{4-[[4-Benzyl-5-(2,6-dimethyl-morpholin-4-yl)-oxazol-2-yl]-(3,4-dihydro-1H-isochinolin-2-yl)-methyl]-cyclohexyl}-(3-fluor-phenyl)-methyl]-dimethyl-amin
129. [{4-[(4-Benzyl-5-pyrrolidin-1-yl-oxazol-2-yl)-morpholin-4-yl-methyl]-cyclohexyl}-(4-chlor-phenyl)-methyl]-dimethyl-amin
130. [{4-[(4-Benzyl-5-morpholin-4-yl-oxazol-2-yl)-morpholin-4-yl-methyl]-cyclohexyl}-(4-chlor-phenyl)-methyl]-dimethyl-amin
131. [[4-({4-Benzyl-5-[4-(4-methoxy-phenyl)-piperazin-1-yl]-oxazol-2-yl}-morpholin-4-yl-methyl)-cyclohexyl]-(4-chlor-phenyl)-methyl]-dimethyl-amin
132. [(4-{[4-Benzyl-5-(2,6-dimethyl-morpholin-4-yl)-oxazol-2-yl]-morpholin-4-yl-methyl}-cyclohexyl)-(4-chlor-phenyl)-methyl]-dimethyl-amin
133. [(4-{(4-Benzyl-5-pyrrolidin-1-yl-oxazol-2-yl)-[4-(4-fluor-phenyl)-piperazin-1-yl]-methyl}-cyclohexyl)-(4-chlor-phenyl)-methyl]-dimethyl-amin
134. [(4-{(4-Benzyl-5-pyrrolidin-1-yl-oxazol-2-yl)-[4-(4-methoxy-phenyl)-piperazin-1-yl]-methyl}-cyclohexyl)-(4-chlor-phenyl)-methyl]-dimethyl-amin
135. [(4-{(4-Benzyl-5-morpholin-4-yl-oxazol-2-yl)-[4-(4-methoxy-phenyl)-piperazin-1-yl]-methyl}-cyclohexyl)-(4-chlor-phenyl)-methyl]-dimethyl-amin
136. [(4-{[4-Benzyl-5-(2,6-dimethyl-morpholin-4-yl)-oxazol-2-yl]-[4-(4-methoxyphenyl)-piperazin-1-yl]-methyl}-cyclohexyl)-(4-chlor-phenyl)-methyl]-dimethyl-amin
137. [{4-[(4-Benzyl-5-pyrrolidin-1-yl-oxazol-2-yl)-(3,4-dihydro-1H-isochinolin-2-yl)-methyl]-cyclohexyl}-(4-chlor-phenyl)-methyl]-dimethyl-amin
138. ({4-[(4-Benzyl-5-pyrrolidin-1-yl-oxazol-2-yl)-morpholin-4-yl-methyl]-cyclohexyl}-thiophen-2-yl-methyl)-dimethyl-amin
139. ({4-[(4-Benzyl-5-morpholin-4-yl-oxazol-2-yl)-morpholin-4-yl-methyl]-cyclohexyl}-t.hiophen-2-yl-methyl)-dimethyl-amin
140. {[4-({4-Benzyl-5-[4-(4-methoxy-phenyl)-piperazin-1-yl]-oxazol-2-yl}-morpholin-4-yl-methyl)-cyclohexyl]-thiophen-2-yl-methyl}-dimethyl-amin
141. [(4-{[4-Benzyl-5-(2,6-dimethyl-morpholin-4-yl)-oxazol-2-yl]-morpholin-4-yl-methyl}-cyclohexyl)-thiophen-2-yl-methyl]-dimethyl-amin
142. [(4.-{(4-Benzyl-5-pyrrolidin-1-yl-oxazol-2-yl)-[4-(4-fluor-phenyl)-piperazin-1-yl]-methyl}-cyclohexyl)-thiophen-2-yl-methyl]-dimethyl-amin
143. [(4-{(4-Benzyl-5-pyrrolidin-1-yl-oxazol-2-yl)-[4-(4-methoxy-phenyl)-piperazin-1-yl]-methyl}-cyclohexyl)-thiophen-2-yl-methyl]-dimethyl-amin
144. [(4-{(4-Benzyl-5-morpholin-4-yl-oxazol-2-yl)-[4-(4-methoxy-phenyl)-piperazin-1-yl]-methyl}-cyclohexyl)-thiophen-2-yl-methyl]-dimethyl-amin
145. [(4-{[4-Benzyl-5-(2,6-dimethyl-morpholin-4-yl)-oxazol-2-yl]-[4-(4-methoxyphenyl)-piperazin-1-yl]-methyl}-cyclohexyl)-thiophen-2-yl-methyl]-dimethyl-amin
146. ({4-[(4-Benzyl-5-pyrrolidin-1-yl-oxazol-2-yl)-(3,4-dihydro-1H-isochinolin-2-yl)-methyl]-cyclohexyl}-thiophen-2-yl-methyl)-dimethyl-amin
147. ({4-[{4-Benzyl-5-[4-(4-methoxy-phenyl)-piperazin-1-yl]-oxazol-2-yl}-(3,4-dihydro-1H-isochinolin-2-yl)-methyl]-cyclohexyl}-thiophen-2-yl-methyl)-dimethyl-amin
148. ({4-[[4-Benzyl-5-(2,6-dimethyl-morpholin-4-yl)-oxazol-2-yl]-(3,4-dihydro-1H-isochinolin-2-yl)-methyl]-cyclohexyl}-thiophen-2-yl-methyl)-dimethyl-amin
149. [{4-[2-(4-Benzyl-5-pyrrolidin-1-yl-oxazol-2-yl)-2-morpholin-4-yl-ethyl]-cyclohexyl}-(3-fluor-phenyl)-methyl]-dimethyl-amin
150. [{4-[2-(4-Benzyl-5-morpholin-4-yl-oxazol-2-yl)-2-morpholin-4-yl-ethyl]-cyclohexyl}-(3-fluor-phenyl)-methyl]-dimethyl-amin
151. [[4-(2-{4-Benzyl-5-[4-(4-methoxy-phenyl)-piperazin-1-yl]-oxazol-2-yl}-2-morpholin-4-yl-ethyl)-cyclohexyl]-(3-fluor-phenyl)-methyl]-dimethyl-amin
152. [(4-{2-[4-Benzyl-5-(2,6-dimethyl-morpholin-4-yl)-oxazol-2-yl]-2-morpholin-4-yl-ethyl}-cyclohexyl)-(3-fluor-phenyl)-methyl]-dimethyl-amin
153. [(4-{2-[4-Benzyl-5-(2,6-dimethyl-morpholin-4-yl)-oxazol-2-yl]-2-[4-(4-fluorphenyl)-piperazin-1-yl]-ethyl}-cyclohexyl)-(3-fluor-phenyl)-methyl]-dimethyl-amin
154. [(4-{2-(4-Benzyl-5-morpholin-4-yl-oxazol-2-yl)-2-[4-(4-methoxy-phenyl)-piperazin-1-yl]-ethyl}-cyclohexyl)-(3-fluor-phenyl)-methyl]-dimethyl-amin
155. [(4-{2-{4-Benzyl-5-[4-(4-methoxy-phenyl)-piperazin-1-yl]-oxazol-2-yl}-2-[4-(4-methoxy-phenyl)-piperazin-1-yl]-ethyl}-cyclohexyl)-(3-fluor-phenyl)-methyl]-dimethyl-amin
156. [(4-{2-[4-Benzyl-5-(2,6-dimethyl-morpholin-4-yl)-oxazol-2-yl]-2-[4-(4-methoxyphenyl)-piperazin-1-yl]-ethyl}-cyclohexyl)-(3-fluor-phenyl)-methyl]-dimethyl-amin
157. [{4-[2-{4-Benzyl-5-[4-(4-methoxy-phenyl)-piperazin-1-yl]-oxazol-2-yl}-2-(3,4-dihydro-1H-isochinolin-2-yl)-ethyl]-cyclohexyl}-(3-fluor-phenyl)-methyl]-dimethyl-amin
158. ({4-[2-(4-Benzyl-5-morpholin-4-yl-oxazol-2-yl)-2-morpholin-4-yl-ethyl]-cyclohexyl}-thiophen-2-yl-methyl)-dimethyl-amin
159. {[4-(2-{4-Benzyl-5-[4-(4-methoxy-phenyl)-piperazin-1-yl]-oxazo)-2-yl}-2-morpholin-4-yl-ethyl)-cyclohexyl]-thiophen-2-yl-methyl}-dimethyl-amin
160. [(4-{2-[4-Benzyl-5-(2,6-dimethyl-morpholin-4-yl)-oxazol-2-yl]-2-morpholin-4-yl-ethyl}-cyclohexyl)-thiophen-2-yl-methyl]-dimethyl-amin
161. [(4-{2-(4-Benzyl-5-morpholin-4-yl-oxazol-2-yl)-2-[4-(4-fluor-phenyl)-piperazin-1-yl]-ethyl}-cyclohexyl)-thiophen-2-yl-methyl]-dimethyl-amin
162. [(4-{2-{4-Benzyl-5-[4-(4-methoxy-phenyl)-piperazin-1-yl]-oxazol-2-yl}-2-[4-(4-fluor-phenyl)-piperazin-1-yl]-ethyl}-cyclohexyl)-thiophen-2-yl-methyl]-dimethyl-amin
163. [(4-{2-(4-Benzyl-5-pyrrolidin-1-yl-oxazol-2-yl)-2-[4-(4-methoxy-phenyl)-piperazin-1-yl]-ethyl}-cyclohexyl)-thiophen-2-yl-methyl]-dimethyl-amin
164. [(4-{2-{4-Benzyl-5-[4-(4-methoxy-phenyl)-piperazin-1-yl]-oxazol-2-yl}-2-[4-(4-methoxy-phenyl)-piperazin-1-yl]-ethyl}-cyclohexyl)-thiophen-2-yl-methyl]-dimethyl-amin
165. [(4-{2-[4-Benzyl-5-(2,6-dimethyl-morpholin-4-yl)-oxazol-2-yl]-2-[4-(4-methoxyphenyl)-piperazin-1-yl]-ethyl}-cyclohexyl)-thiophen-2-yl-methyl]-dimethyl-amin
166. ({4-[2-{4-Benzyl-5-[4-(4-methoxy-phenyl)-piperazin-1-yl]-oxazol-2-yl}-2-(3,4-dihydro-1H-isochinolin-2-yl)-ethyl]-cyclohexyl}-thiophen-2-yl-methyl)-dimethyl-amin

3. Verfahren zur Herstellung eines erfindungsgemäßen substituierten Oxazol-Derivates gemäß Anspruch 1, wobei man Aldehyde der allgemeinen Formel **A** mit Aminen der allgemeinen Formel **B** und Isonitrilamiden der allgemeinen Formel **C** 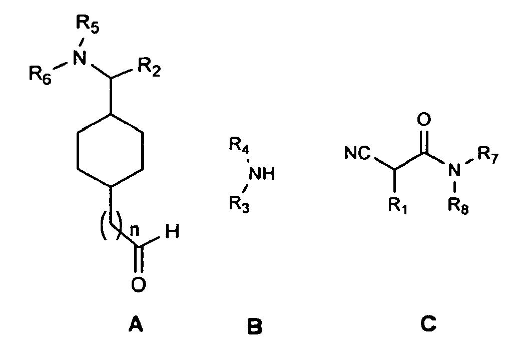 in einem organischen Lösungsmittel, beispielsweise Methanol oder Ethanol, 1-10 h bei einer Temperatur zwischen 30 und 100°C, vorzugsweise 40-80°C, erwärmt.

4. Arzneimittel enthaltend wenigstens ein substituiertes Oxazol-Derivat gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatz- und/oder Hilfsstoffe und/oder gegebenenfalls weiterer Wirkstoffe.

5. Verwendung eines substituierten Oxazol-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, neuropathischem oder chronischem Schmerz.

6. Verwendung eines substituierten Oxazol-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Depressionen, Harninkontinenz, Diarrhöe, Pruritus, Alkohol- und Drogenmißbrauch, Medikamentenabhängigkeit, Antriebslosigkeit und/oder zur Anxiolyse.

## Claims

1. Substituted oxazole derivatives of the general formula I: 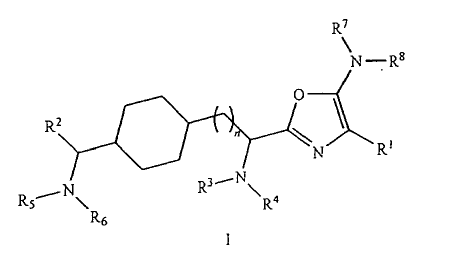 wherein
n is 0 or 1,
R¹ denotes benzyl,
R² denotes phenyl optionally monosubstituted with Cl or F, or denotes thienyl,
R³ and R⁴ independently denote phenyl, ethyl or methyl or the R³ and R⁴ radicals together denote CH₂CH₂OCH₂CH₂, CH₂CH₂NR⁹CH₂CH₂, (CH₂)₄₋₅ or 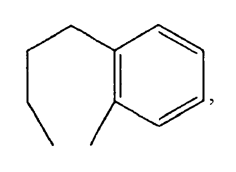 wherein R⁹ denotes benzyl, 4-F-phenyl or 4-methoxyphenyl,
R⁵ and R⁶ denote CH₃,
R⁷ and R⁸ independently denote methyl, ethyl, benzyl or phenethyl;
or the R⁷ and R⁸ radicals denote CH₂CH₂SCH₂CH₂, CH₂CH₂OCH₂CH₂, (CH₂)₄ or (CH₂)₅, CH₂CH₂NR¹⁰CH₂CH₂, wherein individual H atoms can be replaced by methyl or benzyl, and R¹⁰ denotes phenyl, 4-methoxyphenyl or benzyl;
in the form of the racemate; of the enantiomers, diasteromers, mixtures of the enantiomers or diastereomers or of an individual enantiomer or diasteromer; of the bases and/or salts of physiologically acceptable acids.

2. Substituted oxazole derivatives according to Claim 1 from the group of
17. [{4-[(4-benzyl-5-pyrrolidin-1-yl-oxazol-2-yl)-piperidin-1-yl-methyl]-cyclohexyl}-(4-fluorophenyl)-methyl]-dimethylamine;
18. ((4-benzyl-5-morpholin-4-yl-oxazol-2-yl)-{4-[dimethylamino-(4-fluorophenyl)-methyl]-cyclohexyl}-methyl)-methylphenylamine;
19. ([4-benzyl-5-(4-methylpiperidin-1-yl)-oxazol-2-yl]-{4-[dimethylamino-(4-fluorophenyl)-methyl]-cyclohexyl} -methyl)-methylphenylamine;
20. ([4-benzyl-5-(4-methylpiperldin-1-yl)-oxazol-2-yl]-{4-[(4-chlorophenyl)-dimethylaminomethyl]-cyclohexyl}-methyl)-diethylamine;
21. benzyl-[4-benzyl-2-({4-[(4-chlorophenyl)-dimethylaminomethyl]-cyclohexyl} -diethylaminomethyl)-oxazol-5-yl]-methylamine;
22. benzyl-{4-benzyl-2-[{4-[(4-chlorophenyl)-dimethylaminomethyl]-cyclohexyl}-(methylphenylamino)-methyl]-oxazol-5-yl}-methylamine;
23. [(4-{[4-benzyl-5-(4-benzylpiperidin-1-yl)-oxazol-2-yl]-piperidin-1-yl-methyl}-cyclohexyl)-thiophen-2-yl-methyl]-dimethylamine;
24. [(4-{[4-benzyl-5-(4-methylpiperidin-1-yl)-oxazol-2-yl]-piperidin-1-yl-methyl} -cyclohexyl)-thiophen-2-yl-methyl]-dimethylamine;
25. benzyl-(4-benzyl-2-{[4-(dimethylaminothiophen-2-yl-methyl)-cyclohexyl]-piperidin-1-yl-methyl}-oxazol-5-yl)-methylamine;
26. [(4-{[4-benzyl-5-(4-phenylpiperazin-1-yl)-oxazol-2-yl]-piperidin-1-yl-methyl}-cyclohexyl)-thiophen-2-yl-methyl]-dimethylamine;
27. {[4-benzyl-5-(4-benzylpiperidin-1-yl)-oxazol-2-yl]-[4-(dimethyl-aminothiophen-2-yl-methyl)-cyclohexyl]-methyl}-diethylamine;
28. {[4-benzyl-5-(4-benzylpiperazin-1-yl)-oxazol-2-yl]-[4-(dimethylamino-thiophen-2-yl-methyl)-cyclohexyl]-methyl}-diethylamine;
29. {[4-benzyl-5-(4-methylpiperidin-1-yl)-oxazol-2-yl]-[4-(dimethyl-aminothiophen-2-yl-methyl)-cyclohexyl]-methyl}-diethylamine;
30. benzyl-(4-benzyl-2-{diethylamino-[4-(dimethylaminothiophen-2-yl-methyl)-cyclohexyl]-methyl}-oxazol-5-yl)-methylamine;
31. {[4-benzyl-5-(4-phenylpiperazin-1-yl)-oxazol-2-yl]-[4-(dimethylamino-thiophen-2-yl-methyl)-cyclohexyl]-methyl}-diethylamine;
32. ({4-[[4-benzyl-5-(4-benzylpiperidin-1-yl)-oxazol-2-yl]-(4-benzyl-piperazin-1-yl)-methyl]-cyclohexyl}-thiophen-2-yl-methyl)-dimethylamine;
33. [(4-{[4-benzyl-5-(3-methylpiperidin-1-yl)-oxazol-2-yl]-piperidin-1-yl-methyl}-cyclohexyl)-(4-fluorophenyl)-methyl]-dimethylamine;
34. [4-benzyl-2-(diethylamino-{4-[dimethylamino-(4-fluorophenyl)-methyl]-cyclohexyl}-methyl)-oxazol-5-yl]-methylphenethylamine;
35. [4-benzyl-2-(diethylamino-{4-[dimethylamino-(4-fluorophenyl)-methyl]-cyclohexyl}-methyl)-oxazol-5-yl]-diethylamine;
36. ([4-benzyl-5-(3-methylpiperidin-1-yl)-oxazol-2-yl]-{4-[dimethylamino-(4-fluorophenyl)-methyl]-cyclohexyl}-methyl)-diethylamine;
37. ([4-benzyl-5-(3,5-dimethylpiperidin-1-yl)-oxazol-2-yl]-{4-[dimethyl-amino-(4-fluorophenyl)-methyl]-cyclohexyl}-methyl)-methylphenylamine;
38. {4-benzyl-2-[{4-[dimethylamino-(4-fluorophenyl)-methyl]-cyclohexyl}-(methylphenylamino)-methyl]-oxazol-5-yl}-methylphenethylamine;
39. [4-benzyl-2-({4-[(4-chlorophenyl)-dimethylaminomethyl]-cyclohexyl}-piperidin-1-yl-methyl)-oxazol-5-yl]-diethylamine;
40. [(4-{[4-benzyl-5-(3-methylpiperidin-1-yl)-oxazol-2-yl]-piperidin-1-yl-methyl}-cyclohexyl)-(4-chlorophenyl)-methyl]-dimethylamine;
41. ([4-benzyl-5-(3,5-dimethylpiperidin-1-yl)-oxazol-2-yl]-{4-[(4-chlorophenyl)-dimethylaminomethyl]-cyclohexyl}-methyl)-diethylamine;
42. ([4-benzyl-5-(3,5-dimethylpiperidin-1-yl)-oxazol-2-yl]-{4-[(4-chlorophenyl)-dimethylaminomethyl]-cyclohexyl}-methyl)-methylphenylamine;
43. {4-benzyl-2-[{4-[(4-chlorophenyl)-dimethylaminomethyl]-cyclohexyl}-(methylphenylamino)-methyl]-oxazol-5-yl}-methylphenethylamine;
44. [4-benzyl-2-((4-benzylpiperazin-1-yl)-{4-[(4-chlorophenyl)-dimethylaminomethyl]-cyclohexyl}-methyl)-oxazol-5-yl]-diethylamine;
45. [(4-{[4-benzyl-5-(3,5-dimethylpiperidin-1-yl)-oxazol-2-yl]-piperidin-1-yl-methyl}-cyclohexyl)-thiophen-2-yl-methyl]-dimethylamine;
46. (4-benzyl-2-{[4-(dimethylaminothiophen-2-yl-methyl)-cyclohexyl]-piperidin-1-yl-methyl}-oxazol-5-yl)-methylphenethylamine;
47. (4-benzyl-2-{[4-(dimethylaminothiophen-2-yl-methyl)-cyclohexyl]-piperidin-1-yl-methyl}-oxazol-5-yl)-diethylamine;
48. ({4-[(4-benzyl-5-thiomorpholin-4-yl-oxazol-2-yl)-piperidin-1-yl-methyl]-cyclohexyl}-thiophen-2-yl-methyl)-dimethylamine;
49. (4-benzyl-2-{diethylamino-[4-(dimethylaminothiophen-2-yl-methyl)-cyclohexyl]-methyl}-oxazol-5-yl)-methylphenethylamine;
50. (4-benzyl-2-{diethylamino-[4-(dimethylaminothiophen-2-yl-methyl)-cyclohexyl]-methyl}-oxazol-5-yl)-diethylamine;
51. {[4-benzyl-5-(3-methylpiperidin-1-yl)-oxazol-2-yl]-[4-(dimethylamino-thiophen-2-yl-methyl)-cyclohexyl]-methyl}-diethylamine;
52. {(4-benzyl-5-thiomorpholin-4-yl-oxazol-2-yl)-[4-(dimethylaminothio-phen-2-yl-methyl)-cyclohexyl]-methyl} -methylphenylamine;
53. ({4-[[4-benzyl-5-(3,5-dimethylpiperidin-1-yl)-oxazol-2-yl]-(4-benzyl-piperazin-1-yl)-methyl]-cyclohexyl}-thiophen-2-yl-methyl)-dimethylamine;
54. (4-benzyl-2-{(4-benzylpiperazin-1-yl)-[4-(dimethylaminothiophen-2-yl-methyl)-cyclohexyl]-methyl}-oxazol-5-yl)-methylphenethylamine;
55. ({4-[(4-benzylpiperazin-1-yl)-(4-benzyl-5-thiomorpholin-4-yl-oxazol-2-yl)-methyl]-cyclohexyl} -thiophen-2-yl-methyl)-dimethylamine;
56. [(4-{2-[4-benzyl-5-(4-benzylpiperazin-1-yl)-oxazol-2-yl]-2-piperidin-1-yl-ethyl}-cyclohexyl)-(4-chlorophenyl)-methyl]-dimethylamine;
57. [(4-{2-[4-benzyl-5-(4-methylpiperidin-1-yl)-oxazol-2-yl]-2-piperidin-1-yl-ethyl}-cyclohexyl)-(4-chlorophenyl)-methyl]-dimethylamine;
58. benzyl-[4-benzyl-2-(2-{4-[(4-chlorophenyl)-dimethylaminomethyl]-cyclohexyl}-1-piperidin-1-yl-ethyl)-oxazol-5-yl]-methylamine;
59. (1-[4-benzyl-5-(4-benzylpiperidin-1-yl)-oxazol-2-yl]-2-{4-[(4-chlorophenyl)-dimethylaminomethyl]-cyclohexyl}-ethyl)-diethylamine;
60. (1-[4-benzyl-5-(4-methylpiperidin-1-yl)-oxazol-2-yl]-2-{4-[(4-chlorophenyl)-dimethylaminomethyl]-cyclohexyl}-ethyl)-diethylamine;
61. benzyl-[4-benzyl-2-(2-{4-[(4-chlorophenyl)-dimethylaminomethyl]-cyclohexyl}-1-diethylaminoethyl)-oxazol-5-yl]-methylamine;
62. [{4-[2-[4-benzyl-5-(4-benzylpiperidin-1-yl)-oxazol-2-yl]-2-(4-benzyl-piperazin-1-yl)-ethyl]-cyclohexyl}-(4-chlorophenyl)-methyl]-dimethylamine;
63. [{4-[2-[4-benzyl-5-(4-benzylpiperazin-1-yl)-oxazol-2-yl]-2-(4-benzyl-piperazin-1-yl)-ethyl]-cyclohexyl}-(4-chlorophenyl)-methyl]-dimethylamine;
64. [{4-[2-[4-benzyl-5-(4-methylpiperidin-1-yl)-oxazol-2-yl]-2-(4-benzyl-piperazin-1-yl)-ethyl]-cyclohexyl}-(4-chlorophenyl)-methyl]-dimethylamine;
65. [(4-{2-[4-benzyl-5-(4-methylpiperidin-1-yl)-oxazol-2-yl]-2-piperidin-1-yl-ethyl}-cyclohexyl)-(4-fluorophenyl)-methyl]-dimethylamine;
66. benzyl-[4-benzyl-2-(2-{4-[dimethylamino-(4-fluorophenyl)-methyl]-cyclohexyl}-1-piperidin-1-yl-ethyl)-oxazol-5-yl]-methylamine;
67. (1-[4-benzyl-5-(4-benzylpiperidin-1-yl)-oxazol-2-yl]-2-{4-[dimethyl-amino-(4-fluorophenyl)-methyl]-cyclohexyl}-ethyl)-diethylamine;
68. (1-[4-benzyl-5-(4-benzylpiperazin-1-yl)-oxazol-2-yl]-2-{4-[dimethyl-amino-(4-fluorophenyl)-methyl]-cyclohexyl}-ethyl)-diethylamine;
69. (1-[4-benzyl-5-(4-methylpiperidin-1-yl)-oxazol-2-yl]-2-{4-[dimethyl-amino-(4-fluorophenyl)-methyl]-cyclohexyl}-ethyl)-diethylamine;
70. benzyl-[4-benzyl-2-(1-diethylamino-2-{4-[dimethylamino-(4-fluorophenyl)-methyl]-cyclohexyl}-ethyl)-oxazol-5-yl]-methylamine;
71. [{4-[2-[4-benzyl-5-(4-methylpiperidin-1-yl)-oxazol-2-yl]-2-(4-benzyl-piperazin-1-yl)-ethyl]-cyclohexyl}-(4-fluorophenyl)-methyl]-dimethylamine;
72. [(4-{2-[4-benzyl-5-(4-benzylpiperidin-1-yl)-oxazol-2-yl]-2-piperidin-1-yl-ethyl}-cyclohexyl)-thiophen-2-yl-methyl]-dimethylamine;
73. [(4-{2-[4-benzyl-5-(4-benzylpiperazin-1-yl)-oxazol-2-yl]-2-piperidin-1-yl-ethyl}-cyclohexyl)-thiophen-2-yl-methyl]-dimethylamine;
74. [(4-{2-[4-benzyl-5-(4-methylpiperidin-1-yl)-oxazol-2-yl]-2-piperidin-1-yl-ethyl}-cyclohexyl)-thiophen-2-yl-methyl]-dimethylamine;
75. benzyl-(4-benzyl-2-{2-[4-(dimethylaminothiophen-2-yl-methyl)-cyclohexyl]-1-piperidin-1-yl-ethyl}-oxazol-5-yl)-methylamine;
76. [(4-{2-[4-benzyl-5-(4-phenylpiperazin-1-yl)-oxazol-2-yl]-2-piperidin-1-yl-ethyl}-cyclohexyl)-thiophen-2-yl-methyl]-dimethylamine;
77. {1-[4-benzyl-5-(4-benzylpiperidin-1-yl)-oxazol-2-yl]-2-[4-(dimethyl-aminothiophen-2-yl-methyl)-cyclohexyl]-ethyl}-diethylamine;
78. {1 -[4-benzyl-5-(4-benzylpiperazin-1-yl)-oxazol-2-yl]-2-[4-(dimethyl-aminothiophen-2-yl-methyl)-cyclohexyl]-ethyl}-diethylamine;
79. {1-[4-benzyl-5-(4-methylpiperidin-1-yl)-oxazol-2-yl]-2-[4-(dimethyl-aminothiophen-2-yl-methyl)-cyclohexyl]-ethyl}-diethylamine;
80. benzyl-(4-benzyl-2-{1-diethylamino-2-[4-(dimethylaminothiophen-2-yl-methyl)-cyclohexyl]-ethyl}-oxazol-5-yl)-methylamine;
81. ({4-[2-[4-benzyl-5-(4-benzylpiperidin-1-yl)-oxazol-2-yl]-2-(4-benzyl-piperazin-1-yl)-ethyl]-cyclohexyl}-thiophen-2-yl-methyl)-dimethylamine;
82. ({4-[2-[4-benzyl-5-(4-benzylpiperazin-1-yl)-oxazol-2-yl]-2-(4-benzylpiperazin-1-yl)-ethyl]-cyclohexyl}-thiophen-2-yl-methyl)-dimethylamine;
83. ({4-[2-[4-benzyl-5-(4-methylpiperidin-1-yl)-oxazol-2-yl]-2-(4-benzylpiperazin-1-yl)-ethyl]-cyclohexyl}-thiophen-2-yl-methyl)-dimethylamine;
84. benzyl-(4-benzyl-2-{1-(4-benzylpiperazin-1-yl)-2-[4-(dimethyl-aminothiophen-2-yl-methyl)-cyclohexyl]-ethyl}-oxazol-5-yl)-methylamine;
85. ({4-[2-[4-benzyl-5-(4-phenylpiperazin-1-yl)-oxazol-2-yl]-2-(4-benzylpiperazin-1-yl)-ethyl]-cyclohexyl}-thiophen-2-yl-methyl)-dimethylamine;
86. [4-benzyl-2-(2-{4-[(4-chlorophenyl)-dimethylaminomethyl]-cyclohexyl}-1-piperidin-1-yl-ethyl)-oxazol-5-yl]-methylphenethylamine;
87. [4-benzyl-2-(2-{4-[(4-chlorophenyl)-dimethylaminomethyl]-cyclohexyl}-1-piperidin-1-yl-ethyl)-oxazol-5-yl]-diethylamine;
88. (1-[4-benzyl-5-(3,5-dimethylpiperidin-1-yl)-oxazol-2-yl]-2-{4-[(4-chlorophenyl)-dimethylaminomethyl]-cyclohexyl}-ethyl)-diethylamine;
89. [4-benzyl-2-(2-{4-[(4-chlorophenyl)-dimethylaminomethyl]-cyclohexyl}-1-diethylaminoethyl)-oxazol-5-yl]-methylphenethylamine;
90. [4-benzyl-2-(2-{4-[(4-chlorophenyl)-dimethylaminomethyl]-cyclohexyl}-1-diethylaminoethyl)-oxazol-5-yl]-diethylamine;
91. (1-(4-benzyl-5-thiomorpholin-4-yl-oxazol-2-yl)-2-{4-[(4-chlorophenyl)-dimethylaminomethyl]-cyclohexyl}-ethyl)-diethylamine;
92. (1-(4-benzyl-5-thiomorpholin-4-yl-oxazol-2-yl)-2-{4-[(4-chlorophenyl)-dimethylaminomethyl]-cyclohexyl}-ethyl)-methylphenylamine;
93. [4-benzyl-2-(1-(4-benzylpiperazin-1-yl)-2-{4-[(4-chlorophenyl)-dimethylaminomethyl]-cyclohexyl}-ethyl)-oxazol-5-yl]-methylphenethylamine;
94. [4-benzyl-2-(1-(4-benzylpiperazin-1-yl)-2-{4-[(4-chlorophenyl)-dimethylaminomethyl]-cyclohexyl}-ethyl)-oxazol-5-yl]-diethylamine;
95. [4-benzyl-2-(2-{4-[dimethylamino-(4-fluorophenyl)-methyl]-cyclohexyl}-1-piperidin-1-yl-ethyl)-oxazol-5-yl]-methylphenethylamine;
96. (1-[4-benzyl-5-(3,5-dimethylpiperidin-1-yl)-oxazol-2-yl]-2-{4-[dimethylamino-(4-fluorophenyl)-methyl]-cyclohexyl}-ethyl)-diethylamine;
97. [4-benzyl-2-(1-diethylamino-2-{4-[dimethylamino-(4-fluorophenyl)-methyl]cyclohexyl}-ethyl)-oxazol-5-yl]-methylphenethylamine;
98. [4-benzyl-2-(1-diethylamino-2-{4-[dimethylamino-(4-fluorophenyl)-methyl]cyclohexyl} -ethyl)-oxazol-5-yl]-diethylamine;
99. (1-(4-benzyl-5-thiomorpholin-4-yl-oxazol-2-yl)-2-{4-[dimethylamino-(4-fluorophenyl)-methyl]-cyclohexyl}-ethyl)-diethylamine;
100. (1-[4-benzyl-5-(3-methylpiperidin-1-yl)-oxazol-2-yl]-2-{4-[dimethyl-amino(4-fluorophenyl)-methyl]-cyclohexyl}-ethyl)-diethylamine;
101. (1-[4-benzyl-5-(3,5-dimethylpiperidin-1-yl)-oxazol-2-yl]-2-{4-[dimethyl-amino-(4-fluorophenyl)-methyl]-cyclohexyl}-ethyl)-methylphenylamine;
102. {4-benzyl-2-[2-{4-[dimethylamino-(4-fluorophenyl)-methyl]-cyclohexyl}-1-(methylphenylamino)-ethyl]-oxazol-5-yl}-methylphenethylamine;
103. {4-benzyl-2-[2-{4-[dimethylamino-(4-fluorophenyl)-methyl]-cyclohexyl}-1-(methylphenylamino)-ethyl]-oxazol-5-yl}-diethylamine;
104. (1-(4-benzyl-5-thiomorpholin-4-yl-oxazol-2-yl)-2-{4-[dimethylamino-(4-fluorophenyl)-methyl]-cyclohexyl}-ethyl)-methylphenylamine;
105. (1-[4-benzyl-5-(3-methylpiperidin-1-yl)-oxazol-2-yl]-2-{4-[dimethyl-amino(4-fluorophenyl)-methyl]-cyclohexyl}-ethyl)-methylphenylamine;
106. [{4-[2-[4-benzyl-5-(3-methylpiperidin-1-yl)-oxazol-2-yl]-2-(4-benzyl-piperazin-1-yl)-ethyl]-cyclohexyl}-(4-fluorophenyl)-methyl]-dimethylamine;
107. [(4-{2-[4-benzyl-5-(3,5-dimethylpiperidin-1-yl)-oxazol-2-yl]-2-piperidin-1-yl-ethyl}-cyclohexyl)-thiophen-2-yl-methyl]-dimethylamine;
108. (4-benzyl-2-{2-[4-(dimethylaminothiophen-2-yl-methyl)-cyclohexyl]-1-piperidin-1-yl-ethyl}-oxazol-5-yl)-methylphenethylamine;
109. (4-benzyl-2-{2-[4-(dimethylaminothiophen-2-yl-methyl)-cyclohexyl]-1-piperidin-1-yl-ethyl}-oxazol-5-yl)-diethylamine;
110. ({4-[2-(4-benzyl-5-thiomorpholin-4-yl-oxazol-2-yl)-2-piperidin-1-yl-ethyl]-cyclohexyl}-thiophen-2-yl-methyl)-dimethylamine;
111. [(4-{2-[4-benzyl-5-(3-methylpiperidin-1-yl)-oxazol-2-yl]-2-piperidin-1-yl-ethyl}-cyclohexyl)-thiophen-2-yl-methyl]-dimethylamine;
112. {1-[4-benzyl-5-(3,5-dimethylpiperidin-1-yl)-oxazol-2-yl]-2-[4-(dimethylaminothiophen-2-yl-methyl)-cyclohexyl]-ethyl}-diethylamine;
113. (4-benzyl-2-{1-diethylamino-2-[4-(dimethylaminothiophen-2-yl-methyl)-cyclohexyl]-ethyl}-oxazol-5-yl)-methylphenethylamine;
114. (4-benzyl-2-{1-diethylamino-2-[4-(dimethylaminothiophen-2-yl-methyl)-cyclohexyl]-ethyl}-oxazol-5-yl)-diethylamine;
115. {1-(4-benzyl-5-thiomorpholin-4-yl-oxazol-2-yl)-2-[4-(dimethyl-aminothiophen-2-yl-methyl)-cyclohexyl]-ethyl}-diethylamine;
116. {1-[4-benzyl-5-(3-methylpiperidin-1-yl)-oxazol-2-yl]-2-[4-(dimethyl-aminothiophen-2-yl-methyl)-cyclohexyl]-ethyl}-diethylamine;
117. {1-[4-benzyl-5-(3-methylpiperidin-1-yl)-oxazol-2-yl]-2-[4-(dimethyl-aminothiophen-2-yl-methyl)-cyclohexyl]-ethyl}-methylphenylamine;
118. ({4-[2-(4-benzylpiperazin-1-yl)-2-(4-benzyl-5-thiomorpholin-4-yl-oxazol-2-yl)-ethyl]-cyclohexyl}-thiophen-2-yl-methyl)-dimethylamine;
119. ({4-[2-[4-benzyl-5-(3-methylpiperidin-1-yl)-oxazol-2-yl]-2-(4-benzyl-piperazin-1-yl)-ethyl]-cyclohexyl}-thiophen-2-yl-methyl)-dimethylamine;
120. [{4-[(4-benzyl-5-pyrrolidin-1-yl-oxazol-2-yl)-morpholin-4-yl-methyl]-cyclohexyl}-(3-fluorophenyl)-methyl]-dimethylamine;
121. [{4-[(4-benzyl-5-morpholin-4-yl-oxazol-2-yl)-morpholin-4-yl-methyl]-cyclohexyl}-(3-fluorophenyl)-methyl]-dimethylamine;
122. [(4-{(4-benzyl-5-pyrrolidin-1-yl-oxazol-2-yl)-[4-(4-fluorophenyl)-piperazin-1-yl]-methyl }-cyclohexyl)-(3-fluorophenyl)-methyl]-dimethylamine;
123. [(4-{(4-benzyl-5-pyrrolidin-1-yl-oxazol-2-yl)-[4-(4-methoxyphenyl)-piperazin-1-yl]-methyl}-cyclohexyl)-(3-fluorophenyl)-methyl]-dimethylamine;
124. [(4-{(4-benzyl-5-morpholin-4-yl-oxazol-2-yl)-[4-(4-methoxyphenyl)-piperazin-1-yl]-methyl}-cyclohexyl)-(3-fluorophenyl)-methyl]-dimethylamine;
125. [{4-[(4-benzyl-5-pyrrolidin-1-yl-oxazol-2-yl)-(3,4-dihydro-1H-isoquinolin-2-yl)-methyl]-cyclohexyl}-(3-fluorophenyl)-methyl]-dimethylamine;
126. [{4-[(4-benzyl-5-morpholin-4-yl-oxazol-2-yl)-(3,4-dihydro-1H-isoquinolin-2-yl)-methyl]-cyclohexyl}-(3-fluorophenyl)-methyl]-dimethylamine;
127. [{4-[{4-benzyl-5-[4-(4-methoxyphenyl)-piperazin-1-yl]-oxazol-2-yl}-(3,4-dihydro-1H-isoquinolin-2-yl)-methyl]-cyclohexyl}-(3-fluorophenyl)-methyl]-dimethylamine;
128. [{4-[[4-benzyl-5-(2,6-dimethylmorpholin-4-yl)-oxazol-2-yl]-(3,4-dihydro-1H-isoquinolin-2-yl)-methyl]-cyclohexyl}-(3-fluorophenyl)-methyl]-dimethylamine;
129. [{4-[(4-benzyl-5-pyrrolidin-1-yl-oxazol-2-yl)-morpholin-4-yl-methyl]-cyclohexyl}-(4-chlorophenyl)-methyl]-dimethylamine;
130. [{4-[(4-benzyl-5-morpholin-4-yl-oxazol-2-yl)-morpholin-4-yl-methyl]-cyclohexyl}-(4-chlorophenyl)-methyl]-dimethylamine;
131. [[4-({4-benzyl-5-[4-(4-methoxyphenyl)-piperazin-1-yl]-oxazol-2-yl}-morpholin-4-yl-methyl)-cyclohexyl]-(4-chlorophenyl)-methyl]-dimethylamine;
132. [(4-{[4-benzyl-5-(2,6-dimethylmorpholin-4-yl)-oxazol-2-yl]-morpholin-4-yl-methyl}-cyclohexyl)-(4-chlorophenyl)-methyl]-dimethylamine;
133. [(4-{(4-benzyl-5-pyrrolidin-1-yl-oxazol-2-yl)-[4-(4-fluorophenyl)-piperazin-1-yl]-methyl}-cyclohexyl)-(4-chlorophenyl)-methyl]-dimethylamine;
134. [(4-{(4-benzyl-5-pyrrolidin-1-yl-oxazol-2-yl)-[4-(4-methoxyphenyl)-piperazin-1-yl]-methyl}-cyclohexyl)-(4-chlorophenyl)-methyl]-dimethylamine;
135. [(4-{(4-benzyl-5-morpholin-4-yl-oxazol-2-yl)-[4-(4-methoxyphenyl)-piperazin-1-yl]-methyl}-cyclohexyl)-(4-chlorophenyl)-methyl]-dimethylamine;
136. [(4-{[4-benzyl-5-(2,6-dimethylmorpholin-4-yl)-oxazol-2-yl]-[4-(4-methoxyphenyl)-piperazin-1-yl]-methyl}-cyclohexyl)-(4-chlorophenyl)-methyl]-dimethylamine;
137. [{4-[(4-benzyl-5-pyrrolidin-1-yl-oxazol-2-yl)-(3,4-dihydro-1H-isoquinolin-2-yl)-methyl]-cyclohexyl}-(4-chlorophenyl)-methyl]-dimethylamine;
138. ({4-[(4-benzyl-5-pyrrolidin-1-yl-oxazol-2-yl)-morpholin-4-yl-methyl]-cyclohexyl}-thiophen-2-yl-methyl)-dimethylamine;
139. ({4-[(4-benzyl-5-morpholin-4-yl-oxazol-2-yl)-morpholin-4-yl-methyl]-cyclohexyl}-thiophen-2-yl-methyl)-dimethylamine;
140. {[4-({4-benzyl-5-[4-(4-methoxyphenyl)-piperazin-1-yl]-oxazol-2-yl}-morpholin-4-yl-methyl)-cyclohexyl]-thiophen-2-yl-methyl}-dimethylamine;
141. [(4-{[4-benzyl-5-(2,6-dimethylmorpholin-4-yl)-oxazol-2-yl]-morpholin-4-yl-methyl}-cyclohexyl)-thiophen-2-yl-methyl]-dimethylamine;
142. [(4-{(4-benzyl-5-pyrrolidin-1-yl-oxazol-2-yl)-[4-(4-fluorophenyl)-piperazin-1-yl]-methyl}-cyclohexyl)-thiophen-2-yl-methyl]-dimethylamine
143. [(4-{(4-benzyl-5-pyrrolidin-1-yl-oxazol-2-yl)-[4-(4-methoxyphenyl)-piperazin-1-yl]-methyl}-cyclohexyl)-thiophen-2-yl-methyl]-dimethylamine;
144. [(4-{(4-benzyl-5-morpholin-4-yl-oxazol-2-yl)-[4-(4-methoxyphenyl)-piperazin-1-yl]-methyl}-cyclohexyl)-thiophen-2-yl-methyl]-dimethylamine;
145. [(4-{[4-benzyl-5-(2,6-dimethylmorpholin-4-yl)-oxazol-2-yl]-[4-(4-methoxyphenyl)-piperazin-1-yl]-methyl}-cyclohexyl)-thiophen-2-yl-methyl]-dimethylamine;
146. ({4-[(4-benzyl-5-pyrrolidin-1-yl-oxazol-2-yl)-(3,4-dihydro-1H-isoquinolin-2-yl)-methyl]-cyclohexyl}-thiophen-2-yl-methyl)-dimethylamine;
147. ({4-[{4-benzyl-5-[4-(4-methoxyphenyl)-piperazin-1-yl]-oxazol-2-yl}-(3,4-dihydro-1H-isoquinolin-2-yl)-methyl]-cyclohexyl}-thiophen-2-yl-methyl)-dimethylamine;
148. ({4-[[4-benzyl-5-(2,6-dimethylmorpholin-4-yl)-oxazol-2-yl]-(3,4-dihydro-1H-isoquinolin-2-yl)-methyl]-cyclohexyl}-thiophen-2-yl-methyl)-dimethylamine;
149. [{4-[2-(4-benzyl-5-pyrrolidin-1-yl-oxazol-2-yl)-2-morpholin-4-yl-ethyl]-cyclohexyl}-(3-fluorophenyl)-methyl]-dimethylamine;
150. [{4-[2-(4-benzyl-5-morpholin-4-yl-oxazol-2-yl)-2-morpholin-4-yl-ethyl]-cyclohexyl}-(3-fluorophenyl)-methyl]-dimethylamine;
151. [[4-(2-{4-benzyl-5-[4-(4-methoxyphenyl)-piperazin-1-yl]-oxazol-2-yl}-2-morpholin-4-yl-ethyl)-cyclohexyl]-(3-fluorophenyl)-methyl]-dimethylamine;
152. [(4-{2-[4-benzyl-5-(2,6-dimethylmorpholin-4-yl)-oxazol-2-yl]-2-morpholin-4-yl-ethyl}-cyclohexyl)-(3-fluorophenyl)-methyl]-dimethylamine;
153. [(4-{2-[4-benzyl-5-(2,6-dimethylmorpholin-4-yl)-oxazol-2-yl]-2-[4-(4-fluorophenyl)-piperazin-1-yl]-ethyl}-cyclohexyl)-(3-fluorophenyl)-methyl]-dimethylamine;
154. [(4-{2-(4-benzyl-5-morpholin-4-yl-oxazol-2-yl)-2-[4-(4-methoxyphenyl)-piperazin-1-yl]-ethyl}-cyclohexyl)-(3-fluorophenyl)-methyl]-dimethylamine;
155. [(4-{2-{4-benzyl-5-[4-(4-methoxyphenyl)-piperazin-1-yl]-oxazol-2-yl}-2-[4-(4-methoxyphenyl)-piperazin-1-yl]-ethyl}-cyclohexyl)-(3-fluorophenyl)-methyl]-dimethylamine;
156. [(4-{2-[4-benzyl-5-(2,6-dimethylmorpholin-4-yl)-oxazol-2-yl]-2-[4-(4-methoxyphenyl)-piperazin-1-yl]-ethyl}-cyclohexyl)-(3-fluorophenyl)-methyl]-dimethylamine;
157. [{4-[2-{4-benzyl-5-[4-(4-methoxyphenyl)-piperazin-1-yl]-oxazol-2-yl}-2-(3,4-dihydro-1H-isoquinolin-2-yl)-ethyl]-cyclohexyl}-(3-fluorophenyl)-methyl]-dimethylamine;
158. ({4-[2-(4-benzyl-5-morpholin-4-yl-oxazol-2-yl)-2-morpholin-4-yl-ethyl]-cyclohexyl}-thiophen-2-yl-methyl)-dimethylamine;
159. {[4-(2-{4-benzyl-5-[4-(4-methoxyphenyl)-piperazin-1-yl]-oxazol-2-yl}-2-morpholin-4-yl-ethyl)-cyclohexyl]-thiophen-2-yl-methyl}-dimethylamine;
160. [(4-{2-[4-benzyl-5-(2,6-dimethylmorpholin-4-yl)-oxazol-2-yl]-2-morpholin-4-yl-ethyl}-cyclohexyl)-thiophen-2-yl-methyl]-dimethylamine;
161. [(4-{2-(4-benzyl-5-morpholin-4-yl-oxazol-2-yl)-2-[4-(4-fluorophenyl)-piperazin-1-yl]-ethyl}-cyclohexyl)-thiophen-2-yl-methyl]-dimethylamine;
162. [(4-{2-{4-benzyl-5-[4-(4-methoxyphenyl)-piperazin-1-yl]-oxazol-2-yl}-2-[4-(4-fluorophenyl)-piperazin-1-yl]-ethyl}-cyclohexyl)-thiophen-2-yl-methyl]-dimethylamine;
163. [(4-{2-(4-benzyl-5-pyrrolidin-1-yl-oxazol-2-yl)-2-[4-(4-methoxyphenyl)-piperazin-1-yl]-ethyl}-cyclohexyl)-thiophen-2-yl-methyl]-dimethylamine;
164. [(4-{2-{4-benzyl-5-[4-(4-methoxyphenyl)-piperazin-1-yl]-oxazol-2-yl}-2-[4-(4-methoxyphenyl)-piperazin-1-yl]-ethyl}-cyclohexyl)-thiophen-2-yl-methyl]-dimethylamine;
165. [(4-{2-[4-benzyl-5-(2,6-dimethylmorpholin-4-yl)-oxazol-2-yl]-2-[4-(4-methoxyphenyl)-piperazin-1-yl]-ethyl}-cyclohexyl)-thiophen-2-yl-methyl]-dimethylamine; and
166. ({4-[2-{4-benzyl-5-[4-(4-methoxyphenyl)-piperazin-1-yl]-oxazol-2-yl}-2-(3,4-dihydro-1H-isoquinolin-2-yl)-ethyl]-cyclohexyl}-thiophen-2-yl-methyl)-dimethylamine.

3. Process for preparing a substituted oxazole derivative according to Claim 1, said process comprising heating aldehydes of the general formula **A** with amines of the general formula **B** and isonitrile amides of the general formula **C** 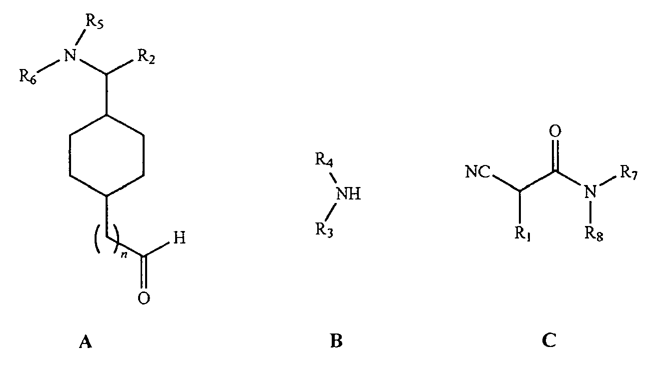 in an organic solvent, for example methanol or ethanol, for 1-10 hours at a temperature between 30° and 100°C, preferably 40-80°C.

4. Medicament comprising at least one substituted oxazole derivate according to Claim 1, optionally in the form of the racemate; of the enantiomers, diasteromers, mixtures of the enantiomers or of diastereomers or of an individual enantiomer or diasteromer; of the bases and/or salts of physiologically acceptable acids, and optionally comprising suitable additives and/or auxiliaries and/or optional further active ingredients.

5. Use of substituted oxazole derivate according to Claim 1, optionally in the form of the racemate; of the enantiomers, diasteromers, mixtures of the enantiomers or diastereomers or of an individual enantiomer or diasteromer; of the bases and/or salts of physiologically acceptable acids, for production of a medicament for treatment of pain, especially of acute, neuropathic or chronic pain.

6. Use of substituted oxazole derivate according to Claim 1, optionally in the form of the racemate; of the enantiomers, diasteromers, mixtures of the enantiomers or diastereomers or of an individual enantiomer or diasteromer; of the bases and/or salts of physiologically acceptable acids, for production of a medicament for treating depression, urinary incontinence, diarrhoea, pruritus, alcohol and drug misuse, prescription drug dependency, lethargy and/or for treatment of anxiety.

## Revendications

1. Dérivés substitués de l'oxazole de formule générale I 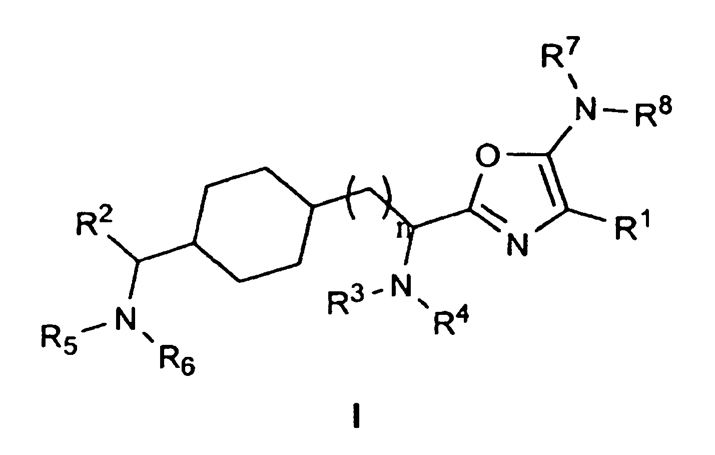 dans laquelle
n vaut 0 ou 1,
R¹ est le groupe benzyle,
R² est le groupe phényle, non substitué ou une fois substitué par Cl ou F ou un substituant thiényle,
R³ et R⁴ représentent chacun indépendamment de l'autre un groupe phényle, éthyle ou méthyle, ou les radicaux R³ et R⁴ forment ensemble un radical CH₂CH₂OCH₂CH₂, CH_{z2}CH₂NR⁹CH₂CH₂, (CH₂) ₄₋₅ ou 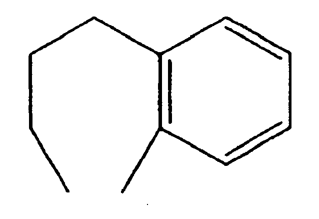 où R⁹ est le groupe benzyle, 4-F-phényle ou 4-méthoxyphényle,
R⁵ et R⁶ représentent CH₃,
R⁷ et R⁸ représentent chacun indépendamment de l'autre un groupe méthyle, éthyle, benzyle ou phénéthyle ;
ou les radicaux R⁷ et R⁸ représentent CH₂CH₂SCH₂CH₂, CH₂CH₂OCH₂CH₂, (CH₂)₄ ou (CH₂) ₅, CH₂CH₂NR¹⁰CH₂CH₂, les atomes d'hydrogène individuels pouvant être remplacés par des groupes méthyle ou benzyle, et R¹⁰ est le groupe phényle, 4-méthoxyphényle ou benzyle ;
sous forme du racémate ; des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles.

2. Dérivés substitués de l'oxazole selon la revendication 1, choisis dans le groupe consistant en les composés suivantes :
17. [{4-[(4-benzyl-5-pyrrolidin-1-yl-oxazol-2-yl)-pipéridin-1-yl-méthyl]-cyclohexyl}-(4-fluorophényl)-méthyl]-diméthyl-amine
18. ((4-benzyl-5-morpholin-4-yl-oxazol-2-yl)-{4-[diméthylamino-(4-fluoro-phényl)-méthyl]-cyclohexyl}-méthyl)-méthyl-phényl-amine
19. ([4-benzyl-5-(9-méthyl-pipéridin-1-yl)-oxazol-2-yl]-{4-[diméthylamino-(4-fluoro-phényl)-méthyl]-cyclohexyl}-méthyl)-méthyl-phényl-amine
20. ([4-benzyl-5-(9-méthyl-pipéridin-1-yl)-oxazol-2-yl]-(4-[(4-chloro-phényl)-diméthylamino-méthyl]-cyclohexyl)-méthyl)-diéthyl-amine
21. benzyl-[4-benzyl-2-({9-[(9-chloro-phényl)-diméthylamino-méthyl]-cyclohexyl}-diéthylamino-méthyl)-oxazol-5-yl]-méthyl-amine
22. benzyl-{4-benzyl-2-[{4-[(4-chloro-phényl)-diméthylamino-méthyl]-cyclohexyl}-(méthyl-phényl-amino)-méthyl]-oxazol-5-yl}-méthyl-amine
23. [(4-{[4-benzyl-5-(4-benzyl-pipéridin-1-yl)-oxazol-2-yl]-pipéridin-1-yl-méthyl}-cyclohexyl)-thiophèn-2-yl-méthyl]-diméthyl-amine
24. [(4-{[4-benzyl-5-(4-méthyl-pipéridin-1-yl)-oxazol-2-yl]-pipéridin-1-yl-méthyl}-cyclohexyl)-thiophèn-2-yl-méthyl]-diméthyl-amine
25. benzyl-(4-benzyl-2-{[4-(diméthylamino-thiophèn-2-yl-méthyl}-cyclohexyl]-pipéridin-1-yl-méthyl}-oxazol-5-yl)-méthyl-amine
26. [(4-{[4-benzyl-5-(4-phényl-pipérazin-1-yl)-oxazol-2-yl]-pipéridin-1-yl-méthyl}-cyclohexyl)-thiophèn-2-yl-méthyl]-diméthyl-amine
27. {[4-benzyl-5-(4-benzyl-pipéridin-1-yl)-oxazol-2-yl]-[4-(diméthylamino-thiophèn-2-yl-méthyl)-cyclohexyl]-méthyl}-diéthyl-amine
28. {[4-benzyl-5-(4-benzyl-pipérazin-1-yl)-oxazol-2-yl]-[4-(diméthylamino-thiophèn-2-yl-méthyl)-cyclohexyl]-méthyl}-diéthyl-amine
29. {[4-benzyl-5-(4-méthyl-pipéridin-1-yl)-oxazol-2-yl]-[4-(diméthylamino-thiophèn-2-yl-méthyl)-cyclohexyl]-méthyl}-diéthyl-amine
30. benzyl-(9-benzyl-2-{diéthylamino-[4-(diméthylamino-thiophèn-2-yl-méthyl)-cyclohexyl]-méthyl}-oxazol-5-yl)-méthyl-amine
31. {[4-benzyl-5-(4-phényl-pipérazin-1-yl)-oxazol-2-yl]-[4-(diméthylamino-thiophèn-2-yl-méthyl)-cyclohexyl]-méthyl}-diéthyl-amine
32. ({4-[[4-benzyl-5-(4-benzyl-pipéridin-1-yl)-oxazol-2-yl]-(4-benzyl-pipérazin-1-yl)-méthyl]-cyclohexyl}-thiophèn-2-yl-méthyl)-diméthyl-amine
33. [(4-{[4-benzyl-5-(3-méthyl-pipéridin-1-yl)-oxazol-2-yl]-pipéridin-1-yl-méthyl}-cyclohexyl)-(9-fluoro-phényl)-méthyl]-diméthyl-amine
34. [4-benzyl-2-(diéthylamino-{4-[diméthylamino-(4-fluoro-phényl)-méthyl]-cyclohexyl}-méthyl)-oxazol-5-yl]-méthyl-phénéthyl-amine
35. [4-benzyl-2-(diéthylamino-{4-[diméthylamino-(4-fluoro-phényl)-méthyl]-cyclohexyl}-méthyl)-oxazol-5-yl]-diéthyl-amine
36. ([4-benzyl-5-(3-méthyl-pipéridin-1-yl)-oxazol-2-yl]-{4-[diméthylamino-(4-fluoro-phényl)-méthyl]-cyclohexyl}-méthyl)-diéthyl-amine
37. ([4-benzyl-5-(3,5-diméthyl-pipéridin-1-yl)-oxazol-2-yl]-{4-[diméthylamino-(4-fluoro-phényl)-méthyl]-cyclohexyl}-méthyl)-méthyl-phényl-amine
38. {4-benzyl-2-[{4-[diméthylamino-(4-fluoro-phényl)-méthyl]-cyclohexyl}-(méthyl-phényl-amino)-méthyl]-oxazol-5-yl}-méthyl-phénéthyl-amine
39. [4-benzyl-2-({4-[(4-chloro-phényl)-diméthylamino-méthyl]-cyclohexyl}-pipéridin-1-yl-méthyl)-oxazol-5-yl]-diéthyl-amine
40. [(4-{[4-benzyl-5-(3-méthyl-pipéridin-1-yl)-oxazol-2-yl]-pipéridin-1-yl-méthyl}-cyclohexyl)-(4-chloro-phényl)-méthyl]-diméthyl-amine
41. ([4-benzyl-5-(3,5-diméthyl-pipéridin-1-yl)-oxazol-2-yl]-{4-[(4-chloro-phényl)-diméthylamino-méthyl]-cyclohexyl}-méthyl)-diéthyl-amine
42. ([4-benzyl-5-(3,5-diméthyl-pipéridin-1-yl)-oxazol-2-yl]-{4-[(4-chloro-phényl)-diméthylamino-méthyl]-cyclohexyl}-méthyl)-méthyl-phényl-amine
43. {4-benzyl-2-[{4-[(4-chloro-phényl)-diméthylamino-méthyl]-cyclohexyl}-(méthyl-phényl-amino)-méthyl]-oxazol-5-yl}-méthyl-phénéthyl-amine
44. [4-benzyl-2-((4-benzyl-pipérazin-1-yl)-{4-[(4-chloro-phényl)-diméthylamino-méthyl]-cyclohexyl}-méthyl)-oxazol-5-yl]-diéthyl-amine
45. [(4-{[4-benzyl-5-(3,5-diméthyl-pipéridin-1-yl)-oxazol-2-yl]-pipéridin-1-yl-méthyl}-cyclohexyl)-thiophèn-2-yl-méthyl]-diméthyl-amine
46. (4-benzyl-2-{[4-(diméthylamino-thiophèn-2-yl-méthyl)-cyclohexyl]-pipéridin-1-yl-méthyl}-oxazol-5-yl)-méthyl-phénéthyl-amine
47. (4-benzyl-2-{[4-diméthylamino-thiophèn-2-yl-méthyl)-cyclohexyl]-pipéridin-1-yl-méthyl}-oxazol-5-yl)-diéthyl-amine
48. ({4-[(4-benzyl-5-thiomorpholin-4-yl-oxazol-2-yl)-pipéridin-1-yl-méthyl]-cyclohexyl}-thiophèn-2-yl-méthyl)-diméthyl-amine
49. (4-benzyl-2-{diéthylamino-[4-(diméthylamino-thiophèn-2-yl-méthyl)-cyclohexyl]-méthyl}-oxazol-5-yl)-méthyl-phénéthyl-amine
50. (4-benzyl-2-{diéthylamino-[4-(diméthylamino-thiophèn-2-yl-méthyl)-cyclohexyl]-méthyl}-oxazol-5-yl)-diéthyl-amine
51. {[4-benzyl-5-(3-méthyl-pipéridin-1-yl)-oxazol-2-yl]-[4-(diméthylamino-thiophèn-2-yl-méthyl)-cyclohexyl]-méthyl}-diéthyl-amine
52. {(4-benzyl-5-thiomorpholin-4-yl-oxazol-2-yl)-[4-(diméthylamino-thiophèn-2-yl-méthyl)-cyclohexyl]-méthyl}-méthyl-phényl-amine
53. ({4-[[4-benzyl-5-(3,5-diméthyl-pipéridin-1-yl)-oxazol-2-yl]-(4-benzyl-pipérazin-1-yl)-méthyl]-cyclohexyl}-thiophèn-2-yl-méthyl)-diméthyl-amine
54. (4-benzyl-2-{(4-benzyl-pipérazin-1-yl)-[4-(diméthylamino-thiophèn-2-yl-méthyl)-cyclohexyl]-méthyl}-oxazol-5-yl)-méthyl-phénéthyl-amine
55. ({4-[(4-benzyl-pipérazin-1-yl)-(4-benzyl-5-thiomorpholin-4-yl-oxazol-2-yl)-méthyl]-cyclohexyl}-thiophèn-2-yl-méthyl)-diméthyl-amine
56. [(4-{2-[4-benzyl-5-(4-benzyl-pipérazin-1-yl)-oxazol-2-yl]-2-pipéridin-1-yl-éthyl}-cyclohexyl)-(4-chloro-phényl)-méthyl]-diméthyl-amine
57. [(4-{2-[4-benzyl-5-(4-méthyl-pipéridin-1-yl)-oxazol-2-yl]-2-pipéridin-1-yl-éthyl}-cyclohexyl)-(4-chloro-phényl)-méthyl]-diméthyl-amine
58. benzyl-[4-benzyl-2-(2-{4-[(4-chloro-phényl)-diméthylamino-méthyl]-cyclohexyl}-1-pipéridin-1-yl-éthyl)-oxazol-5-yl]-méthyl-amine
59. (1-[4-benzyl-5-(9-benzyl-pipéridin-1-yl)-oxazol-2-yl]-2-{4-[(4-chloro-phényl)-diméthylamino-méthyl]-cyclohexyl}-éthyl)-diéthyl-amine
60. (1-[4-benzyl-5-(4-méthyl-pipéridin-1-yl)-oxazol-2-yl]-2-{4-[(4-chloro-phényl)-diméthylamino-méthyl]-cyclohexyl}-éthyl)-diéthyl-amine
61. benzyl-[4-benzyl-2-(2-{4-[(4-chloro-phényl)-diméthylamino-méthyl]-cyclohexyl}-1-diéthylamino-éthyl)-oxazol-5-yl]-méthyl-amine
62. [{4-[2-[4-benzyl-5-(4-benzyl-pipéridin-1-yl)-oxazol-2-yl]-2-(4-benzyl-pipérazin-1-yl)-éthyl]-cyclohexyl}-(4-chloro-phényl)-méthyl]-diméthyl-amine
63. [{4-[2-[4-benzyl-5-(4-benzyl-pipérazin-1-yl)-oxazol-2-yl]-2-(4-benzyl-pipérazin-1-yl)-éthyl]-cyclohexyl}-(4-chloro-phényl)-méthyl]-diméthyl-amine
64. [{4-[2-[4-benzyl-5-(4-méthyl-pipéridin-1-yl)-oxazol-2-yl]-2-(4-benzyl-pipérazin-1-yl)-éthyl]-cyclohexyl}-(4-chloro-phényl)-méthyl]-diméthyl-amine
65. [(4-{2-[4-benzyl-5-(4-méthyl-pipéridin-1-yl)-oxazol-2-yl]-2-pipéridin-1-yl-éthyl}-cyclohexyl)-(4-fluoro-phényl)-méthyl]-diméthyl-amine
66. benzyl-[4-benzyl-2-(2-{4-[diméthylamino-(4-fluoro-phényl)-méthyl]-cyclohexyl}-1-pipéridin-1-yl-éthyl)-oxazol-5-yl]-méthyl-amine
67. (1-[4-benzyl-5-(4-benzyl-pipéridin-1-yl)-oxazol-2-yl]-2-{4-[diméthylamino-(4-fluoro-phényl)-méthyl]-cyclohexyl}-éthyl)-diéthyl-amine
68. (1-[4-benzyl-5-(4-benzyl-pipérazin-1-yl)-oxazol-2-yl]-2-{4-[diméthylamino-(4-fluoro-phényl)-méthyl]-cyclohexyl}-éthyl)-diéthyl-amine
69. (1-[4-benzyl-5-(4-méthyl-pipéridin-1-yl)-oxazol-2-yl]-2-{4-[diméthylamino-(4-fluoro-phényl)-méthyl]-cyclohexyl}-éthyl)-diéthyl-amine
70. benzyl-[4-benzyl-2-(1-diéthylamino-2-{4-[diméthylamino-(4-fluoro-phényl)-méthyl]-cyclohexyl}-éthyl)-oxazol-5-yl]-méthyl-amine
71. [{4-[2-[4-benzyl-5-(4-méthyl-pipéridin-1-yl)-oxazol-2-yl]-2-(4-benzyl-pipérazin-1-yl)-éthyl]-cyclohexyl}-(4-fluoro-phényl)-méthyl]-diméthyl-amine
72. [(4-{2-[4-benzyl-5-(4-benzyl-pipéridin-1-yl)-oxazol-2-yl]-2-pipéridin-1-yl-éthyl}-cyclohexyl)-thiophèn-2-yl-méthyl]-diméthyl-amine
73. [(4-{2-[4-benzyl-5-(4-benzyl-pipérazin-1-yl)-oxazol-2-yl]-2-pipéridin-1-yl-éthyl}-cyclohexyl)-thiophèn-2-yl-méthyl]-diméthyl-amine
74. [(4-{2-[4-benzyl-5-(4-méthyl-pipéridin-1-yl)-oxazol-2-yl]-2-pipéridin-1-yl-éthyl}-cyclohexyl)-thiophèn-2-yl-méthyl]-diméthyl-amine
75. benzyl-(4-benzyl-2-{2-[4-(diméthylamino-thiophèn-2-yl-méthyl)-cyclohexyl]-1-pipéridin-1-yl-éthyl}-oxazol-5-yl)-méthyl-amine
76. [(4-{2-[4-benzyl-5-(4-phényl-pipérazin-1-yl)-oxazol-2-yl]-2-pipéridin-1-yl-éthyl}-cyclohexyl)-thiophèn-2-yl-méthyl]-diméthyl-amine
77. {1-[4-benzyl-5-(4-benzyl-pipéridin-1-yl)-oxazol-2-yl]-2-[4-(diméthylamino-thiophèn-2-yl-méthyl)-cyclohexyl]-éthyl}-diéthyl-amine
78. {1-[4-benzyl-5-(4-benzyl-pipérazin-1-yl)-oxazol-2-yl]-2-[4-(diméthylamino-thiophèn-2-yl-méthyl)-cyclohexyl]-éthyl}-diéthyl-amine
79. {1-[4-benzyl-5-(4-méthyl-pipéridin-1-yl)-oxazol-2-yl]-2-[4-(diméthylamino-thiophèn-2-yl-méthyl)-cyclohexyl]-éthyl}-diéthyl-amine
80. benzyl-(4-benzyl-2-{1-diéthylamino-2-[4-(diméthylamino-thiophèn-2-yl-méthyl)-cyclohexyl]-éthyl}-oxazol-5-yl)-méthyl-amine
81. ({4-[2-[4-benzyl-5-(4-benzyl-pipéridin-1-yl)-oxazol-2-yl]-2-(4-benzyl-pipérazin-1-yl)-éthyl]-cyclohexyl}-thiophèn-2-yl-méthyl)-diméthyl-amine
82. ({4-[2-[4-benzyl-5-(4-benzyl-pipérazin-1-yl)-oxazol-2-yl]-2-(4-benzyl-pipérazin-1-yl)-éthyl]-cyclohexyl}-thiophèn-2-yl-méthyl)-diméthyl-amine
83. ({4-[2-[4-benzyl-5-(4-méthyl-pipéridin-1-yl)-oxazol-2-yl]-2-(4-benzyl-pipérazin-1-yl)-éthyl]-cyclohexyl}-thiophèn-2-yl-méthyl)-diméthyl-amine
84. benzyl-(4-benzyl-2-{1-(4-benzyl-pipérazin-1-yl)-2-[4-(diméthylamino-thiophèn-2-yl-méthyl)-cyclohexyl]-éthyl}-oxazol-5-yl)-méthyl-amine
85. ({4-[2-[4-benzyl-5-(4-phényl-pipérazin-1-yl)(oxazol-2-yl]-2-(4-benzyl-pipérazin-1-yl)-éthyl]-cyclohexyl}-thiophèn-2-yl-méthyl)-diméthyl-amine
86 [4-benzyl-2-(2-{4-[(4-chloro-phényl)-diméthylamino-méthyl]-cyclohexyl}-1-pipéridin-1-yl-éthyl)-oxazol-5-yl]-méthyl-phénéthyl-amine
87. [4-benzyl-2-(2-{4-[(4-chloro-phényl)-diméthylamino-méthyl]-cyclohexyl}-1-pipéridin-1-yl-éthyl)-oxazol-5-yl]-diéthyl-amine
88. (1-[4-benzyl-5-(3,5-diméthyl-pipéridin-1-yl)-oxazol-2-yl]-2-{4-[(4-chloro-phényl)-diméthylamino-méthyl]-cyclohexyl}-éthyl)-diéthyl-amine
89. [4-benzyl-2-(2-{4-[(4-chloro-phényl)-diméthylamino-méthyl]-cyclohexyl}-1-diéthylamino-éthyl)-oxazol-5-yl]-méthyl-phénéthyl-amine
90. [4-benzyl-2-(2-{4-[(4-chloro-phényl)-diméthylamino-méthyl]-cyclohexyl}-1-diéthylamino-éthyl)-oxazol-5-yl]-diéthyl-amine
91. (1-(4-benzyl-5-thiomorpholin-4-yl-oxazol-2-yl)-2-{4-[(4-chloro-phényl)-diméthylamino-méthyl]-cyclohexyl}-éthyl)-diéthyl-amine
92. (1-(4-benzyl-5-thiomorpholin-4-yl-oxazol-2-yl)-2-{4-[(4-chloro-phényl)-diméthylamino-méthyl]-cyclohexyl}-éthyl)-méthyl-phényl-amine
93. [4-benzyl-2-(1-(4-benzyl-pipérazin-1-yl)-2-{4-[(4-chloro-phényl)-diméthylamino-méthyl]-cyclohexyl}-éthyl)-oxazol-5-yl]-méthyl-phénéthyl-amine
94. [4-benzyl-2-(1-(4-benzyl-pipérazin-1-yl)-2-{4-[(4-chloro-phényl)-diméthylamino-méthyl]-cyclohexyl}-éthyl)-oxazol-5-yl]-diéthyl-amine
95. [4-benzyl-2-(2-{4-[diméthylamino-(4-fluoro-phényl)-méthyl]-cyclohexyl}-1-pipéridin-1-yl-éthyl)-oxazol-5-yl]-méthyl-phénéthyl-amine
96. (1-[4-benzyl-5-(3,5-diméthyl-pipéridin-1-yl)-oxazol-2-yl]-2-{4-[diméthylamino-(4-fluoro-phényl)-méthyl]-cyclohexyl}-éthyl)-diéthyl-amine
97. [4-benzyl-2-(1-diéthylamino-2-{9-[diméthylamino-(4-fluoro-phényl)-méthyl]-cyclohexyl}-éthyl)-oxazol-5-yl]-méthyl-phénéthyl-amine
98. [4-benzyl-2-(1-diéthylamino-2-{4-[diméthylamino-(4-fluoro-phényl)-méthyl]-cyclohexyl}-éthyl)-oxazol-5-yl]-diéthyl-amine
99. (1-(4-benzyl-5-thiomorpholin-9-yl-oxazol-2-yl)-2-{4-[diméthylamino-(4-fluoro-phényl)-méthyl]-cyclohexyl}-éthyl)-diéthyl-amine
100. (1-[4-benzyl-5-(3-méthyl-pipéridin-1-yl)-oxazol-2-yl]-2-{4-[diméthylamino-(4-fluoro-phényl)-méthyl]-cyclohexyl}-éthyl)-diéthyl-amine
101. (1-[4-benzyl-5-(3,5-diméthyl-pipéridin-1-yl)-oxazol-2-yl]-2-{4-[diméthylamino-(4-fluoro-phényl)-méthyl]-cyclohexyl}-éthyl)-méthyl-phényl-amine
102. {4-benzyl-2-[2-{4-[diméthylamino-(4-fluoro-phényl)-méthyl]-cyclohexyl}-1-(méthyl-phényl-amino)-éthyl]-oxazol-5-yl}-méthyl-phénéthyl-amine
103. {4-benzyl-2-[2-{4-[diméthylamino-(4-fluoro-phényl)-méthyl]-cyclohexyl}-1-(méthyl-phényl-amino)-éthyl]-oxazol-5-yl}-diéthyl-amine
104. (1-(4-benzyl-5-thiomorpholin-4-yl-oxazol-2-yl)-2-{4-[diméthylamino-(4-fluoro-phényl)-méthyl]-cyclohexyl}-éthyl)-méthyl-phényl-amine
105. (1-[4-benzyl-5-(3-méthyl-pipéridin-1-yl)-oxazol-2-yl]-2-{4-[diméthylamino-(4-fluoro-phényl)-méthyl]-cyclohexyl}-éthyl)-méthyl-phényl-amine
106. [{4-[2-[4-benzyl-5-(3-méthyl-pipéridin-1-yl)-oxazol-2-yl]-2-(4-benzyl-pipérazin-1-yl)-éthyl]-cyclohexyl}-(4-fluoro-phényl)-méthyl]-diméthyl-amine
107. [(4-{2-[4-benzyl-5-(3,5-diméthyl-pipéridin-1-yl)-oxazol-2-yl]-2-pipéridin-1-yl-éthyl}-cyclohexyl)-thiophèn-2-yl-méthyl]-diméthyl-amine
108. (4-benzyl-2-{2-[4-(diméthylamino-thiophèn-2-yl-méthyl)-cyclohexyl]-1-pipéridin-1-yl-éthyl}-oxazol-5-yl)-méthyl-phénéthyl-amine
109. (4-benzyl-2-{2-[4-(diméthylamino-thiophèn-2-yl-méthyl)-cyclohexyl]-1-pipéridin-1-yl-éthyl}-oxazol-5-yl)-diéthyl-amine
110. ({4-[2-(4-benzyl-5-thiomorpholin-4-yl-oxazol-2-yl)-2-pipéridin-1-yl-éthyl]-cyclohexyl}-thiophèn-2-yl-méthyl)-diméthyl-amine
111. [(4-{2-[4-benzyl-5-(3-méthyl-pipéridin-1-yl)-oxazol-2-yl]-2-pipéridin-1-yl-éthyl}-cyclohexyl)-thiophèn-2-yl-méthyl]-diméthyl-amine
112. {1-[4-benzyl-5-(3,5-diméthyl-pipéridin-1-yl)-oxazol-2-yl]-2-[4-(diméthylamino-thiophèn-2-yl-méthyl)-cyclohexyl]-éthyl}-diéthyl-amine
113. (4-benzyl-2-{1-diéthylamino-2-[4-(diméthylamino-thiophèn-2-yl-méthyl)-cyclohexyl]-éthyl}-oxazol-5-yl)-méthyl-phénéthyl-amine
114. (4-benzyl-2-{1-diéthylamino-2-[4-(diméthylamino-thiophèn-2-yl-méthyl)-cyclohexyl]-éthyl}-oxazol-5-yl)-diéthyl-amine
115. {1-(4-benzyl-5-thiomorpholin-4-yl-oxazol-2-yl)-2-[4-(diméthylamino-thiophèn-2-yl-méthyl)-cyclohexyl]-éthyl}-diéthyl-amine
116. {1-[4-benzyl-5-(3-méthyl-pipéridin-1-yl)-oxazol-2-yl]-2-[4-(diméthylamino-thiophèn-2-yl-méthyl)-cyclohexyl]-éthyl}-diéthyl-amine
117. {1-[4-benzyl-5-(3-méthyl-pipéridin-1-yl)-oxazol-2-yl]-2-[4-(diméthylamino-thiophèn-2-yl-méthyl)-cyclohexyl]-éthyl}-méthyl-phényl-amine
118. ({4-[2-(4-benzyl-pipérazin-1-yl)-2-(4-benzyl-5-thiomorpholin-4-yl-oxazol-2-yl)-éthyl]-cyclohexyl}-thiophèn-2-yl-méthyl)-diméthyl-amine
119. ({4-[2-[4-benzyl-5-(3-méthyl-pipéridin-l-yl)-oxazol-2-yl]-2-(4-benzyl-pipérazin-1-yl)-éthyl]-cyclohexyl}-thiophèn-2-yl-méthyl)-diméthyl-amine
120. [{4-[(4-benzyl-5-pyrrolidin-1-yl-oxazol-2-yl)-morpholin-4-yl-méthyl]-cyclohexyl}-(3-fluoro-phényl)-méthyl]-diméthyl-amine
121. [{4-[(4-benzyl-5-morpholin-4-yl-oxazol-2-yl)-morpholin-4-yl-méthyl]-cyclohexyl}-(3-fluoro-phényl)-méthyl]-diméthyl-amine
122. [(4-{(4-benzyl-5-pyrrolidin-1-yl-oxazol-2-yl)-[4-(4-fluoro-phényl)-pipérazin-1-yl]-méthyl}-cyclohexyl)-(3-fluoro-phényl)-méthyl]-diméthyl-amine
123. [(4-{(4-benzyl-5-pyrrolidin-1-yl-oxazol-2-yl)-[4-(4-méthoxy-phényl)-pipérazin-1-yl]-méthyl}-cyclohexyl)-(3-fluoro-phényl)-méthyl]-diméthyl-amine
124. [(4-{(4-benzyl-5-morpholin-4-yl-oxazol-2-yl)-[4-(4-méthoxy-phényl)-pipérazin-1-yl]-méthyl}-cyclohexyl)-(3-fluoro-phényl)-méthyl]-diméthyl-amine
125. [{4-[(4-benzyl-5-pyrrolidin-1-yl-oxazol-2-yl)-(3,4-dihydro-1H-isoquinoléin-2-yl)-méthyl]-cyclohexyl}-(3-fluoro-phényl)-méthyl]-diméthyl-amine
126. [{4-[(4-benzyl-5-morpholin-4-yl-oxazol-2-yl)-(3,4-dihydro-1H-isoquinoléin-2-yl)-méthyl]-cyclohexyl}-(3-fluoro-phényl)-méthyl]-diméthyl-amine
127. [{4-[{4-benzyl-5-[4-(4-méthoxy-phényl)-pipérazin-1-yl]-oxazol-2-yl}-(3,4-dihydro-1H-isoquinoléin-2-yl)-méthyl]-cyclohexyl}-(3-fluoro-phényl)-méthyl]-diméthyl-amine
128. [{4-[[4-benzyl-5-(2,6-diméthyl-morpholin-4-yl)-oxazol-2-yl]-(3,4-dihydro-1H-isoquinoléin-2-yl)-méthyl]-cyclohexyl}-(3-fluoro-phényl)-méthyl]-diméthyl-amine
129. [{4-[(4-benzyl-5-pyrrolidin-1-yl-oxazol-2-yl)-morpholin-4-yl-méthyl]-cyclohexyl}-(4-chloro-phényl)-méthyl]-diméthyl-amine
130. [{4-[(4-benzyl-5-morpholin-4-yl-oxazol-2-yl)-morpholin-4-yl-méthyl]-cyclohexyl}-(4-chloro-phényl)-méthyl]-diméthyl-amine
131. [[4-({4-benzyl-5-[4-(4-méthoxy-phényl)-pipérazin-1-yl]-oxazol-2-yl}-morpholin-4-yl-méthyl)-cyclohexyl]-(4-chloro-phényl)-méthyl]-diméthyl-amine
132. [(4-{[4-benzyl-5-(2,6-diméthyl-morpholin-4-yl)-oxazol-2-yl]-morpholin-4-yl-méthyl}-cyclohexyl)-(4-chloro-phényl)-méthyl]-diméthyl-amine
133. [(4-{(4-benzyl-5-pyrrolidin-1-yl-oxazol-2-yl)-[9-(4-fluoro-phényl)-pipérazin-1-yl]-méthyl}-cyclohexyl)-(4-chloro-phényl)-méthyl]-diméthyl-amine
134. [(4-{(4-benzyl-5-pyrrolidin-1-yl-oxazol-2-yl)-[4-(4-méthoxy-phényl)-pipérazin-1-yl]-méthyl}-cyclohexyl)-(4-chloro-phényl)-méthyl]-diméthyl-amine
135. [(4-{(4-benzyl-5-morpholin-4-yl-oxazol-2-yl)-[4-(4-méthoxy-phényl)-pipérazin-1-yl]-méthyl}-cyclohexyl)-(4-chloro-phényl)-méthyl]-diméthyl-amine
136. [(4-{[4-benzyl-5-(2,6-diméthyl-morpholin-4-yl)-oxazol-2-yl]-[4-(4-méthoxy-phényl)-pipérazin-1-yl]-méthyl}-cyclohexyl)-(4-chloro-phényl)-méthyl]-diméthyl-amine
137. [{4-[(4-benzyl-5-pyrrolidin-1-yl-oxazol-2-yl)-(3,4-dihydro-1H-isoquinoléin-2-yl)-méthyl]-cyclohexyl}-(4-chloro-phényl)-méthyl]-diméthyl-amine
138. ({4-[(4-benzyl-5-pyrrolidin-1-yl-oxazol-2-yl)-morpholin-4-yl-méthyl]-cyclohexyl}-thiophèn-2-yl-méthyl)-diméthyl-amine
139. ({4-[(4-benzyl-5-morpholin-4-yl-oxazol-2-yl)-morpholin-4-yl-méthyl]-cyclohexyl}-thiophèn-2-yl-méthyl)-diméthyl-amine
140. {[4-({4-benzyl-5-[4-(4-méthoxy-phényl)-pipérazin-1-yl]-oxazol-2-yl}-morpholin-4-yl-méthyl)-cyclohexyl]-thiophèn-2-yl-méthyl}-diméthyl-amine
141. [(4-{[4-benzyl-5-(2,6-diméthyl-morpholin-4-yl)-oxazol-2-yl]-morpholin-4-yl-méthyl}-cyclohexyl)-thiophèn-2-yl-méthyl]-diméthyl-amine
142. [(4-{(4-benzyl-5-pyrrolidin-1-yl-oxazol-2-yl)-[4-(4-fluoro-phényl)-pipérazin-1-yl]-méthyl}-cyclohexyl)-thiophèn-2-yl-méthyl]-diméthyl-amine
143. [(4-{(4-benzyl-5-pyrrolidin-1-yl-oxazol-2-yl)-[4-(4-méthoxy-phényl)-pipérazin-1-yl]-méthyl}-cyclohexyl)-thiophèn-2-yl-méthyl]-diméthyl-amine
144. [(4-{(4-benzyl-5-morpholin-4-yl-oxazol-2-yl)-[4-(4-méthoxy-phényl)-pipérazin-1-yl]-méthyl}-cyclohexyl)-thiophèn-2-yl-méthyl]-diméthyl-amine
145. [(4-{[4-benzyl-5-(2,6-diméthyl-morpholin-4-yl)-oxazol-2-yl]-[4-(4-méthoxy-phényl)-pipérazin-1-yl]-méthyl}-cyclohexyl)-thiophèn-2-yl-méthyl]-diméthyl-amine
146. ({4-[(4-benzyl-5-pyrrolidin-1-yl-oxazol-2-yl)-(3,4-dihydro-1H-isoquinoléin-2-yl)-méthyl]-cyclohexyl}-thiophèn-2-yl-méthyl)-diméthyl-amine
147. ({4-[{4-benzyl-5-[4-(4-méthoxy-phényl)-pipérazin-1-yl]-oxazol-2-yl}-(3,4-dihydro-1H-isoquinoléin-2-yl)-méthyl]-cyclohexyl}-thiophèn-2-yl-méthyl)-diméthyl-amine
148. ({4-[[4-benzyl-5-(2,6-diméthyl-morpholin-4-yl)-oxazol-2-yl]-(3,4-dihydro-1H-isoquinoléin-2-yl)-méthyl]-cyclohexyl}-thiophèn-2-yl-méthyl)-diméthyl-amine
149. [{4-[2-(4-benzyl-5-pyrrolidin-1-yl-oxazol-2-yl)-2-morpholin-4-yl-éthyl]-cyclohexyl}-(3-fluoro-phényl)-méthyl]-diméthyl-amine
150. [{4-[2-(4-benzyl-5-morpholin-4-yl-oxazol-2-yl)-2-morpholin-4-yl-éthyl]-cyclohexyl}-(3-fluoro-phényl)-méthyl]-diméthyl-amine
151. [[4-(2-{4-benzyl-5-[4-(4-méthoxy-phényl)-pipérazin-1-yl]-oxazol-2-yl}-2-morpholin-4-yl-éthyl)-cyclohexyl]-(3-fluoro-phényl)-méthyl]-diméthyl-amine
152. [(4-{2-[4-benzyl-5-(2,6-diméthyl-morpholin-4-yl)-oxazol-2-yl]-2-morpholin-4-yl-éthyl}-cyclohexyl)-(3-fluoro-phényl)-méthyl]-diméthyl-amine
153. [(4-{2-[4-benzyl-5-(2,6-diméthyl-morpholin-4-yl)-oxazol-2-yl]-2-[4-(4-fluoro-phényl)-pipérazin-1-yl]-éthyl}-cyclohexyl)-(3-fluoro-phényl)-méthyl]-diméthyl-amine
154. [(4-{2-(4-benzyl-5-morpholin-4-yl-oxazol-2-yl)-2-[4-(4-méthoxy-phényl)-pipérazin-1-yl]-éthyl}-cyclohexyl)-(3-fluoro-phényl)-méthyl]-diméthyl-amine
155. [(4-{2-{4-benzyl-5-[4-(4-méthoxy-phényl)-pipérazin-1-yl}-oxazol-2-yl}-2-[4-(4-méthoxy-phényl)-pipérazin-1-yl]-éthyl}-cyclohexyl)-(3-fluoro-phényl)-méthyl]-diméthyl-amine
156. [(4-{2-[4-benzyl-5-(2,6-diméthyl-morpholin-4-yl)-oxazol-2-yl]-2-[4-(4-méthoxy-phényl)-pipérazin-1-yl]-éthyl}-cyclohexyl)-(3-fluoro-phényl)-méthyl]-diméthyl-amine
157. [{4-[2-{4-benzyl-5-[4-(4-méthoxy-phényl)-pipérazin-l-yl]-oxazol-2-yl}-2-(3,4-dihydro-1H-isoquinoléin-2-yl)-éthyl]-cyclohexyl}-(3-fluoro-phényl)-méthyl]-diméthyl-amine
158. ({4-[2-(4-benzyl-5-morpholin-4-yl-oxazol-2-yl)-2-morpholin-4-yl-éthyl]-cyclohexyl}-thiophèn-2-yl-méthyl)-diméthyl-amine
159. {[4-(2-{4-benzyl-5-[4-(4-méthoxy-phényl)-pipérazin-1-yl]-oxazol-2-yl}-2-morpholin-4-yl-éthyl)-cyclohexyl]-thiophèn-2-yl-méthyl}-diméthyl-amine
160. [(4-{2-[4-benzyl-5-(2,6-diméthyl-morpholin-4-yl)-oxazol-2-yl]-2-morpholin-4-yl-éthyl}-cyclohexyl)-thiophèn-2-yl-méthyl]-diméthyl-amine
161. [(4-{2-(4-benzyl-5-morpholin-4-yl-oxazol-2-yl)-2-[4-(4-fluoro-phényl)-pipérazin-1-yl]-éthyl}-cyclohexyl)-thiophèn-2-yl-méthyl]-diméthyl-amine
162. [(4-{2-{4-benzyl-5-[4-(4-méthoxy-phényl)-pipérazin-1-yl]-oxazol-2-yl}-2-[4-(4-fluoro-phényl)-pipérazin-1-yl]-éthyl}-cyclohexyl)-thiophèn-2-yl-méthyl]-diméthyl-amine
163. [(4-{2-(4-benzyl-5-pyrrolidin-1-yl-oxazol-2-yl)-2-[4-(4-méthoxy-phényl)-pipérazin-1-yl]-éthyl}-cyclohexyl)-thiophèn-2-yl-méthyl]-diméthyl-amine
164. [(4-{2-{4-benzyl-5-[4-(4-méthoxy-phényl)-pipérazin-1-yl]-oxazol-2-yl}-2-[4-(4-méthoxy-phényl)-pipérazin-1-yl]-éthyl}-cyclohexyl)-thiophèn-2-yl-méthyl]-diméthyl-amine
165. [(4-{2-[4-benzyl-5-(2,6-diméthyl-morpholin-4-yl)-oxazol-2-yl]-2-[4-(4-méthoxy-phényl)-pipérazin-1-yl]-éthyl}-cyclohexyl)-thiophèn-2-yl-méthyl]-diméthyl-amine
166. ({4-[2-{4-benzyl-5-[4-(4-méthoxy-phényl)-pipérazin-1-yl]-oxazol-2-yl}-2-(3,4-dihydro-1H-isoquinoléin-2-yl)-éthyl]-cyclohexyl}-thiophèn-2-yl-méthyl)-diméthyl-amine.

3. Procédé de préparation d'un dérivé substitué de l'oxazole selon la revendication 1, dans lequel on chauffe pendant 1 à 10 heures à une température comprise entre 30 et 100°C, de préférence entre 40 et 80°C, dans un solvant organique, par exemple le méthanol ou l'éthanol, des aldéhydes de formule générale A avec des amines de formule générale B et des isonitrilamides de formule générale C :

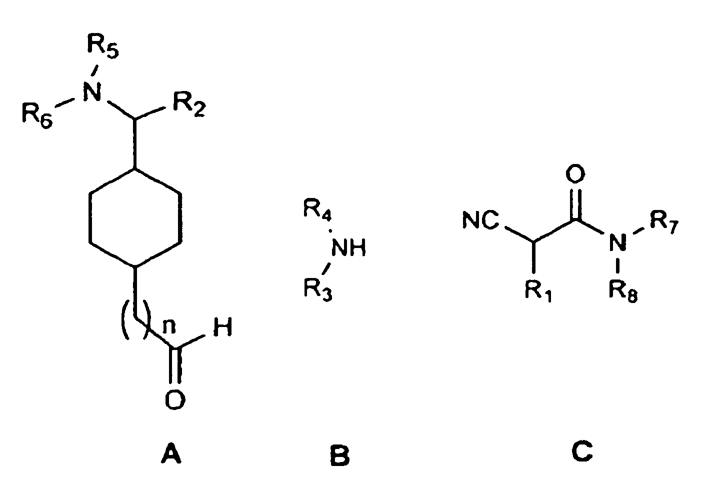

4. Médicament contenant au moins un dérivé substitué de l'oxazole selon la revendication 1, éventuellement sous forme du racémate ; des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles, ainsi qu'éventuellement des additifs et/ou adjuvants éventuellement présents, et/ou éventuellement d'autres principes actifs.

5. Utilisation d'un dérivé substitué de l'oxazole selon la revendication 1, éventuellement sous forme du racémate ; des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles, pour préparer un médicament destiné au traitement de la douleur, en particulier de la douleur aiguë, neuropathique ou chronique.

6. Utilisation d'un dérivé substitué de l'oxazole selon la revendication 1, éventuellement sous forme du racémate ; des énantiomères, des diastéréoisomères, des mélanges des énantiomères ou des diastéréoisomères, ou d'un énantiomère ou d'un diastéréoisomère unique ; des bases et/ou des sels d'acides physiologiquement compatibles, pour préparer un médicament destiné au traitement des dépressions, de l'incontinence urinaire, de la diarrhée, du prurit, de l'alcoolisme et de la toxicomanie, de la dépendance aux médicaments, de l'avolition et/ou pour l'anxiolyse.
